(19) Europäisches Patentamt
European Patent Office
Office européen des brevets

(11) Publication number: 0 356 788 A2

# (12) EUROPEAN PATENT APPLICATION

(21) Application number: 89114889.2

(22) Date of filing: 11.08.89

(51) Int. Cl.[5]: C07D 213/55 , C07D 213/89 , C07D 215/14 , C07D 215/60 , C07D 221/10 , C07D 221/16 , C07D 405/04 , C07D 409/04 , A61K 31/44 , A61K 31/47 , //(C07D405/04,317:00,213:00), (C07D409/04,333:00,213:00)

(30) Priority: 29.08.88 US 237349
17.11.88 US 272610

(43) Date of publication of application:
07.03.90 Bulletin 90/10

(84) Designated Contracting States:
AT BE CH DE ES FR GB GR IT LI LU NL SE

(71) Applicant: E.R. SQUIBB & SONS, INC.
Lawrenceville-Princeton Road
Princeton New Jersey 08543-4000(US)

(72) Inventor: Robl, Jeffrey Adam
103 Plumly Way
Holland Pennsylvania 18966(US)

(74) Representative: Vannini, Torquato et al
JACOBACCI-CASETTA & PERANI S.p.A. 7 Via Visconti di Modrone
I-20122 Milan(IT)

## (54) Quinoline and pyridine anchors for HMG-CoA reductase inhibitors.

(57) Compounds which are useful as inhibitors of cholesterol biosynthesis and thus as hypocholesterolemic agents are provided which have a quinoline or a pyridine anchor attached by means of a linker to a binding domain sidechain, which compounds inhibit the enzyme 3-hydroxy-3-methylglutaryl-coenzyme A reductase.

EP 0 356 788 A2

## QUINOLINE AND PYRIDINE ANCHORS FOR HMG-CoA REDUCTASE INHIBITORS

The present invention is related to compounds and pharmaceutical compositions useful as hypocholesterolemic and hypolipidemic agents. More particularly, this invention concerns certain inhibitors of the enzyme 3-hydroxy-3-methylglutarylcoenzyme A reductase (HMG-CoA reductase) which contain a quinoline or pyridine nucleus, attached by means of a linker, to an HMG-binding domain sidechain, of pharmaceutical compositions containing such compounds and a method of lowering blood serum cholesterol levels employing such pharmaceutical compositions.

In accordance with the present invention, there are provided certain quinoline and pyridine containing compounds which are potent inhibitors of cholesterol biosynthesis by virtue of their ability to inhibit the enzyme 3-hydroxy-3-methyl-glutaryl-coenzyme A reductase (HMG-CoA reductase).

In particular, in its broadest chemical compound aspect, the present invention provides compounds containing the following quinoline and pyridine structures (I):

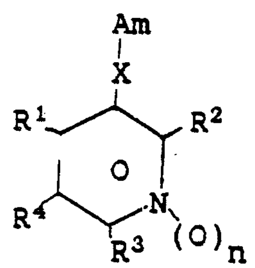

I

wherein $R^1$, $R^2$, $R^3$, and $R^4$ are the same or different and are each independently selected from H or a lower alkyl, aryl, substituted aryl or aralkyl, or aralkoxy and n is 0 or 1, linked by an appropriate linker (X) to an HMG-binding domain sidechain (Am). In addition, $R^3$ and $R^4$ taken together can be $-(CH_2)p-$,

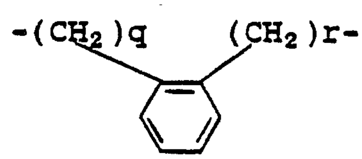

or $(CH=CH)_2$ wherein p = 3-5; q=0-3 and r=0-3; except when Am = $A_2$, $R^3$, $R^4$ cannot be $(CH=CH)_2$.

In another aspect, the present invention provides pharmaceutical compositions, useful as hypolipidemic or hypocholesterolemic agents comprising a hypolipidemic or hypocholesteromic amount of a compound in accordance with this invention as set forth above, in combination with a pharmaceutioally acceptable carrier.

In another aspect, the present invention provides a method of inhibiting cholesterol biosynthesis in a patient in need of such treatment by administering a pharmaceutical composition in accordance with the present invention as defined above.

In accordance with the present invention, there is provided compounds useful in inhibiting the enzyme 3-hydroxy-3-methyglutaryl-coenzyme A (HMG-CoA reductase), which inhibitors are useful as hypocholesterolemic and hypolipidemic agents, and which compounds comprise a quinoline nucleus or pyridine nucleus

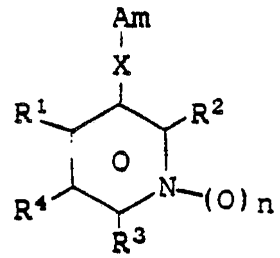

I

linked, by a suitable linker (X) to an HMG-binding domain sidechain (Am). Wherein $R^1$, $R^2$, $R^3$, and $R^4$ are the same or different and are each independently selected from H or a lower alkyl, aryl, substituted aryl or aralkyl, or aralkoxy and n is 0 or 1. In addition, $R_3$ and $R_4$ taken together can be $-(CH_2)p-$,

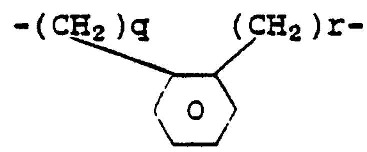

or $(CH=CH)_2$ wherein p = 3-5, q=0-3 and r=0-3; except when $Am=A_2$, $R^3$, $R^4$ cannot be $(CH=CH)_2$.

The quinoline and pyridine nuclei appear to act as hydrophobic anchors of the inhibitor, by binding to a hydrophobic pocket of the reductase enzyme, which results in enhanced inhibitory activity relative to compounds without such an anchor.

In preferred embodiments of the invention (Am) is an HMG-binding domain sidechain having a dihydroxy or a phosphinic acid function.

A suitable phosphinic acid HMG-binding domain sidechain ($A_1$) is

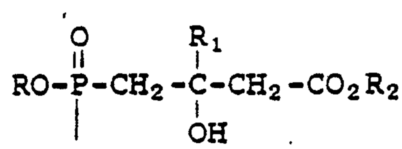

wherein R and $R^2$ are independently selected from hydrogen, lower alkyl, alkali metal salt ion and alkaline earth metal salt ion; and $R_1$ is H or lower alkyl.

A suitable dihydroxy acid binding domain sidechain ($A_2$) is

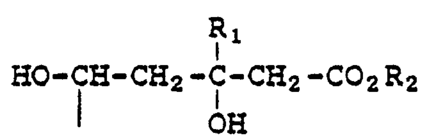

wherein $R_1$ is H or lower alkyl, $R_2$ is H or lower alkyl in free acid form or in the form of a physiologically acceptable and hydrolyzable ester or $\delta$ lactone thereof. In addition $R_2$ can be alkali metal salt ion or alkaline earth metal salt ion. A suitable linker (X) is $-(CH_2)a-$, $-CH=CH-$, $-C\equiv C-$, $-CH_2O-$ (where O in the case of a $Am=A_1$ is linked to the phosphorous atom or the aromatic anchor, and, in the case of $Am=A_2$ O is linked to the aromatic anchor), "a" is 1, 2, or 3 carbon atoms.

The terms "salt" and "salts" refer to basic salts formed with inorganic and organic bases. Such salts include ammonium salts, alkali metal salts like, lithium, sodium and potassium salts (which are preferred), alkaline earth metal salts like the calcium and magnesium salts, salts with organic bases, such as amine like salts, e.g., dicyclohexylamine salt, benzathine, N-methyl-D-glucamine, hydrabamine salts, salts with amino acids like arginine, lysine and the like. The nontoxic, pharmaceutically acceptable salts are preferred, although other salts are also useful, e.g., in isolating or purifying the product.

The term "lower alkyl" or "alkyl" as employed herein alone or as part of another group includes both straight and branched chain hydrocarbons, containing 1 to 12 carbons in the normal chain or the various

branched isomers thereof, preferably 1 to 7 carbons, such as methyl, ethyl, propyl, isopropyl, butyl, t-butyl, isobutyl, pentyl, hexyl, isohexyl, heptyl, 4,4-dimethyl-pentyl, octyl, 2,2,4-trimethylpentyl, nonyl, decyl, undecyl, dodecyl, and the like, as well as such groups including a halo-substituent, such as F, Br, Cl, I or $CF_3$, an alkoxy substituent, an aryl substituent, an alkyl-aryl substituent, a haloaryl substituent, a cycloalkyl substituent, an aryl substituted cycloalkyl substituent an alkylcycloalkyl substituent, hydroxy, a alkylamino substituent, a alkanoylamino substituent, a arylcarbonylamino substituent, a nitro substituent, a cyano substituent, a thiol substituent or a alkylthio substituent.

The term "cycloalkyl" as employed herein alone or as part of another group includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, and of which groups may be substituted with 1 or 2 halogens, 1 or 2 lower alkyl groups, 1 or 2 lower alkoxy groups, 1 or 2 hydroxy groups, 1 or 2 alkylamino groups, 1 or 2 alkanoylamino groups, 1 or 2 arylcarbonylamino groups, 1 or 2 amino groups, 1 or 2 nitro groups, 1 or 2 cyano groups, 1 or 2 thiol groups, and/or 1 or 2 alkylthio groups.

The term "aryl" or "Ar" as employed herein refer to monocyclic or bicyclic aromatic groups containing from 6 to 10 carbons in the ring portion, such as phenyl, naphthyl, substituted phenyl or substituted naphthyl wherein the substituent on either the phenyl or naphthyl may be 1, 2 or 3 lower alkyl groups, halogens (Cl, Br or F), 1, 2 or 3 lower alkoxy groups, 1, 2 or 3 hydroxy groups, 1, 2 or 3 phenyl groups, 1, 2 or 3 alkanoyloxy group, 1, 2 or 3 benzoyloxy groups, 1, 2 or 3 haloalkyl groups, 1, 2 or 3 halophenyl groups, 1, 2 or 3 allyl groups, 1, 2 or 3 cycloalkylalkyl groups, 1, 2 or 3 adamantylalkyl groups, 1, 2 or 3 alkylamino groups, 1, 2 or 3 alkanoylamino groups, 1, 2 or 3 arylcarbonylamino groups, 1, 2 or 3 amino groups, 1, 2 or 3 nitro groups, 1, 2 or 3 cyano groups, 1, 2 or 3 thiol groups, and/or 1, 2 or 3 alkylthio groups with the aryl group preferably containing 3 substituents.

The term "aralkyl," "aryl-alkyl" or "aryl-lower alkyl" as used herein alone or as part of another group refers to lower alkyl groups as discussed above having an aryl substituent, such as benzyl.

The term "lower alkoxy," "alkoxy," or "aryloxy" or aralkoxy" as employed herein alone or as part of another group includes any of the above lower alkyl, alkyl, aralkyl or aryl groups linked to an oxygen atom.

The term "lower alkylthio," "alkylthio," "arylthio" or "aralkylthio" as employed herein alone or as part of another group includes any of the above lower alkyl, alkyl, aralkyl or aryl groups linked to a sulfur atom.

The term "lower alkylamino," "alkylamino," "arylamino," "arylalkylamino" as employed herein alone or as part of another group includes any of the above lower alkyl, alkyl, aryl or arylalkyl groups linked to a nitrogen atom.

The term "alkanoyl" as used herein as part of another group refers to lower alkyl linked to a carbonyl group.

The term "halogen" or "halo" as used herein refers to chlorine, bromine, fluorine, iodine and $CF_3$, with chlorine or fluorine being preferred.

The compounds of the present invention are made generally in the following manner.

The syntheses of aldehyde 5 which are the starting materials for compounds of the invention when $R^3$ & $R^4$ taken together can be $(CH=CH)_2$, are shown in Scheme 1. Reaction of anthranilonitrile 1 with an excess of Grignard reagent ($R^1MgBr$) in an aprotic solvent, such as $Et_2O$ or THF, followed by acidic and basic work-up affords keto-aniline 2. Friedlander condensation of 2 with an appropriately substituted $\beta$-keto-ester under acidic conditions (i.e. $H_2SO_4$/HOAc/$\Delta$, $H_2SO_4$/EtOH/$\Delta$) provides quinoline 3. Reduction of the ester group in 3 with reducing agents such as $LiAlH_4$ or DIBAL-H in inert solvents such as THF, $Et_2O$, or toluene gives alcohol 4. Oxidants such as Dess-Martin periodinane or oxalyl chloride/DMSO/triethylamine convert compound 4 to aldehyde 5.

SCHEME 1

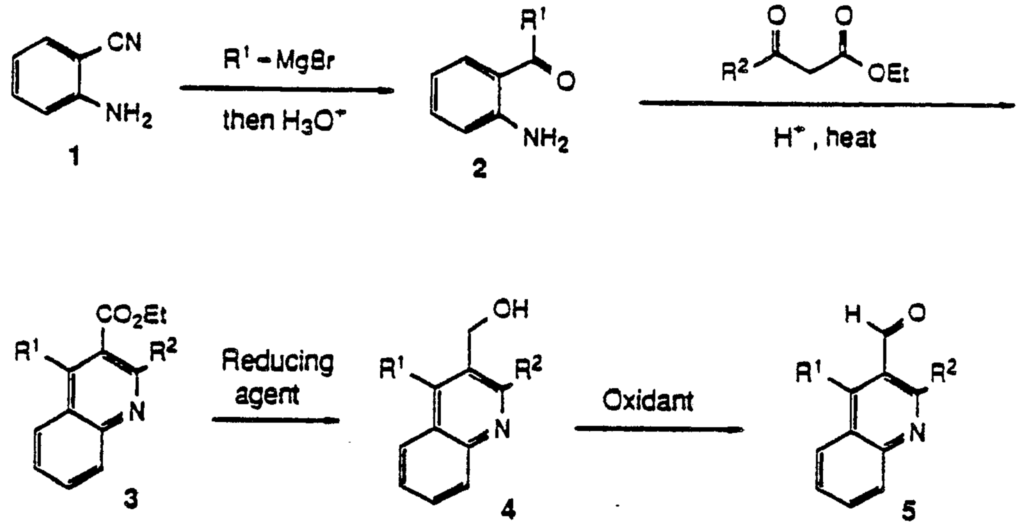


1 2 3 4 5

The syntheses of aldehydes 10 which are the starting materials for compounds of the invention I where $R_3$, $R_4 \neq (CH=CH)_2$, are shown in Scheme 2. Condensation of ketone $R^3COCH_2R^4$ with aldehyde $R^1CHO$ under either acidic or basic conditions (i.e. $H_2SO_4$/EtOH or EtONa/EtOH) provides $\alpha,\beta$-unsaturated ketone 6. Michael addition of a $\beta$-keto-ester to compound 6 under basic conditions (i.e. EtONa/EtOH, KOBut/EtOH, NaH/THF) gives 1,5-diketone 7. Alternately compound 7 may be prepared by an additional route. Knoevenagel condensation of an appropriately substituted $\beta$-keto-ester with aldehyde $R^1CHO$ (piperdine/HOAc/benzene/heat) provides compound 11. Addition of a ketone enolate, generated by treatment of $R^3COCH_2R^4$ with bases such as LDA, LiTMP, or $LiN(TMS)_2$ in an aprotic solvent such as THF, to compound 11 gives, 1,5-diketone 7. Compound 8 is formed by treatment of compound 7 with $NH_2OH.HCl$ or $NH_4OAc/Cu(OAc)_2$ in hot HOAc. Reduction of the ester group in 8 with a reducing agent such as $LiAlH_4$ or DIBAL-H in inert solvents such as THF, $Et_2O$, or toluene gives alcohol 9. Oxidants such as Dess-Martin periodinane or oxalyl chloride/DMSO/triethylamine convert compound 9 to aldehyde 10.

SCHEME 2

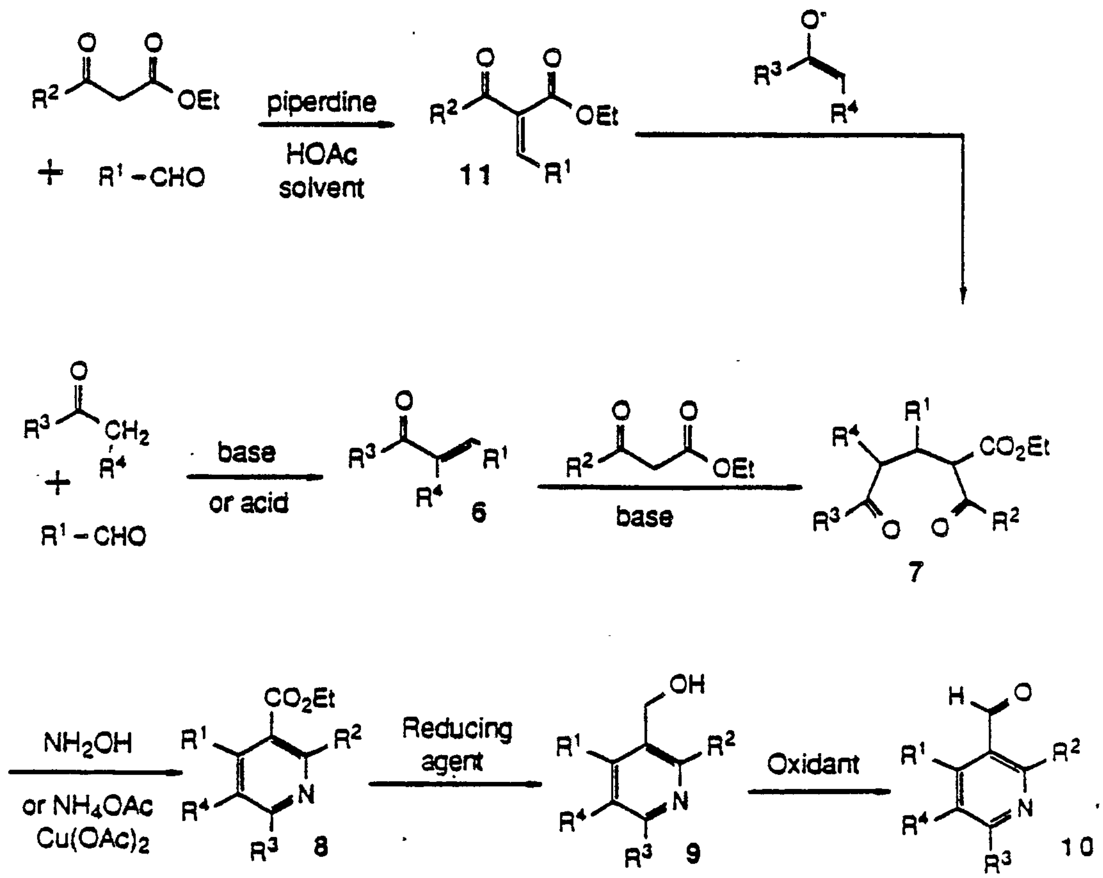


The synthesis of compounds I where Am = $A_2$ and X = CH = CH and $CH_2CH_2$ (Compounds 16 and 18) is described in Scheme 3. Reaction of aldehyde 12 with cis-1-ethoxy-2-lithioethylene at low temperatures (-78° C) in an appropriate solvent such as THF, followed by acidic work-up provides enal 13. Condensation of the dianion of methyl acetoacetate with enal 13 in an aprotic solvent (THF, $Et_2O$) affords compound 14. Compound 15 is obtained via stereoselective reduction of 14 with a trialkylborane and a reducing agent such as $NaBH_4$ in a solvent such as THF containing MeOH and a small amount of catalyst such as $O_2$ or pivalic acid. Compound 15 can be saponified to 16 using aqueous solutions of a metal hydroxide in an appropriate solvent (i.e. MeOH, dioxane). Additionally, 15 may be hydrogenated to 16 in the presence of a suitable catayst (Pd, Pt, Ru) and solvent (MeOH, EtOAc, EtOH) and then saponified as described above to provide compound 18.

SCHEME 3

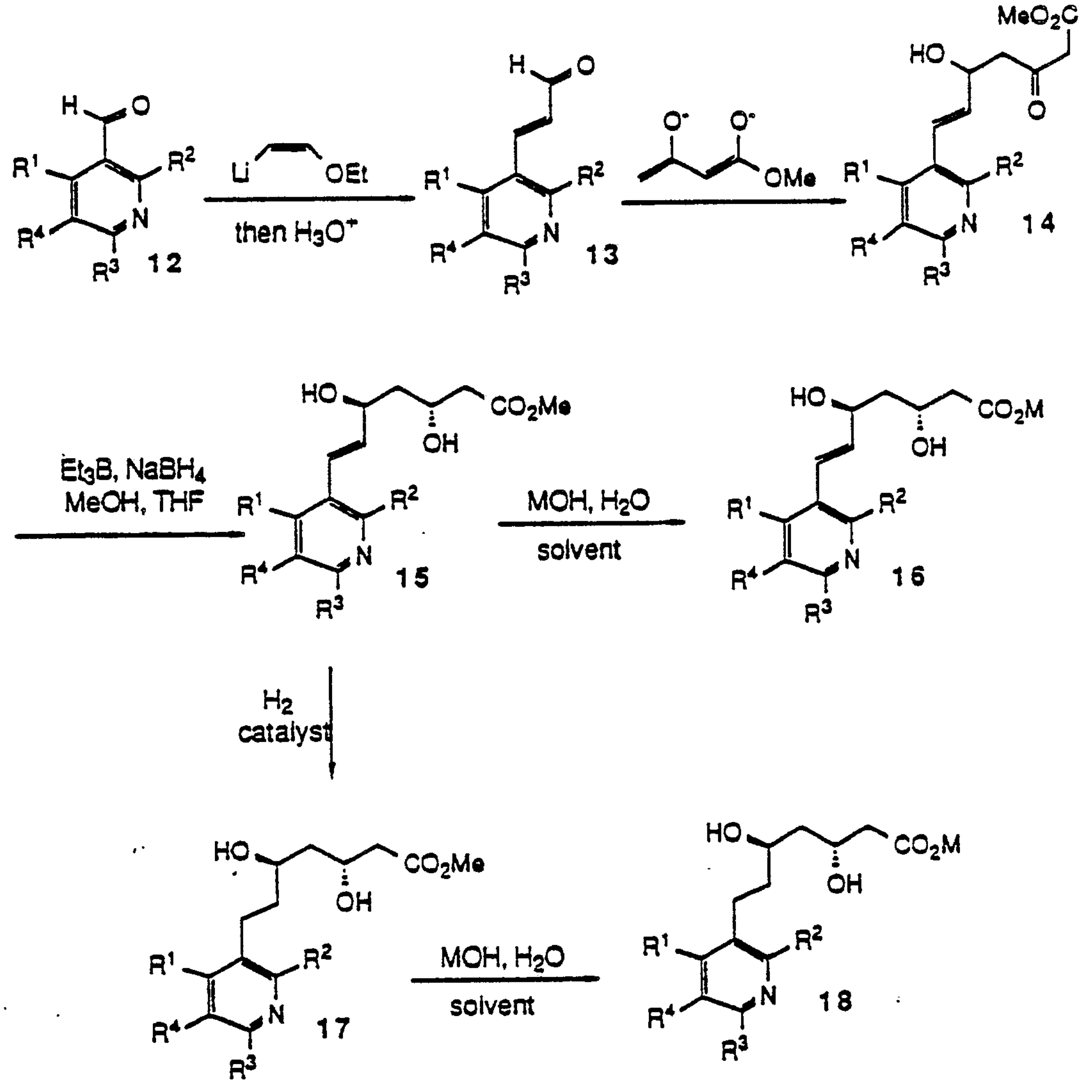


The synthesis of phosphinic acid deriviatives I, where Am = $A_1$ and X = C≡C and $CH_2CH_2$ - (Compounds 24 and 26) is described in Scheme 4. Reaction of aldehyde 12 with carbon tetrabromide and triphenylphosphine in solvents such as $CH_2Cl_2$ or $CH_3CN$ provides dibromide 19. Conversion of 19 to 20 is effected by treatment of 19 with alkyl lithium reagents such as n-BuLi at low temperature (-78° C) in an aprotic solvent such as THF. Conversely, 20 may be obtained directly from 12 utilizing dimethyl diazomethylphosphonate and KOBut in a solvent such as THF (-78° C to r.t.). Metallation of 20 (RLi, THF, 78° C) followed by treatment with the chiral phosphonochloridate 21 affords compounds 22 which can subsequently be desilylated (TBAF/HOAc) in an appropriate solvent such as THF to provide 23. Compound 23 can be saponified to 24 using aqueous solutions of a metal hydroxide in an appropriate solvent (i.e. MeOH, dioxane). Additionally, 23 may be hydrogenated to 25 in the presence of a suitable cataust (Pd, Pt, Ru) and solvent (MeOH, EtOAc, ETOH) and then saponified as above to provide compound 26.

SCHEME 4

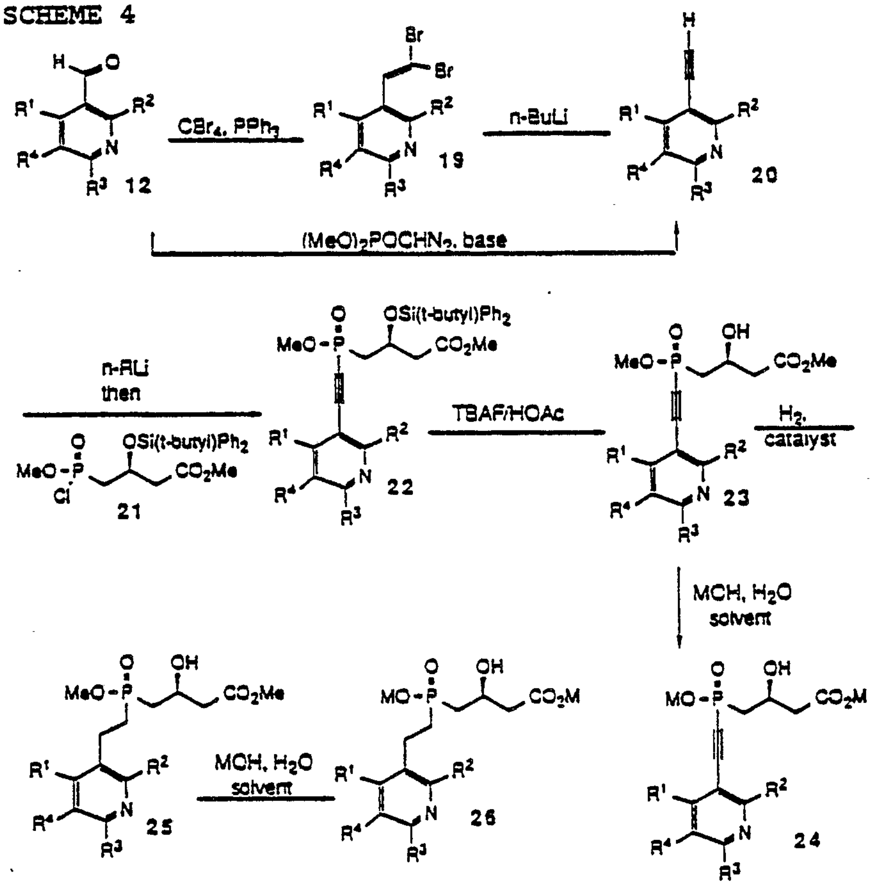


Scheme 5 describes the synthetic route to compounds I where Am = $A_1$ and X = CH = CH (e.g. compound 31. Treatment of acetylene 20 with neat $Bu_3SnH$ in the presence of AIBN at elevated temperature (i.e. 140 °C) affords trans vinyl stannane 27. Compound 27 was converted to 28 by iodine treatment of 27 in an appropriate solvent such as $Et_2O$ or $CHCl_3$. Metallation of 28 (t-ButylLi or n-BuLi) at -78 °C in an aprotic solvent such as THF gives the corresponding vinyl anion which may be coupled with phosphonochloridate 21 at -100 °C to provide 29. Subsequently 29 may be desilylated (TBAF/HOAc) in an appropriate solvent such as THF to provide 30. Compound 30 can be saponified to 31 using aqueous solutions of a metal hydroxide in an appropriate solvent (i.e. MeOH, dioxane).

SCHEME 5

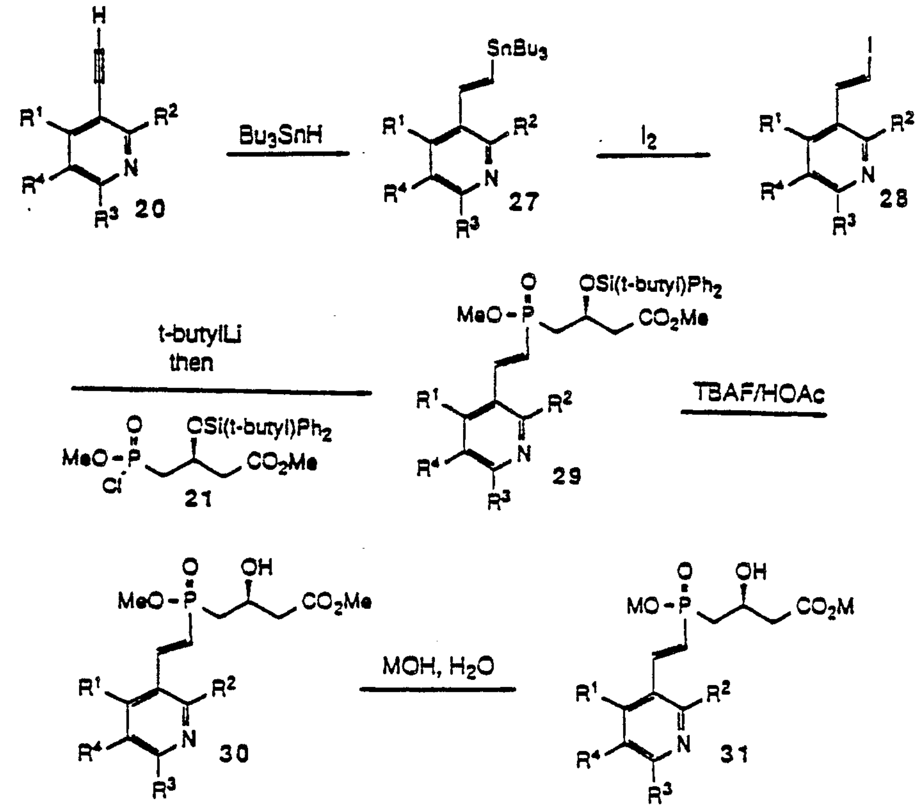


20 27 28 21 29 30 31

The synthesis of compounds I where Am = $A_2$, X = CH = CH and n = 1 (e.g., compound 35 is described iin Scheme 6. Bis-silylation of compound 15 with a bulky silylchloride (i.e. ClSi(t-butyl)$Ph_2$, ClSi(t-butyl)$Me_2$, $ClSiPh_3$) in the presence of a suitable base (i.e. TEA, imidazole, pyridine) and solvent ($CH_2Cl_2$, THF) provides compound 32. Treatment of 32 with oxidants such as m-CPBA or $CF_3CO_3H$ in an appropriate solvent such as $CH_2Cl_2$ or HOAc affords N-oxide 33. Desilylation of 33 (TBAF/HOAc/THF or HF/$CH_3CN$) gives 34 which may be saponified to 35 using aqueous solutions of a metal hydroxide in an appropriate solvent (i.e. MeOH, dioxane).

SCHEME 6

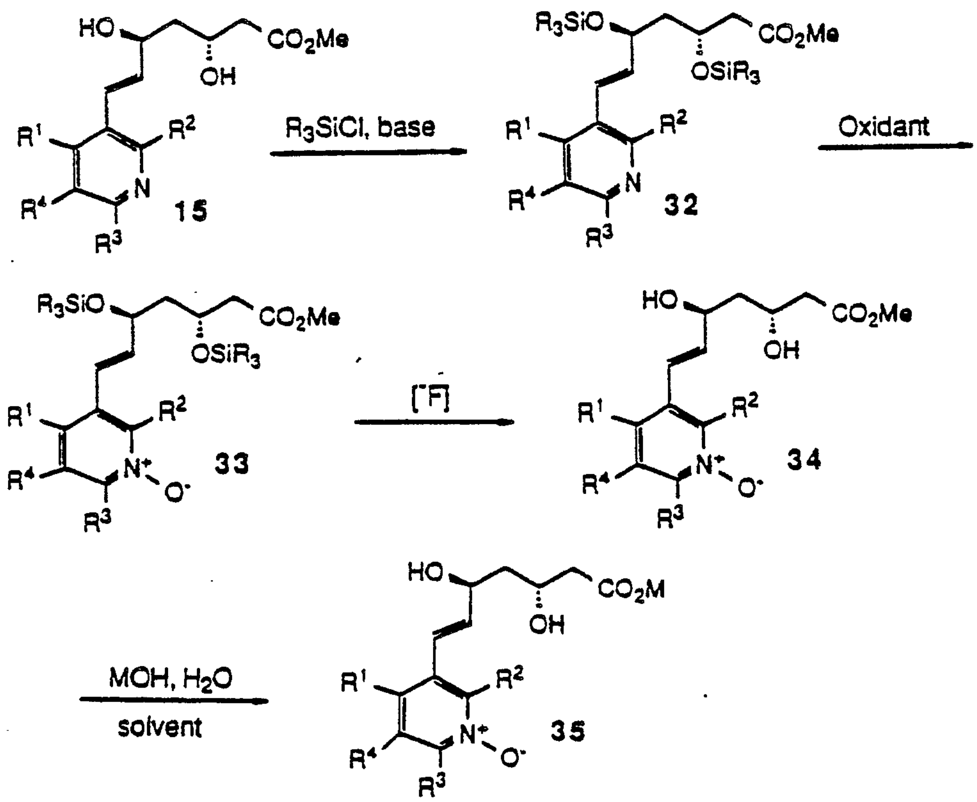


Additionally compound 17 may be oxidized and saponified, as described above, to provide compounds I where Am = $A_2$, X = $CH_2CH_2$ and n = 1 (e.g. compound 37) as shown in Scheme 7.

SCHEME 7

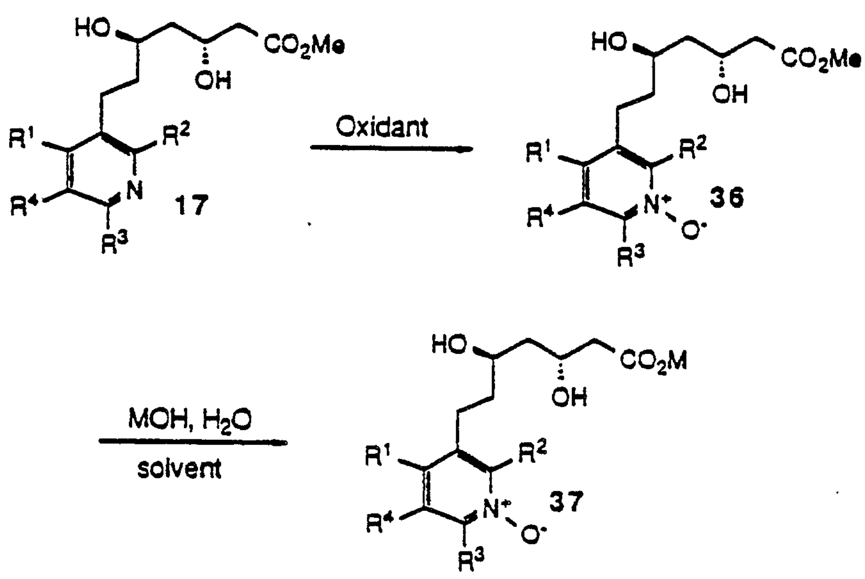


Scheme 8 depicts the chemistry used to synthesize compounds where Am = $A_1$ and n = 1 (i.e., compound 40. Treatment of 38 with oxidants such as m-CPBA Or $CF_3CO_3H$ in an appropriate solvent such as $CH_2Cl_2$ or HOAc affords N-oxide 39. Compound 39 may be saponified to 40 using aqueous solutions of

a metal hydroxide in an appropriate solvent (i.e. MeOH, dioxane).

SCHEME 8

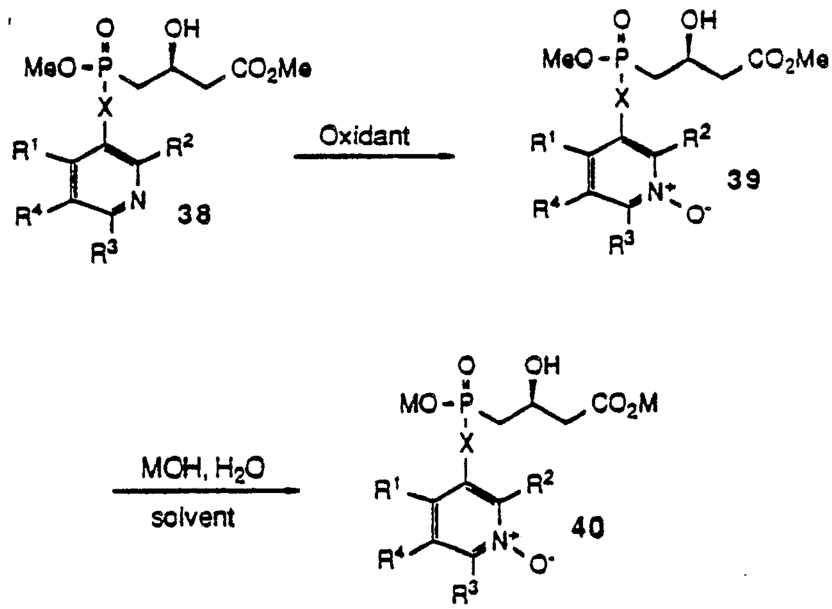


38

39

40

Specific examples of compounds contemplated as being within the scope of the present invention include the following:

Example 1

(3R*, 5S*, 6E)-7-[4-(4-Fluorophenyl)-2-(1-methylethyl)-6-phenyl-3-pyridinyl]-3,5-dihydroxy-6-heptenoic acid, monolithium salt.

Example 2

(3R*, 5S*, 6E)-7-[4-(4-Fluorophenyl)-2-methyl-6-phenyl-3-pyridinyl]-3,5-dihydroxy-6-heptenoic acid, monolithium salt.

Example 3

(3R*, 5S*, 6E)-7-[2-(4-Fluorophenyl)-4-(1-methylethyl)-6-phenyl-3-pyridinyl]-3,5-dihydroxy-6-heptenoic acid, monolithium salt.

Example 4

(S)-4-[[[4-(4-Fluorophenyl)-2-(1-methylethyl)-6-phenyl-3-pyridinyl]ethynyl]hydroxyphosphinyl]-3-hydroxybutanoic acid, dilithium salt.

Example 5

(S)-4-[[[2-(4-Fluorophenyl)-4-(1-methylethyl)-3-quinolinyl]ethynyl]hydroxyphosphinyl]-3-hydroxybutanoic acid, dilithium salt.

Example 6

(S)-4-[[2-[4-(4-Fluorophenyl)-2-(1-methylethyl)-6-phenyl-3-pyridinyl]ethyl]hydroxyphosphinyl]-3-hydroxybutanoic acid, dilithium salt.

Example 7

(S,E)-4-[[2-[4-(4-Fluorophenyl)-2-(1-methethyl)-6-phenyl-3-pyridinyl]ethenyl]hydroxyphosphinyl]-3-hydroxybutanoic acid, dilithium salt

Example 8

(S)-4-[[[4-(4-Fluorophenyl)-2-(1-methyl-ethyl)-3-quinolinyl]ethynyl]hydroxyphosphinyl]-3-hydroxybutanoic acid, dilithium salt

Example 9

(S)-4-[[[4-(4-Fluorophenyl)-2-(1-methylethyl)3-quinolinyl]ethynyl]hydroxyphosphinyl]-3-hydroxybutanoic acid, N-oxide, dilithium salt

Example 10

[3S,4(E)]-4-[[2-[2-(4-Fluorophenyl)-4-(1-methylethyl)-3-quinolinyl]ethenyl]hydroxyphosphinyl]-3-hydroxybutanoic acid, dilithium salt

Example 11

[3S,4(E)]-4-[[2-[4-(4-Fluorophenyl)-2-(1-methylethyl)-3-quinolinyl]ethenyl]hydroxy-phosphinyl]-3-hydroxybutanoic acid, dilithium salt

Example 12

(S)-4-[[[2-(4-Fluorophenyl)-4-(1-methyl-ethyl)-3-quinolinyl]ethynyl]hydroxyphosphinyl]-3-hydroxybutanoic acid, 1-oxide, dilithium salt

Example 13

[3S, 4(E)]-4-[[2-[4-(4-Fluorophenyl)-2-(1-methylethyl)-3-quinolinyl]ethenyl]hydroxyphosphinyl]-3-hydroxybutanoic acid, 1-oxide, dilithium salt

## Example 14

(S)-4-[[[2-(4-Fluorophenyl)-4-(1-methylethyl)-6-phenyl-3-pyridinyl]ethynyl]hydroxyphosphinyl]-3-hydroxybutanoic acid, dilithium salt

## Example 15

[3S,4(E)]-4-[[2-[2-(4-Fluorophenyl)-4-(1-methylethyl)-6-phenyl-3-pyridinyl]ethenyl]-hydroxyphosphinyl]-3-hydroxybutanoic acid, dilithium salt

A further aspect of the present invention is a pharmaceutical composition consisting of at least one of the compounds of the present invention association with a pharmaceutical vehicle or diluent. The pharmaceutical composition can be formulated employing conventional solid or liquid vehicles of diluents and pharmaceutical additives of a type appropriate to the mode of desired administration. The compounds can be administered by an oral route, for example, in the form of tablets, capsules, granules or powders, or they can be administered by a parenteral route in the form of injectable preparations, such dosage forms containing from 1 to 2000 mg of active compound per dosage, for use in the treatment. The dose to be administered depends on the unitary dose, the symptoms, and the age and the body weight of the patient.

The compounds of the present invention can be administered in a similar manner as known compounds suggested for use in inhibiting cholesterol biosynthesis, such as lovastatin, in mammalian species such as humans, dogs, cats and the like. Thus, the compounds of the invention may be administered in an amount from about 4 to 2000 mg in a single dose or in the form of individual doses from 1 to 4 times per day, preferably 4 to 200 mg in divided dosages of 1 to 100 mg, suitably 0.5 to 50 mg 2 to 4 times daily or in sustained release form.

Compounds containing dihydroxy acid HMG-CoA binding domain side chains are prepared as racemic mixtures (3S*, 5R*) and may later be resolved to obtain the 3S, 5R isomer which is preferred.

Phosphorus containing HMG-CoA binding domain sidechains may be prepared as racemic mixtures and may later be resolved to obtain the S-isomer which is preferred. However, these compounds may be prepared directly in the form of their S-isomers as described herein and in the working examples set out hereinafter.

The compounds of the invention are inhibitors of 3-hydroxy-3-methyl-glutaryl coenzyme A (HMG-CoA) reductase and thus are useful in inhibiting cholesterol biosynthesis as demonstrated by the following tests.

### 1) Rat Hepatic HMG-CoA Reductase

Rat hepatic HMG-CoA reductase activity is measured using a modification of the method described by Edwards (Edwards, P.A., et al., J. Lipid Res. 20-:40, 1979). Rat hepatic microsomes are used as a source of enzyme, and the enzyme activity is determined by measuring the conversion of the $^{14}C$-HMG-CoA substrate to $^{14}C$-mevalonic acid.

#### a. Preparation of Microsomes

Livers are removed from 2-4 cholestyramine-fed, decapitated, Sprague Dawley rats, and homogenized in phosphate buffer A (potassium phosphate, 0.04 M, pH 7.2; KCl, 0.05 M; sucrose, 0.1 M; EDTA, 0.03 M; aprotinin, 500 KI units/ml). The homogenate is spun at 16,000 x g for 15 minutes at 4° C. The supernatant is removed and recentrifuged under the same conditions a second time. The second 16,000 x g supernatant is spun at 100,000 x g for 70 minutes at 4° C. Pelleted microsomes are resuspended in a minimum volume of buffer A (3-5 ml per liver), and homogenized in a glass/glass homogenizer. Dithiothreitol is added (10 mM), and the preparation is aliquoted, quick frozen in acetone/dry ice, and stored at -80° C. The specific activity of the first microsomal preparation was 0.68 nmole mevalonic acid/mg protein/minute.

b. Enzyme Assay

The reductase is assayed in 0.25 ml which contains the following components at the indicated final concentrations:
0.04 M Potassium phosphate, pH 7.0
0.05 M KCl
0.10 M Sucrose
0.03 M EDTA
0.01 M Dithiothreitol
3.5 mM NaCl
1% Dimethylsulfoxide
50-200 μg Microsomal protein
100 μM $^{14}C$-[DL]HMG-CoA (0.05 μCi, 30-60 mCi/mmole
2.7 mM NADPH (nicotinamide adenine dinucleotide phosphate)
Reaction mixtures are incubated at 37° C. Under conditions described, enzyme activity increases linearly up to 300 μg microsomal protein per reaction mixture, and is linear with respect to incubation time up to 30 minutes. The standard incubation time chosen for drug studies is 20 minutes, which results in 12-15% conversion of HMG-CoA substrate to the mevalonic acid product. [DL-]HMG-CoA substrate is used at 100 μM, twice the concentration needed to saturate the enzyme under the conditions described. NADPH is used in excess at a level 2.7 times the concentration required to achieve maximum enzyme velocity.

Standardized assays for the evaluation of inhibitors are conducted according to the following procedure. Microsomal enzyme is incubated in the present of NADPH at 37° C for 15 minutes. DMSO vehicle with or without test compound is added, and the mixture further incubated for 15 minutes at 37° C. The enzyme assay is initiated by adding $^{14}C$-HMG-CoA substrate. After 20 minutes incubation at 37° C the reaction is stopped by the addition of 25 μl of 33% KOH. $^{3}H$-mevalonic acid (0.05 μCi) is added, and the reaction mixture allowed to stand at room temperature for 30 minutes. Fifty μl 5N HCl is added to lactonize the mevalonic acid. Bromophenol blue is added as a pH indicator to monitor an adequate drop in pH. Lactonization is allowed to proceed for 30 minutes at room temperature. Reaction mixtures are centrifuged for 15 minutes at 2800 rpm. The supernatants are layered onto 2 grams AG 1-X8 anion exchange resin (Biorad, formate form) poured in 0.7 cm (id) glass columns, and eluted with 2.0 ml $H_2O$. The first 0.5 ml is discarded, and the next 1.5 ml is collected and counted for both tritium and carbon 14 in 10.0 ml Opti-fluor scintillation fluid. Results are calculated as nmoles mevalonic acid produced per 20 minutes, and are corrected to 100% recovery of tritium. Drug effects are expressed as $I_{50}$ values (concentration of drug producing 50% inhibition of enzyme activity) derived from composite dose response data with the 95% confidence interval indicated.

Conversion of drugs in lactone form to their sodium salts is accomplished by solubilizing the lactone in DMSO, adding a 10-fold molar excess of NaOH, and allowing the mixture to stand at room temperature for 15 minutes. The mixture is then partially neutralized (pH 7.5-8.0) using 1N HCl, and diluted into the enzyme reaction mixture.

2) Cholesterol Synthesis in Freshly Isolated Rat Hepatocytes

Compounds which demonstrate activity as inhibitors of HMG-CoA reductase are evaluated for their ability to inhibit $^{14}C$-acetate incorporation into cholesterol in freshly isolated rat hepatocyte suspensions using methods originally described by Capuzzi et al., (Capuzzi, D. M. and Margolis, S., Lipids, 6:602, 1971).

a. Isolation of Rat Hepatocytes

Sprague Dawley rats (180-220 grams) are anesthetized with Nembutal (50 mg/kg). The abdomen is opened and the first branch of the portal vein is tied closed. Heparin (100-200 units) is injected directly into the abdominal vena cava. A single closing suture is placed on the distal section of the portal vein, and the portal vein is canulated between the suture and the first branching vein. The liver is perfused at a rate of 20 ml/minute with prewarmed (37° C), oxygenated buffer A (HBSS without calcium or magnesium containing 0.5 mM EDTA) after severing the vena cava to allow drainage of the effluent. The liver is additionally perfused with 200 ml of prewarmed buffer B (HBSS containing 0.05% bacterial collagenase). Following perfusion with buffer B, the liver is excised and decapsulated in 60 ml Waymouth's medium allowing free

cells to disperse into the medium. Hepatocytes are isolated by low speed centrifugation for 3 minutes at 50xg at room temperature. Pelleted hepatocytes are washed once in Waymouth's medium, counted and assayed for viability by trypan blue exclusion. These hepatocyte enriched cell suspensions routinely show 70-90% viability.

b. $^{14}$C-Acetate Incorporation into Cholesterol

Hepatocytes are resuspended at 5x10$^6$ cells per 2.0 ml in incubation medium (IM) [0.02 M Tris-HCl (ph 7.4), 0.1 M KCl, 3.3 mM sodium citrate, 6.7 mM nicotinamide, 0.23 mM NADP, 1.7 mM glucose-6-phosphate].

Test compounds are routinely dissolved in DMSO or DMSO:$H_2O$ (1:3) and added to the IM. Final DMSO concentration in the IM is ≦ 1.0%, and has no significant effect on cholesterol synthesis.

Incubation is initiated by adding $^{14}$C-acetate (58 mCi/mmol, 2 µci/ml), and placing the cell suspensions (2.0 ml) in 35 mm tissue culture dishes, at 37°C for 2.0 hours. Following incubation, cell suspensions are transferred to glass centrifuge tubes and spun at 50xg for 3 minutes at room temperature. Cell pellets are resuspended and lysed in 1.0 ml $H_2O$, and placed in an ice bath.

Lipids are extracted essentially as described by Bligh, E. G. and W. J. Dyer, Can. J. Biochem. and Physiol., 37:911, 1959. The lower organic phase is removed and dried under a stream of nitrogen, and the residue resuspended in (100 µl) chloroform:methanol (2:1). The total sample is spotted on silica gel (LK6D) thin-layer plates and developed in hexane:ethyl ether:acetic acid (75:25:1). Plates are scanned and counted using a BioScan automated scanning system. Radiolabel in the cholesterol peak (RF 0.28) is determined and expressed at total counts per peak and as a percent of the label in the total lipid extract. Cholesterol peaks in control cultures routinely contain 800-1000 cpm, and are 9-20 of the label present in the total lipid extract; results compatable with Capuzzi, et al., indicating 9% of extracted label in cholesterol.

Drug effects (% inhibition of cholesterol synthesis) are determined by comparing % of label in cholesterol for control and drug treated cultures. Dose response curves are constructed from composite data from two or more studies, and results are expressed as $I_{10}$ values with a 95% confidence interval.

3) Cholesterol Synthesis in Human Skin Fibroblasts

Compound selectivity favoring greater inhibitory activity in hepatic tissue would be an attribute for a cholesterol synthesis inhibitor. Therefore, in addition to evaluating cholesterol synthesis inhibitors in hepatocytes, these compounds are also tested for their activity as inhibitors of cholesterol synthesis in cultured fibroblasts.

a. Human Skin Fibroblast Cultures

Human skin fibroblasts (passage 7-27) are grown in Eagles' minimal essential medium (EM) containing 10% fetal calf serum. For each experiment, stock cultures are trypsinized to disperse the cell monolayer, counted, and plated in 35 mm tissue culture wells (5x10$^5$ cells/2.0 ml). Cultures are incubated for 18 hours at 37°C in 5% $CO_2$/95% humidified room air. Cholesterol biosynthetic enzymes are induced by removing the serum containing medium, washing the cell monolayers, and adding 1.0 ml of EM containing 1.0% fatty acid free bovine serum albumin, and incubating the cultures an additional 24 hours.

b. $^{14}$C-Acetate Incorporation into Cholesterol

Induced fibroblast cultures are washed with $EMEM_{100}$ (Earle's minimal essential medium). Test compounds are dissolved in DMSO or DMSO:EM (1:3) (final DMSO concentration in cell cultures ≦ 1.0%), added to the cultures, and the cultures preincubated for 30 minutes at 37°C in 5% $CO_2$/95% humidified room air. Following preincubation with drugs, [1-$^{14}$C]Na acetate (2.0 µCi/ml, 58 mCi/mmole) is added, and the cultures reincubated for 4 hours. After incubation, the culture medium is removed, and the cell monolayer (200 µg cell protein per culture) is scraped into 1.0 ml of $H_2O$. Lipids in the lysed cell suspension are extracted ito chloroform:methanol as described for hepatocyte suspensions. The organic phase is dried under nitrogen, and the residue resuspended in chloroform:methanol (2:1) (100 µl), and the

total sample spotted on silica gel (LK6D) thin-layer plates, and analyzed as described for hepatocytes.

Inhibition of cholesterol synthesis is determined by comparing the percent of label in the cholesterol peak from control and drug-treated cultures. Results are expressed as $I_{50}$ values, and are derived from composite dose response curves form two or more experiments. A 95% confidence interval for the $I_{50}$ value is also calcualted from the composite dose response curves.

The following working Examples represent preferred embodiments of the present invention. Unless otherwise indicated, all temperatures are expressed in degrees Centigrade.

## Example 1

### (3R*, 5S*, 6E)-7-[4-(4-Fluorophenyl)-2-(1-methylethyl)-6-phenyl-3-pyridinyl]-3,5-dihydroxy-6-heptenoic acid, monolithium salt.

#### A. (E)-3-(4-fluorophenyl)-1-phenyl-2-propen-1-one

A mixture of acetophenone (7.02 gm, 58.4 mmol), p-fluorobenzaldehyde (7.24 gm, 58.4 mmol) and concentrated $H_2SO_4$ (10 ml) in glacial HOAc (116 ml) was stirred at room temperature for 2 days. The solution was poured into $H_2O$ (250 ml) and neutralized with 10% NaOH (200 ml). The aqueous layer was extracted once with $Et_2O$ and the $Et_2O$ layer was washed successively with $H_2O$, saturated $NaHCO_3$ (2x) and brine, then dried ($MgSO_4$). Filtration and removal of the solvent afforded a yellow solid which was recrystallized from hot hexane to give (E)-3-(4-fluorophenyl)-1-phenyl-2-propen-1-one (7.94 gm, 60%) as light yellow needles.

m.p. 86-88° C

TLC: $R_f$ 0.36 (20% EtOAc in hexane)

#### B. β-(4-fluorophenyl)-α-(2-methyl-1-oxopropyl)-δ-oxobenzenepentanoic acid, ethyl ester

To a mixture of (E)-3-(4-fluorophenyl)-1-phenyl-2-propen-1-one (4.59 gm, 20.3 mmol) and ethyl isobutyrylacetate (3.86 gm, 24.4 mmol) in absolute EtOH (100 ml) was added a solution of EtONa in EtOH. The EtONa solution was prepared by dissolving Na metal (50 mg, 2.2 mmol) in EtOH (10 ml). After stirring at room temperature for 3 hours, additional ethyl isobutyrylacetate (1.0 gm, 6.3 mmol) was added. Stirring continued for an additional 2 hours. The mixture was poured into a separatory funnel containing $Et_2O$ and saturated $NH_4Cl$. The phases were shaken and separated. The aqueous layer was extracted once with EtOAc and the combined organic layers were washed with brine and dried ($Na_2SO_4$). Filtration and removal of the solvent gave a solid/liquid mixture. Crystallization of the residue from hot hexane/EtOAc gave β-(4-fluorophenyl)-o-(2-methyl-1-oxopropyl)-δ-oxobenzenepentanoic acid, ethyl ester (5.91 gm, ~ 95% one diastereomer) as colorless needles. The mother liquor was stripped and recrystallized from hot hexane to afford additional product, giving a total of 7.21 gm (92%) β-(4-fluorophenyl)-α-(2-methyl-1-oxopropyl)-δ-oxobenzenepentanoic acid, ethyl ester as a 7:3:1 mixture of diastereomers.

m.p. (1st crop) 119.2-120.8° C

TLC: $R_f$ 0.22 and 0.18 (20% EtOAc in hexane)

#### C. 4-(4-fluorophenyl)-2-(1-methylethyl)-6-phenyl-3-pyridinecarboxylic acid, ethyl ester

A mixture of β-(4-fluorophenyl)-α-(2-methyl-1-oxopropyl)-δ-oxobenzenepentanoic acid, ethyl ester (7.92 gm, 20.6 mmol) and hydroxylamine hydrochloride (4.32 gm, 62.2 mmol) in glacial HOAc (100 ml) was refluxed for 2.5 hours. The reaction was cooled to room temperature and poured into an ice cold solution of concentrated $NH_4OH$ (140 ml) in $H_2O$ (400 ml). The resulting mixture was extracted twice with $Et_2O$ and the combined ethereal layers were washed with brine, dried ($Na_2SO_4$), filtered and stripped to yield a gummy orange-red oil. Flash chromatography of the oil (Merck $SiO_2$, 7% EtOAc in hexane) gave 4-(4-fluorophenyl)-2-(1-methylethyl)-6-phenyl-3-pyridinecarboxylic acid, ethyl ester (4.88 gm, 65%) as a near colorless gum.

TLC: $R_f$ 0.47 (20% EtOAc in hexane)

D. 4-(4-fluorophenyl)-2-(1-methylethyl)-6-phenyl-3-pyridinemethanol

A slurry of $LiAlH_4$ (775 mg, 20.4 mmol) in dry $Et_2O$ (70 ml) at 0° C was treated with a solution of 4-(4-fluorophenyl)-2-(1-methylethyl)-6-phenyl-3-pyridinecarboxylic acid, ethyl ester (4.80 gm, 13.2 mmol) in $Et_2O$ (5 ml). After 2.5 hours, the reaction was quenched and diluted with $H_2O$ and the aqueous layer was extracted twice with $Et_2O$ and once with EtOAc. The combined organic layers were washed with $H_2O$ and brine, then dried ($Na_2SO_4$), filtered and stripped. The resulting solid was dissolved in hot EtOAc and directly chromatographed (flash, Merck $SiO_2$, 20% EtOAc in hexane) to give crude 4-(4-fluorophenyl)-2-(1-methylethyl)-6-phenyl-3-pyridinemethanol. Recrystallization from hot hexane/EtOAc gave 4-(4-fluorophenyl)-2-(1-methylethyl)-6-phenyl-3-pyridinemethanol (3.30 gm) as a white solid. The mother liquor was stripped and recrystallized again to give an additional 230 mg of product (total 3.53 gm, 84%).
m.p. 167.2-167.8° C
TLC: $R_f$ 0.26 (20% EtOAc in hexane)

E. 4-(4-fluorophenyl)-2-(1-methylethyl)-6-phenyl-3-pyridinecarboxaldehyde

A solution of Dess-Martin periodinane (1.54 gm, 3.63 mmol) in dry $CH_2Cl_2$ (17 ml) was treated with t-butanol (267 mg, 3.60 mmol) and stirred for 15 minutes at room temperature. A solution of 4-(4-fluorophenyl)-2-(1-methylethyl)-6-phenyl-3-pyridinemethanol (895 mg, 2.78 mmol) in $CH_2Cl_2$ (12 ml) was then added and stirring was continued 30 minutes, after which time the mixture was quenched by the addition of $Et_2O$ (70 ml) and 1 N NaOH (35 ml). The biphasic mixture was stirred for 10 minutes and the layers were separated. The organic layer was washed with 1 N NaOH, $H_2O$ and brine, then dried ($Na_2SO_4$), filtered and stripped. The solid residue was chromatographed (Flash, Merck $SiO_2$, 10% EtOAc in hexane) to afford pure 4-(4-fluorophenyl)-2-(1-methylethyl)-6-phenyl-3-pyridinecarboxaldehyde (782 mg, 88%) as a white solid. Analytically pure material was obtained by recrystallization of the solid from a minimum amount of hot hexane.
m.p. 105-107° C
TLC: $R_f$ 0.50 (20% EtOAc in hexane)

F. (E)-3-[4-(4-fluorophenyl)-2-(1-methylethyl)-6-phenyl-3-pyridinyl]-2-propenal

n-BuLi (1.6 M in hexanes, 1.52 ml, 2.43 mmol) was added to a solution of cis-1-ethoxy-2-(tri-n-butylstannyl)ethylene (961 mg, 2.66 mmol) in dry THF (7.5 ml) at -78° C. The mixture was stirred for 1 hour, then treated with a solution of 4-(4-fluorophenyl)-2-(1-methylethyl)-6-phenyl-3-pyridinecarboxaldehyde (708 mg, 2.22 mmol) in THF (3.5 ml). One hour after the addition, the reaction was immersed in an ice bath, stirred for 5 minutes, then the mixture was quenched with $H_2O$ (8 ml) and 10% HCl (8 ml). The solution was stirred at room temperature for 2 hours, then basicified with 10% NaOH (10 ml) and extracted twice with $Et_2O$. The combined $Et_2O$ layers were washed with $H_2O$ and brine, dried ($Na_2SO_4$), then filtered and stripped to give a yellow oil. Chromatographic purification of the oil (Flash, Merck $SiO_2$, 10% EtOAc in hexane) gave (E)-3-[4-(4-fluorophenyl)-2-(1-methylethyl)-6-phenyl-3-pyridinyl]-2-propenal (665 mg, 87%) as a white solid. Recrystallization from hot hexane gave analytically pure material (580 mg).
m.p. 113.8-114.4° C
TLC: $R_f$ 0.35 (20% EtOAc in hexane)

G. (E)-7-[4-(4-fluorophenyl)-2-(1-methylethyl)-6-phenyl-3-pyridinyl]-5-hydroxy-3-oxo-6-heptenoic acid, methyl ester

Methyl acetoacetate (272 mg, 2.34 mmol) was added dropwise to a slurry of NaH (60% in mineral oil, 93.7 mg, 23.4 mmol) in dry THF (5.5 ml) at 0° C. After 15 minutes, the solution was treated with n-BuLi (1.6 M in hexanes, 1.11 ml, 1.78 mmol) and stirred an additional 15 minutes. (E)-3-[4-(4-fluorophenyl)-2-(1-methylethyl)-6-phenyl-3-pyridinyl]-2-propenal (560 mg, 1.62 mmol) in THF (3.0 ml) was added, resulting in an orange solution. After 25 minutes, the solution was poured into a biphasic mixture $Et_2O$ (30 ml), 1 N HCl (4.5 ml) and $H_2O$ (16 ml). The aqueous phase was neutralized with saturated $NaHCO_3$ and the layers were separated. The aqueous layer was extracted again with $Et_2O$ and the combined $Et_2O$ layers were washed with brine and dried ($Na_2SO_4$). Filtration and solvent removal afforded an oil which was chromatographed

(Flash, Merck $SiO_2$, 30% EtOAc in hexane) to give (E)-7-[4-(4-fluorophenyl)-2-(1-methylethyl)-6-phenyl-3-pyridinyl]-5-hydroxy-3-oxo-6-heptenoic acid, methyl ester (387 mg, 52%) as a light yellow oil.
TLC: $R_f$ 0.31 (40% EtOAc in hexane)

H. (3R, 5S, 6E)-7-[4-(4-fluorophenyl)-2-(1-methylethyl)-6-phenyl-3-pyridinyl]-3,5-dihydroxy-6-heptenoic acid, methyl ester

A solution of (E)-7-[4-(4-fluorophenyl)-2-(1-methylethyl)-6-phenyl-3-pyridinyl]-5-hydroxy-3-oxo-6-heptenoic acid, methyl ester (357 mg, 0.77 mmol) in THF (12 ml) was treated with triethylborane (1.0 M in THF, 1.62 ml, 1.62 mmol). Twenty five milliliters of air was bubbled through the solution and the mixture was stirred at room temperature for 30 minutes. The solution was cooled to -78° C and treated with $NaBH_4$ (29.4 mg, 0.78 mmol) followed by dropwise addition of dry methanol (1.90 ml). After stirring at -78° C for 1.5 hours, the mixture was quenched with 30% $H_2O_2$ (3.5 ml) in $H_2O$ (11 ml). The mixture was kept at -78° C for 15 minutes, then warmed to room temperature and stirred for 30 minutes. The solution was poured into 50% saturated $NaHCO_3$ and extracted with $Et_2O$ (3x). The $Et_2O$ extracts were combined, washed with $H_2O$, saturated $NaHCO_3$ and brine, then dried ($Na_2SO_4$). Filtration and removal of the solvent gave a yellow oil which was chromatographed (Flash, Merck $SiO_2$, 40% EtOAc in hexane) to afford (3R, 5S, 6E)-7-[4-(4-fluorophenyl)-2-(1-methylethyl)-6-phenyl-3-pyridinyl]-3,5-dihydroxy-6-heptenoic acid, methyl ester (320 mg, 89%) as a colorless oily foam.
TLC: $R_f$ 0.17 (40% EtOAc in hexane)

I. (3R*, 5S*, 6E)-7-[4-Fluorophenyl)-2-(1-methyl-ethyl)-6-phenyl-3-pyridinyl]-3,5-dihydroxy-6-heptenoic acid, monolithium salt

A solution of (3R, 5S, 6E)-7-[4-(4-fluorophenyl)-2-(1-methylethyl)-6-phenyl-3-pyridinyl]-3,5-dihydroxy-6-heptenoic acid, methyl ester (290 mg, 0.625 mmol) in dioxane (4.0 ml) and $H_2O$ (4.0 ml) was treated with 1 N LiOH (0.75 ml) at room temperature. After 20 minutes, the solvent was evaporated and the residue was chromatographed on HP-20 (25 mm x 90 mm column) eluting in succession with $H_2O$ (150 ml), 25% $CH_3CN$ in $H_2O$ (100 ml) and 50% $CH_3CN$ in $H_2O$ (200 ml). The desired fractions were pooled and evaporated and the residue was taken up in $H_2O$ and lyophilized to give (3R*, 5S*, 6E)-7-[4-Fluorophenyl)-2-(1-methyl-ethyl)-6-phenyl-3-pyridinyl]-3,5-dihydroxy-6-heptenoic acid, monolithium salt (265 mg, 91%) as a white solid.
TLC: $R_f$ 0.50 (8:1:1, $CH_2Cl_2$:HOAc:MeOH)

## Example 2

(3R*, 5S*, 6E)-7-[4-(4-Fluorophenyl)-2-methyl-6-phenyl-3-pyridinyl]-3,5-dihydroxy-6-heptenoic acid, monolithium salt.

A. α-acetyl-β-(4-fluorophenyl)-δ-oxo-benzenepentanoic acid, ethyl ester

To a mixture of (E)-3-(4-fluorophenyl)-1-phenyl-2-propen-1-one (4.62 gm, 20.4 mmol) (the preparation of which is described in Example 1) and ethyl acetoacetate (5.32 gm, 40.9 mmol) in absolute EtOH (100 ml) was added a solution of EtONa in EtOH. The EtONa solution was prepared by dissolving Na metal (65 mg, 2.8 mmol) in EtOH (10 ml). A precipitate formed after 4 hours of stirring. After 6 hours, the reaction was diluted with a solution of HOAc (170 mg) in $H_2O$ (100 ml), stirred for 5 minutes, then filtered. The solid was washed with $H_2O$ and dried overnight *in vacuo* to afford α-acetyl-β-(4-fluorophenyl)-δ-oxobenzenepentanoic acid, ethyl ester (6.12 gm) in ~ 60-65% purity. This material was used directly in the next reaction.
TLC: $R_f$ 0.15 (20% EtOAc in hexane)

B. 4-(4-fluorophenyl)-2-methyl-6-phenyl-3-pyridinecarboxylic acid, ethyl ester

A mixture of α-acetyl-β-(4-fluorophenyl)-δ-oxobenzenepentanoic acid, ethyl ester (6.12 gm) and hydroxylamine hydrochloride (3.61 gm, 52 mmol) in glacial HOAc (83 ml) was refluxed for 1 hour. The reaction was cooled to room temperature and poured into an ice cold solution of concentrated $NH_4OH$ (115 ml) in $H_2O$ (300 ml). The resulting mixture was extracted twice with $Et_2O$ and the combined $Et_2O$ layers were washed with brine, dried ($Na_2SO_4$), filtered and stripped to yield a dark gummy residue. The residue was chromatographed (Flash, Merck $SiO_2$, 8% EtOAc in hexane) to give 4-(4-fluorophenyl)-2-methyl-6-phenyl-3-pyridine carboxylic acid, ethyl ester which was rechromatographed (8% EtOAc in hexane) to afford pyridine 4-(4-fluorophenyl)-2-methyl-6-phenyl-3-pyridinecarboxylic acid, ethyl ester (1.652 gm, 24% from (E)-3-(4-fluorophenyl)-1-phenyl-2-propen-1-one) in >90% purity.
TLC: $R_f$ 0.35 (20% EtOAc in hexane)

C. 4-(4-fluorophenyl)-2-methyl-6-phenyl-3-pyridinemethanol

A slurry of $LiAlH_4$ (305 mg, 8.04 mmol) in dry $Et_2O$ (30 ml) at 0° C treated with a solution of 4-(4-fluorophenyl)-2-methyl-6-phenyl-3-pyridinecarboxylic acid, ethyl ester (1.587 gm, 4.73 mmol) in $Et_2O$ (4 ml). After 50 minutes, the reaction was quenched and diluted with $H_2O$ and the solution was adjusted to neutral pH with 10% HCl. EtOAc was added to dissolve suspended product into the $Et_2O$ layer and the mixture was subsequently extracted three times with EtOAc. The combined organic layers were washed with $H_2O$ and brine, then dried ($Na_2SO_4$), filtered and stripped to give a yellow solid. Recrystallization of the solid from hot hexane/acetone afforded 4-(4-fluorophenyl)-2-methyl-6-phenyl-3-pyridinemethanol (916 mg) as a white solid. An additional 283 mg of 4-(4-fluorophenyl)-2-methyl-6-phenyl-3-pyridinemethanol was obtained by evaporation of the mother liquor followed by recrystallization of the residue from hot hexane/EtOAc, giving a total of 1.199 gm (86%) product.
m.p. 181-183° C
TLC: $R_f$ 0.29 (40% EtOAc in hexane)

D. 4-(4-fluorophenyl)-2-methyl-6-phenyl-3-pyridinecarboxaldehyde

A solution of Dess-Martin periodinane (1.04 gm, 2.45 mmol) in dry $CH_2Cl_2$ (12 ml) was treated with t-butanol (182 mg, 2.46 mmol) and stirred at room temperature for 15 minutes. A slurry of 4-(4-fluorophenyl)-2-methyl-6-phenyl-3-pyridinemethanol (555 mg, 1.89 mmol) in $CH_2Cl_2$ (10 ml) and $CH_3CN$ (2 ml) was then added and stirring was continued for 25 minutes. The mixture was diluted with $Et_2O$ (50 ml) and poured into 1 N NaOH (25 ml) and stirred for 5 minutes. The phases were separated and the organic layer was washed successfully with 1 N NaOH, $H_2O$, and brine, then dried ($Na_2SO_4$). Filtration and removal of the solvent afforded a solid. The solid was dissolved in hot hexane/EtOAc and the resulting solution was cooled to 0° C, filtered and stripped to give a residue which was chromatographed (Flash, Merck $SiO_2$, 20% EtOAc in hexane). 4-(4-fluorophenyl)-2-methyl-6-phenyl-3-pyridinecarboxaldehyde was obtained as a white solid (458 mg, 83%). Analytically pure material was obtained by recrystallization from hot hexane.
m.p. 119.5-120.5° C
TLC: $R_f$ 0.37 (20% EtOAc in hexane)

E. (E)-3-[4-(4-fluorophenyl)-2-methyl-6-phenyl-3-pyridinyl]-2-propenal

n-BuLi (1.6 M in hexanes, 1.52 ml, 2.43 mmol) was added to a solution of cis-1-ethoxy-2-(tri-n-butylstannyl) ethylene (1.06 gm, 2.94 mmol) in dry THF (7.5 ml) at -78° C. The mixture was stirred for 1 1/4 hours, then cooled to -100° C (liquid $N_2$/methanol) and treated with a solution of 4-(4-fluorophenyl)-2-methyl-6-phenyl-3-pyridinecarboxaldehyde (707 mg, 2.43 mmol) in THF (3.0 ml). Forty-five minutes after the addition, the temperature was raised to -78° C and the mixture was stirred for an additional 50 minutes. The solution was then stirred in an ice bath for 5 minutes and subsequently quenched with $H_2O$ (8 ml) and 10% HCl (8 ml). After stirring at room temperature for 1.5 hours, the mixture was made basic with 10% NaOH (10 ml) and extracted twice with $Et_2O$. The combined $Et_2O$ layers were washed with $H_2O$ and brine, dried ($Na_2SO_4$), filtered and stripped to yield a solid. The solid was washed with cold $Et_2O$. The $Et_2O$ washings were stripped and triturated with cold hexane to give additional solid, which was pooled with the above solid to give 586 mg (76%) (E)-3-[4-(4-fluorophenyl)-2-methyl-6-phenyl-3-pyridinyl]-2-propenal. Analytically pure material was obtained by recrystallization from hot hexane/EtOAc.

m.p. 160.5-163° C
TLC: $R_f$ 0.17 (20% EtOAc in hexane)

F. (E)-7-[4-(4-fluorophenyl)-2-methyl-6-phenyl-3-pyridinyl]-5-hydroxy-3-oxo-6-heptenoic acid, methyl ester

Methyl acetoacetate (324 mg, 2.79 mmol) was added dropwise to a slurry of NaH (60% in mineral oil, 111.7 mg, 2.79 mmol) in dry THF (7 ml) at 0° C. After 15 minutes, n-BuLi (1.5 M in hexanes, 1.46 ml, 2.19 mmol) was added and the mixture was stirred an additional 15 minutes. Addition of a solution of (E)-3-[4-(4-fluorophenyl)-2-methyl-6-phenyl-3-pyridinyl]-2-propenal (633 mg, 1.99 mmol) in THF (7 ml) to the reaction mixture resulted in the formation of a bright red solution. Twenty minutes after the addition, the solution was poured into a biphasic mixture of $Et_2O$ (40 ml) and 1 N HCl (5.5 ml) in $H_2O$ (20 ml). The aqueous layer was made basic with saturated $NaHCO_3$ and the phases were separated. The aqueous layer was extracted again with $Et_2O$, neutralized with 1 N HCl and extracted once more. The combined $Et_2O$ layers were washed with brine, dried ($Na_2SO_4$), filtered and stripped to yield an oil. Flash chromatography (Merck $SiO_2$, 1:1 EtOAc:hexane) afforded (E)-7-[4-(4-fluorophenyl)-2-methyl-6-phenyl-3-pyridinyl]-5-hydroxy-3-oxo-6-heptenoic acid, methyl ester (251 mg, 29%) as a yellow oil
TLC: $R_f$ 0.16 (40% EtOAc in hexane)

G. (3R*, 5S*, 6E)-7-[4-(4-fluorophenyl)-2-methyl-6-phenyl-3-pyridinyl]-3,5-dihydroxy-6-heptenoic acid, methyl ester

A solution of (E)-7-[4-(4-fluorophenyl)-2-methyl-6-phenyl-3-pyridinyl]-5-hydroxy-3-oxo-6-heptenoic acid, methyl ester (235 mg, 0.54 mmol) in THF (8 ml) was treated with triethylborane (1.0 M in THF, 1.13 ml, 1.13 mmol). Twenty milliliters of air was bubbled through the solution and the mixture was stirred at room temperature for 25 minutes. The solution was cooled to -78° C and treated with $NaBH_4$ (20.7 mg, 0.55 mmol) followed by dropwise addition of dry methanol (1.25 ml). After stirring at -78° C for 1.5 hours, the mixture was quenched with 30% $H_2O_2$ (3.5 ml) in $H_2O$ (8 ml). The mixture was kept at -78° C for 15 minutes, then warmed to room temperature and stirred for 30 minutes. The solution was poured into 50% saturated $NaHCO_3$ and extracted with $Et_2O$ (3x). The combined $Et_2O$ extracts were washed with $H_2O$, saturated $NaHCO_3$ and brine, then dried ($Na_2SO_4$), filtered and stripped. Purification of the resulting oil (Flash, Merck $SiO_2$, 70% EtOAc/30% hexane) gave (3R*, 5S*, 6E)-7-[4-(4-fluorophenyl)-2-methyl-6-phenyl-3-pyridinyl]-3,5-dihydroxy-6-heptenoic acid, methyl ester (188 mg, 80%) as a colorless oily foam.
TLC: $R_f$ 0.17 (60% EtOAc/40% hexane)

H. (3R*, 5S*, 6E)-7-[4-(4-Fluorophenyl)-2-methyl-6-phenyl-3-pyridinyl]-3,5-dihydroxy-6-heptenoic acid, monolithium salt

A solution of (3R*, 5S*, 6E)-7-[4-(4-fluorophenyl)-2-methyl-6-phenyl-3-pyridinyl]-3,5-dihydroxy-6-heptenoic acid, methyl ester (186 mg, 0.47 mmol) in dioxane (3.0 ml) and $H_2O$ (3.0 ml) was treated with 1 N LiOH (0.51 ml) at room temperature. After 25 minutes, the solvent was evaporated and the residue was chromatographed on HP-20 (25 mm x 90 mm column) eluting in succession with $H_2O$ (150 ml), 25% $CH_3CN$ in $H_2O$ (100 ml), and 50% $CH_3CN$ in $H_2O$ (200 ml). The desired fractions were pooled and evaporated and the residue was taken up in $H_2O$ and lyophilized to give (3R*, 5S*, 6E)-7-[4-(4-Fluorophenyl)-2-methyl-6-phenyl-3-pyridinyl]-3,5-dihydroxy-6-heptenoic acid, monolithium salt (174 mg, 93%) as a white solid.
TLC: $R_f$ 0.29 (8:1:1, $CH_2Cl_2$: HOAc:MeOH)

## Example 3

(3R*, 5S*, 6E)-7-[2-(4-Fluorophenyl)-4-(1-methylethyl)-6-phenyl-3-pyridinyl]-3,5-dihydroxy-6-heptenoic acid, monolithium salt.

A. 3-hydroxy-4-methyl-1-phenyl-1-pentanone

Acetophenone (11.433 gm, 95 mmol) was added dropwise over a five minute period to a solution of lithium bis(trimethylsilyl)amide (1.0 M in THF, 100 ml, 100 mmol) in THF (50 ml) kept at -78° C. After 30 minutes, isobutyraldehyde (6.87 gm, 95 mmol) was added dropwise and stirring was continued for an additional hour. The mixture was quenched with saturated $NH_4Cl$ (100 ml), warmed to room temperature, diluted with $H_2O$ and extracted 2x with $Et_2O$. The combined $Et_2O$ extracts were washed with brine, dried ($Na_2SO_4$), filtered and stripped to yield an oil. The oil was distilled (P= 1.5 mm) and the fraction boiling between 120-124° C was chromatographed (flash, Merck $SiO_2$, 15% EtOAc in hexane) to give the desired 3-hydroxy-4-methyl-1-phenyl-1-pentanone as a colorless liquid (9.13 gm, 50%)
TLC: $R_f$ 0.20 (20% EtOAc in hexane)

B. (E)-4-methyl-1-phenyl-2-penten-1-one

A mixture of 3-hydroxy-4-methyl-1-phenyl-1-pentanone (8.400 gm, 43.7 mmol) and p-toluenesulfonic acid (820 mg, 4.3 mmol) in benzene (210 ml) was refluxed in a Dean-Stark apparatus for 10 minutes. The solution was cooled, diluted with $Et_2O$, and washed with saturated $NaHCO_3$ and brine. Filtration and removal of the solvent afforded a yellow oil which was subsequently distilled (P= 2.0 mm, 97-100° C) to give the (E)-4-methyl-1-phenyl-2-penten-1-one (6.94 gm) as a colorless liquid. $^1$H-NMR analysis showed the distillate to be 78% the desired product and 22% the $\beta$, $\delta$-unsaturated isomer. The material was used directly for the next reaction.
TLC: $R_f$ 0.35 (10% EtOAc in hexane)

C. $\alpha$-(4-fluorobenzoyl)-$\beta$-(1-methylethyl)-$\delta$-oxobenzenepentanoic, ethyl ester

A mixture of (E)-4-methyl-1-phenyl-2-penten-1-one (6.32 gm crude, 4.93 gm pure, 28.3 mmol) and ethyl 4-fluorobenzoyl acetate (5.95 gm, 28.3 mmol) in EtOH (120 ml) was treated with a solution of EtONa (3.3 mmol) in EtOH (10 ml) at room temperature. After stirring 3.5 hours, the solution was treated with 73 mg ofMeONa. Stirring was continued for an additional 3 hours. The solution was quenched with saturated $NH_4Cl$ (80 ml), diluted with $H_2O$, and extracted 3x with $Et_2O$. The combined $Et_2O$ extracts were washed with brine and subsequently dried ($Na_2SO_4$), filtered and stripped to give a yellow residue. The residue was purified by flash chromatography (Merck $SiO_2$, 50 x 260 mm column, 3% acetone in hexane) to give a diastereomeric mixture of the $\alpha$-(4-fluorobenzoyl)-$\beta$-(1-methylethyl)-6-oxobenzenepentanoic, ethyl ester as a viscous oil (6.99 gm, 64%).
TLC: $R_f$ 0.04 (5% acetone in hexane)

D. 2-(4-fluorophenyl)-4-(1-methylethyl)-6-phenyl-3-pyridinecarboxylic acid, ethyl ester

A mixture of $\alpha$-(4-fluorobenzoyl)-$\beta$-(1-methylethyl)-$\delta$-oxobenzenepentanoic, ethyl ester (6.967 gm, 18.12 mmol) and hydroxylamine hydrochloride (3.778 gm, 54.4 mmol) in glacial HOAc (82 ml) was refluxed under argon for 2 hours. The cooled solution was added to an ice cold mixture of concentrated $NH_4OH$ (120 ml) in $H_2O$ (200 ml). The resulting mixture was extracted twice with $Et_2O$ and the combined $Et_2O$ extracts were washed with brine, dried ($Na_2SO_4$), filtered and stripped to yield a orange-red oil. Chromatographic purification of the oil (flash, Merck $SiO_2$, 3% EtOAc in hexane) gave 2-(4-fluorophenyl)-4-(1-methylethyl)-6-phenyl-3-pyridinecarboxylic acid, ethyl ester as a colorless viscous oil (3.482 gm, 53%)
TLC: $R_f$ 0.39 (10% EtOAc in hexane)

E. 2-(4-fluorophenyl)-4-(1-methylethyl)-6-phenyl-3-pyridinemethanol

To a slurry of $LiAlH_4$ (1.457 gm, 38.4 mmol) in dry THF at 0° C was added a solution of 2-(4-fluorophenyl)-4-(1-methylethyl)-6-phenyl-3-pyridinecarboxylic acid, ethyl ester (3.37 gm, 9.27 mmol) in THF (7 ml). After 4.5 hours, the flask was charged with an additional 0.88 gm $LiAlH_4$ and stirring was continued for 2 more hours. The mixture was quenched with $H_2O$ and diluted with $H_2O$ and EtOAc. The phases were shaken and separated, and the aqueous layer was extracted again with EtOAc and $Et_2O$. The pooled

organic layers were washed with $H_2O$ and brine, then dried ($Na_2SO_4$), filtered and stripped to give a white solid. The solid was recrystallized from hot EtOAc/hexane to afford 2-(4-fluorophenyl)-4-(1-methylethyl)-6-phenyl-3-pyridinemethanol as white crystals. The mother liquor was stripped and recrystallized again from hot EtOAc/hexane to give a total of 2.684 gm (90%) desired product.
m.p. 172.5-173.5° C
TLC: $R_f$ 0.17 (20% EtOAc in hexane)

F. 2-(4-fluorophenyl)-4-(1-methylethyl)-6-phenyl-3-pyridinecarboxaldehyde

t-Butanol (175 mg, 2.35 mmol) was added to a stirring solution of Dess-Martin periodinane (1.005 gm, 2.37 mmol) in dry $CH_3CN$ (15 ml). After 15 minutes, a solution of 2-(4-fluorophenyl)-4-(1-methylethyl)-6-phenyl-3-pyridinemethanol (582 mg, 1.81 mmol) in $CH_3CN$ (9 ml) and $CH_2Cl_2$ (3 ml) was added to the mixture and stirring was continued for 40 minutes. The mixture was diluted with $Et_2O$ and quenched with 1 N NaOH (25 ml). After 25 minutes of rapid mixing, the biphasic mixture was separated and the aqueous layer was extracted with $Et_2O$. The combined organic layers were washed in succession with 1 N NaOH, $H_2O$, and brine, then dried ($Na_2SO_4$), filtered and stripped to yield a solid residue. The residue was chromatographed (Merck $SiO_2$, 30% hexane in $CH_2Cl_2$) to give 2-(4-fluorophenyl)-4-(1-methylethyl)-6-phenyl-3-pyridinecarboxaldehyde as a white solid (532 mg, 92%). An analytical sample was obtained by recrystallization from a minimum amount of hot hexane.
m.p. 115.5-117.0° C
TLC: $R_f$ 0.43 (20% EtOAc in hexane)

G. (E)-3-[2-(4-fluorophenyl)-4-(1-methylethyl)-6-phenyl-3-pyridinyl]-2-propenal

n-BuLi (1.6 M in hexane, 554 ul, 0.83 mmol) was added to a solution of cis-1-ethoxy-2-(tri-n-butylstannyl)ethylene (333 mg, 0.92 mmol) in dry THF (4 ml) at -78° C. The mixture was stirred at -78° C for one hour, then cooled to -100° C (liquid $N_2$/MeOH) and treated with a solution of 2-(4-fluorophenyl)-4-(1-methylethyl)-6-phenyl-3-pyridinecarboxaldehyde (221 mg, 0.69 mmol) in THF (2 ml). One hour after the addition, the temperature was raised to -78° C and the solution was stirred for an additional one hour. TLC analysis indicated that the reaction was incomplete so an additional amount of the vinyl anion reagent (generated from 317 mg vinyl tin reagent, 370 ul of 1.6 M n-BuLi and 4 ml THF) was added *via canula* at -78° C the resulting solution stirred an additional 45 minutes. The mixture was warmed to 0° C, stirred for 15 minutes, then quenched with $H_2O$ (6 ml) and 10% HCl (6 ml). After one hour of vigorous mixing, the solution was made basic with 10% NaOH (12 ml) and extracted twice with $Et_2O$. The combined $Et_2O$ layers were washed with $H_2O$ and brine, dried ($Na_2SO_4$), filtered and stripped to yield a yellow oil.
Chromatographic purification (flash, Merck $SiO_2$, 40% hexane in $CH_2Cl_2$ followed by straight $CH_2Cl_2$ afforded (E)-3-[2-(4-fluorophenyl)-4-(1-methylethyl)-6-phenyl-3-pyridinyl]-2-propenal (141 mg, 59%) as a light yellow foam.
TLC: $R_f$ 0.24 (20% EtOAc in hexane)

H. (E)-7-[2-(4-fluorophenyl)-4-(1-methylethyl)-6-phenyl-3-pyridinyl]-5-hydroxy-3-oxo-6-heptenoic acid, methyl ester

Methyl acetoacetate (160 mg, 1.38 mmol) was dropwise to a slurry of NaH (60% in mineral oil, 55.1 mg, 1.38 mmol) in dry THF (3.5 ml) at 0° C. After 15 minutes, n-BuLi (1.5 M in hexane, 830 ul, 1.25 mmol) was added and the mixture was stirred an additional 15 minutes. The bright yellow solution was cooled to -78° C and treated with a solution of (E)-3-[2-(4-fluorophenyl)-4-(1-methylethyl)-6-phenyl-3-pyridinyl]-2-propenal (317 mg, 0.92 mmol) in THF (2 ml). Stirring continued for 20 minutes, after which time the reaction was warmed to 0° C and stirred for an additional 20 minutes. The yellow-orange solution was then added to a biphasic mixture of 1 N HCl (3 ml), $H_2O$ (15 ml), and $Et_2O$ (30 ml). The aqueous layer was made slightly basic with saturated $NaHCO_3$ and the phases were separated. The aqueous layer was diluted with saturated $NH_4Cl$ and was extracted with $Et_2O$ once again. The combined organic layers were washed with brine and dried ($Na_2SO_4$). Filtration and removal of the solvent afforded an oil which was chromatographed (flash, Merck $SiO_2$, 35% EtOAc in hexane) to give (E)-7-[2-(4-fluorophenyl)-4-(1-methylethyl)-6-phenyl-3-pyridinyl]-5-hydroxy-3-oxo-6-heptenoic acid, methyl ester as an oily solid (187 mg, 44%).

TLC: $R_f$ 0.21 (40% EtOAc in hexane)

I. (3R*, 5S*, 6E)-7-[2-(4-fluorophenyl)-4-(1-methylethyl)-6-phenyl-3-pyridinyl]-3,5-dihydroxy-6-heptenoic acid, methyl ester

A solution of (E)-7-[2-(4-fluorophenyl)-4-(1-methylethyl)-6-phenyl-3-pyridinyl]-5-hydroxy-3-oxo-6-heptenoic acid, methyl ester (182 mg, 0.39 mmol) in THF (5.5 ml) was treated with triethylborane (1.0 N in THF, 830 ul, 0.83 mmol). Twenty milliliters of air was bubbled through the solution and the mixture was stirred at room temperature for 30 minutes. The solution was cooled to -78° C and treated with $NaBH_4$ (15.0 mg, 0.40 mmol) followed by dropwise addition of dry methanol (0.9 ml). After stirring at -78° C for 1.75 hours, the mixture was quenched with 30% $H_2O_2$ (2.8 ml) in $H_2O$ (6 ml). The mixture was kept at -78° C for 15 minutes, then warmed to room temperature and stirred for 45 minutes. The solution was poured into 50% saturated $NaHCO_3$ and extracted with $Et_2O$ (2x). The combined $Et_2O$ extracts were washed with saturated $NaHCO_3$, $H_2O$, and brine, then dried ($Na_2SO_4$), filtered and stripped. Purification of the resulting oil (flash, Merck $SiO_2$, 50% EtOAc in hexane) gave (3R*, 5S*, 6E)-7-[2-(4-fluorophenyl)-4-(1-methylethyl)-6-phenyl-3-pyridinyl]-3,5-dihydroxy-6-heptenoic acid, methyl ester (157 mg, 86%) as a colorless oily foam.
TLC: $R_f$ 0.27 (60% EtOAc/40% hexane)

J. (3R*, 5S*, 6E)-7-[2-(4-Fluorophenyl)-4-(1-methyl-ethyl)-6-phenyl-3-pyridinyl]-3,5-dihydroxy-6-heptenoic acid, monolithium salt

A solution of (3R*, 5S*, 6E)-7-[2-(4-fluorophenyl)-4-(1-methylethyl)-6-phenyl-3-pyridinyl]-3,5-dihydroxy-6-heptenoic acid, methyl ester (152 mg, 0.33 mmol) in dioxane (3 ml) and $H_2O$ (3 ml) was treated with 1 N LiOH (360 ul) at room temperature. After 30 minutes, the solvent was evaporated and the residue was chromatographed on HP-20 eluting in succession with $H_2O$ (150 ml), 25% $CH_3CN$ in $H_2O$ (100 ml), and 50% $CH_3CN$ in $H_2O$ (200 ml). The desired fractions were pooled and evaporated and the residue was taken up in $H_2O$ and lyophilized to give (3R*, 5S*, 6E)-7-[2-(4-Fluorophenyl)-4-(1-methylethyl)-6-phenyl-3-pyridinyl]-3,5-dihydroxy-6-heptenoic acid, monolithium salt (147 mg, 94%) as a white solid.
TLC: $R_f$ 0.22 (20:1:1, $CH_2Cl_2$:HOAc:MeOH)

## Example 4

(S)-4-[[[4-(4-Fluorophenyl)-2-(1-methylethyl)-6-phenyl-3-pyridinyl]ethynyl]hydroxyphosphinyl]-3-hydroxybutanoic acid, dilithium salt.

A. 3-(2,2-dibromoethenyl)-4-(4-fluorophenyl)-2-(1-methylethyl)-6-phenylpyridine

A solution of carbon tetrabromide (2.204 gm, 6.65 mmol) in $CH_2Cl_2$ (8 ml) was added over a 12 minute period to a cold (-12° C) solution of 4-(4-fluorophenyl)-2-(1-methylethyl)-6-phenyl-3-pyridinecarboxaldehyde (1.415 gm, 4.43 mmol) (the preparation of which is described in Example 1) and triphenylphosphine (3.488 gm, 13.3 mmol) in $CH_2Cl_2$ (25 ml). After the addition was complete, the cooling bath was removed and the mixture was stirred for 20 minutes. The solution was quenched with saturated $NaHCO_3$ and extracted 3x with $CH_2Cl_2$. The combined organic layers were washed with saturated $NaHCO_3$, dried ($Na_2SO_4$), filtered and stripped to give a yellow oil. The oil was chromatographed (flash, Merck $SiO_2$, 40% $CH_2Cl_2$ in hexane) to give 3-(2,2-dibromoethenyl)-4-(4-fluorophenyl)-2-(1-methylethyl)-6-phenylpyridine as a colorless foam (2.152 gm, 100%).
TLC: $R_f$ 0.53 (20% EtOAc in hexane)

B. 3-ethynyl-4-(4-fluorophenyl)-2-(1-methylethyl)-6-phenylpyridine

A solution of 3-(2,2-dibromoethenyl)-4-(4-fluorophenyl)-2-(1-methylethyl)-6-phenylpyridine (2.105 gm,

4.43 mmol) in dry THF (23 ml) was cooled to -78°C and treated with n-BuLi (1.5 M in hexane, 5.95 ml, 8.93 mmol) over a 4 minute period. After stirring at -78°C for 1.25 hours, the deep purple solution was quenched with saturated $NH_4Cl$, warmed to room temperature, diluted with $H_2O$, and extracted 2x with $Et_2O$. The combined $Et_2O$ extracts were washed with brine, dried ($Na_2SO_4$), filtered and stripped to give a yellow solid. The solid was recrystallized from a minimum amount of hot hexane to afford analytically 3-ethynyl-4-(4-fluorophenyl)-2-(1-methylethyl)-6-phenylpyridine as white needles. The mother liquor was stripped and recrystallized again from hot hexane to give additional product (total yield 1.199 gm, 86%)

TLC: $R_f$ 0.31 (20% $CH_2Cl_2$ in hexane)

C. (S)-3-[[(1,1-dimethylethyl)diphenylsilyl]oxy]-4-[[[4-(4-fluorophenyl)-2-(1-methylethyl)-6-phenyl-3-pyridinyl]-ethynyl]methoxyphosphinyl]butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (4.501 gm, 7.123 mmol) was partitioned between EtOAc and 5% $KHSO_4$. The EtOAc layer was washed 3x with 5% $KHSO_4$, then with brine, then dried ($Na_2SO_4$), filtered and stripped to give a colorless oil (phosphinic acid monomethyl ester). The oil was dissolved in dry $CH_2Cl_2$ (15 ml) and treated with N,N-diethyltrimethylsilylamine (2.70 ml, 2.07 gm, 14.25 mmol). After stirring at room temperature for one hour, the solvent was removed *in vacuo* and the residue was azeotroped with dry benzene (20 ml). The residue was re-dissolved in dry $CH_2Cl_2$ (15 ml), cooled to -12°C and treated with 2 drops DMF and oxalyl chloride (700 ul, 1.018 gm, 8.02 mmol). After 15 minutes, the solution was warmed to room temperature and stirred for an additional 45 minutes. The solvent was stripped and the yellow residue (phosphinylchloridate) was azeotroped with benzene (20 ml) and dried *in vacuo* (oil pump) for 30 minutes.

Meanwhile, a solution of acetylene 3 (1.321 gm, 4.19 mmol) in THF (10 ml) at -78°C was treated with n-BuLi (1.5 M in hexane, 2.8 ml, 4.2 mmol) and the resulting deep green mixture was stirred for 30 minutes. The acetylenic anion solution was added dropwise *via canula* over a 20 minute period to a -78°C solution of the phosphinylchloridate in THF (9 ml). The resulting yellow-brown mixture was stirred at -78°C for 40 minutes, then quenched with 50% saturated $NH_4Cl$. The solution was warmed to room temperature, diluted with $H_2O$, and poured into saturated $NaHCO_3$. The aqueous phase was extracted 3x with $Et_2O$. The combined $Et_2O$ layers were washed with brine, dried ($Na_2SO_4$), filtered and stripped. The residue was chromatographed (flash, Merck $SiO_2$, 30% EtOAc in hexane) to afford (S)-3-[[(1,1-dimethylethyl)-diphenylsilyl]oxy]-4-[[[4-(4-fluorophenyl)-2-(1-methylethyl)-6-phenyl-3-pyridinyl]ethynyl]methoxyphosphinyl] butanoic acid, methyl ester as a colorless foam (2.316 gm, 74%).

TLC: $R_f$ 0.30 (40% EtOAc in hexane)

D. (S)-4-[[[4-(4-fluorophenyl)-2-(1-methylethyl)-6-phenyl-3-pyridinyl]-ethynyl]methoxy-phosphinyl]-3-hydroxybutanoic acid, methyl ester

A mixture of (S)-3-[[(1,1-dimethylethyl)diphenylsilyl]oxy]-4-[[[4-(4-fluorophenyl)-2-(1-methylethyl)-6-phenyl-3-pyridinyl]ethynyl]methoxyphosphinyl] butanoic acid, methyl ester (800 mg, 1.07 mmol), tetra-n-butylammonium fluoride (1.0 M in THF, 3.21 ml, 3.21 mmol), and HOAc (325 mg, 5.41 mmol) in THF (12 ml) was stirred at room temperature for 17 hours. The solution was cooled to 0°C, diluted with $H_2O$, and extracted twice with EtOAc. The pooled EtOAc layers were washed with saturated $NaHCO_3$ and brine. The organic layer was then washed twice 5% $KHSO_4$, then with $H_2O$ and brine. Drying ($Na_2SO_4$), followed by filtration and removal of the solvent afforded a yellow oil. The oil was dissolved in $Et_2O$ (10 ml) and treated with excess diazomethane for 20 minutes. The excess diazomethane was destroyed by the addition of HOAc and the solvent was removed *in vacuo*. The residue was chromatographed (flash, Merck $SiO_2$, 50 x 70 mm column 35% acetone in hexane (650 ml) followed by 1:1 acetone:hexane) to afford (S)-4-[[[4-(4-fluorophenyl)-2-(1-methylethyl)-6-phenyl-3-pyridinyl]-ethynyl]methoxyphosphinyl]-3-hydroxybutanoic acid, methyl ester (463 mg, 85%) as a colorless foam.

TLC: $R_f$ 0.38 (1:1 acetone:hexane)

E. (S)-4-[[[4-(4-Fluorophenyl)-2-(1-methylethyl)-6-phenyl-3-pyridinyl]ethynyl]hydroxyphosphinyl]-3-hydroxybutanoic acid, dilithium salt

A solution of (S)-4-[[[4-(4-fluorophenyl)-2-(1-methylethyl)-6-phenyl-3-pyridinyl]-ethynyl]-

methoxyphosphinyl]-3-hydroxybutanoic acid, methyl ester (446 mg, 0.875 mmol) in dioxane (7 ml) was treated with 1 N LiOH (2.63 ml, 2.63 mmol) at room temperature and the mixture was subseqently heated at 55 °C under argon for one hour. The solvent was evaporated and the residue was chromatographed on HP-20 eluting in succession with $H_2O$ (200 ml), 25% MeOH in $H_2O$ (100 ml), 50% MeOH in $H_2O$ (200 ml), and 50% $CH_3CN$ in $H_2O$ (100 ml). The desired fractions were pooled and evaporated and the residue was taken up in $H_2O$ and lyophilized to give (S)-4-[[[4-(4-Fluorophenyl)-2-(1-methylethyl)-6-phenyl-3-pyridinyl]ethynyl]-hydroxyphosphinyl]-3-hydroxybutanoic acid, dilithium salt (416 mg, 94%) as a white solid.
TLC: $R_f$ 0.46 (7:2:1,i-PrOH,$NH_4OH$,$H_2O$)

## Example 5

### (S)-4-[[[2-(4-Fluorophenyl)-4-(1-methylethyl)-3-quinolinyl]ethynyl]hydroxyphosphinyl]-3-hydroxybutanoic acid, dilithium salt.

#### A. 1-(2-aminophenyl)-2-methyl-1-propanone

A solution of anthranilonitrile (8.507 gm, 72 mmol) in dry $Et_2O$ (20 ml) was added to i-propylmagnesium chloride (2.0 M in $Et_2O$, 100 ml, 200 mmol) in dry $Et_2O$ (30 ml) at 0 °C over a 15 minute period. After the addition was complete, the mixture was warmed to room temperature and stirred for 4.5 hours. The solution was cooled to 0 °C, quenched with 10% HCl (150 ml), then stirred for 25 minutes. The aqueous layer was made basic with solid NaOH (25 gm) and then extracted with $Et_2O$ (3x). The combined $Et_2O$ extracts were washed with brine, then dried ($Na_2SO_4$), filtered and stripped to give a yellow oil. Flash Chromatography of the oil (Merck $SiO_2$, 15% EtOAc in hexane) afforded 1-(2-aminophenyl)-2-methyl-1-propanone (10.916 gm, 93%) as a golden yellow oil.
TLC: $R_f$ 0.32 (20% EtOAc in hexane)

#### B. 2-(4-fluorophenyl)-4-(1-methylethyl)-3-quinoline-carboxylic acid, ethyl ester.

A mixture of 1-(2-aminophenyl)-2-methyl-1-propanone (5.526 gm, 33.9 mmol), ethyl p-fluorobenzoylacetate (7.12 gm, 33.9 mmol) and concentrated $H_2SO_4$ (0.34 ml) in glacial HOAc (34 ml) was refluxed for 22 hours. The reaction mixture was cooled to room temperature and poured into an ice-cold solution of concentrated $NH_4OH$ (48 ml) in $H_2O$ (120 ml). The resulting mixture was extracted twice with $Et_2O$ and the combined $Et_2O$ layers were washed with $H_2O$ and brine, then dried ($Na_2SO_4$), filtered and stripped to yield a cloudy brown oil. Flash chromatography (Merck $SiO_2$, 10% EtOAc in hexane) afforded impure 2-(4-fluorophenyl)-4-(1-methylethyl)-3-quinoline-carboxylic acid, ethyl ester as an oil. Most of the volatile impurities were distilled off under an oil pump vacuum (bath temp. 85 °C), leaving a yellow oil (5.096 gm) which was 77% by weight 2-(4-fluorophenyl)-4-(1-methylethyl)-3-quinolinecarboxylic acid, ethyl ester (34% effective yield).
TLC: $R_f$ 0.36 (20% EtOAc in hexane)

#### C. 2-(4-fluorophenyl)-4-(1-methylethyl)-3-quinoline-methanol

A solution of ester 2-(4-fluorophenyl)-4-(1-methylethyl)-3-quinoline-carboxylic acid, ethyl ester (5.00 gm crude, 3.85 gm pure, 114 mmol) in dry $Et_2O$ (5 ml) was added to a slurry of $LiAlH_4$ (1.41 gm, 37.1 mmol) in $Et_2O$ (70 ml) at 0 °C. After 6.5 hours, the solution was quenched and diluted with $H_2O$ and the aqueous layer was extracted with $Et_2O$ (3x). The combined $Et_2O$ extracts were washed with brine, then dried ($Na_2SO_4$), filtered and stripped to yield a semi-solid yellow residue. The residue was crystallized from hot hexane/EtOAc to give 2-(4-fluorophenyl)-4-(1-methylethyl)-3-quinolinemethanol as white needles. The mother liquor was stripped, dissolved in $CH_2Cl_2$ (50 ml) and treated with N-chlorosuccinamide (700 mg). After 15 minutes, the solution was concentrated, diluted with $Et_2O$ and washed first with saturated $NaHCO_3$, then with a solution of $Na_2SO_3$ in saturated $NaHCO_3$. The solvent was removed and the residue was chromatographed (flash, Merck $SiO_2$, 30% EtOAc in hexane) to give additional 2-(4-fluorophenyl)-4-(1-methylethyl)-3-quinoline-methanol as an impure orange semi-solid. Recrystallization from hot hexane/EtOAc gave the desired product as white needles. The pooled solids were recrystallized once again to give analytically pure 2-(4-fluorophenyl)-4-(1-methylethyl)-3-quinolinemethanol (1.17 gm, 35%).
m.p. 208 - 210°C
TLC: $R_f$ 0.42 (40% EtOAc in hexane)

D. 2-(4-fluorophenyl)-4-(1-methylethyl)-3-quinolinecarboxaldehyde

Dess-Martin periodinane (1.002 gm, 2.36 mmol) was dissolved in dry $CH_2Cl_2$ (11 ml), treated with t-butanol (175 mg, 2.36 mmol) and the resulting mixture was stirred at room temperature for 15 minutes. A slurry of 2-(4-fluorophenyl)-4-(1-methylethyl)-3-quinolinemethanol (505 mg, 1.71 mmol) in $CH_2Cl_2$ (8 ml) was then added. After 1.5 hours, the solution was diluted with $Et_2O$ and 1 N NaOH (25 ml), then stirred an additional 10 minutes. The aqueous layer was separated and extracted once with $Et_2O$. The combined $Et_2O$ layers were washed with saturated $NaHCO_3$ and brine, then dried ($Na_2SO_4$), filtered and stripped to give an oil. Chromatographic purification (Flash, Merck $SiO_2$, 20% EtOAc in hexane) afforded aldehyde 2-(4-fluorophenyl)-4-(1-methylethyl)-3-quinolinecarboxaldehyde (430 mg, 86%) as a colorless oil which solidified in the freezer.
TLC: $R_f$ 0.40 (20% EtOAc in hexane)

E. 3-(2,2-dibromoethenyl)-2-(4-fluorophenyl)-4-(1-methylethyl)quinoline

A solution of carbon tetrabromide (2.958 gm, 8.92 mmol) in $CH_2Cl_2$ (10 ml) was added over a 15 minute period to a cold (-10°C) solution of 2-(4-fluorophenyl)-4-(1-methylethyl)-3-quinolinecarboxaldehyde (1.744 gm, 5.95 mmol) (the preparation of which is described in Example 2) and triphenylphosphine (4.680 gm, 17.84 mmol) in $CH_2Cl_2$ (34 ml). After the addition was complete, the mixture was stirred for 20 minutes then quenched with saturated $NaHCO_3$ and extracted 3x with $CH_2Cl_2$. The combined organic layers were washed with brine, dried ($Na_2SO_4$), filtered and stripped to give a yellow-orange oil. The oil was chromatographed (flash, Merck $SiO_2$, 30% $CH_2Cl_2$ in hexane followed by 1:1 $CH_2Cl_2$ in hexane followed by straight $CH_2Cl_2$) to give 3-(2,2-dibromoethenyl)-2-(4-fluorophenyl)-4-(1-methylethyl)quinoline as a colorless foam (2.572 gm, 96%)
TLC: $R_f$ 0.37 (20% EtOAc in hexane)

F. 3-ethynyl-2-(4-fluorophenyl)-4-(1-methylethyl)quinoline

A solution of 3-(2,2-dibromoethenyl)-2-(4-fluorophenyl)-4-(1-methylethyl)quinoline (2.563 gm, 5.71 mmol) in dry THF (30 ml) was cooled to -78°C and treated with n-BuLi (1.5 M in hexane, 8.0 ml, 12 mmol) over a 5 minute period. After stirring at -78°C for 1.25 hours, the brown solution was quenched with saturated $NH_4Cl$, warmed to room temperature, diluted with $H_2O$, and extracted 2x with $Et_2O$. The combined $Et_2O$ extracts were washed with brine, dried ($Na_2SO_4$), filtered and stripped to give a yellow solid. The solid was recrystallized from hot hexane to afford analytically pure 3-ethynyl-2-(4-fluorophenyl)-4-(1-methylethyl)-quinoline as off-white crystals (1.205 gm, 73%).
TLC: $R_f$ 0.44 (20% $CH_2Cl_2$ in hexane)

G. (S)-3-[[(1,1-dimethylethyl)diphenylsilyl]-oxy]-4-[[[2-(4-fluorophenyl)-4-(1-methylethyl)-3-quinolinyl]ethynyl]-methoxyphosphinyl]butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (4.501 gm, 7.123 mmol) (4.390 gm, 6.95 mmol) was partitioned between EtOAc and 5% $KHSO_4$. The EtOAc layer was washed 3x with 5% $KHSO_4$, then with brine, then dried ($Na_2SO_4$), filtered and stripped to give a colorless oil (phosphinic acid monomethyl ester). The oil was dissolved in dry $CH_2Cl_2$ (12 ml) and treated with N,N-diethyltrimethylsilylamine (2.70 ml, 2.07 gm, 14.25 mmol). After stirring at room temperature for 1.75 hours, the solvent was removed *in vacuo* and the residue was azeotroped with dry benzene (20 ml). The residue was dissolved in dry $CH_2Cl_2$ (12 ml), cooled to -10°C and treated with 2 drops DMF and oxalyl chloride (670 ul). After 15 minutes, the solution was warmed to room

temperature and stirred for an additional 50 minutes. The solvent was then stripped and the yellow residue (phosphinylchloridate) was azeotroped with benzene (20 ml) and dried *in vacuo* (oil pump) for 30 minutes.

Meanwhile, a solution of 3-ethynyl-2-(4-fluorophenyl)-4-(1-methylethyl)quinoline (1.180 gm, 4.08 mmol) in THF (10 ml) at -78° C was treated with n-BuLi (1.5 M in hexane, 2.75 ml, 4.13 mmol) and the resulting brown mixture was stirred for 30 minutes. The acetylenic anion solution was added dropwise *via canula* over a 15 minute period to a -78° C solution of the phosphinylchloridate in THF (9 ml). The resulting yellow-brown mixture was stirred at -78° C for 45 minutes, then quenched with 50% saturated $NH_4Cl$. The solution was warmed to room temperature, diluted with $H_2O$, and poured into saturated $NaHCO_3$. The aqueous phase was extracted 2x with $Et_2O$. The combined $Et_2O$ layers were washed with brine, dried ($Na_2SO_4$), filtered and stripped. The cloudy brown residue was chromatographed (flash, Merck $SiO_2$, 40% EtOAc in hexane followed by 1:1 EtOAc:hexane) to afford (S)-3-[[(1,1-dimethylethyl)diphenylsilyl]oxy]-4-[[[2-(4-fluorophenyl)-4-(1-methylethyl)-3-quinolinyl]ethynyl]methoxyphosphinyl]butanoic acid, methyl ester as a colorless foam (1.898 gm, 64%).

TLC: $R_f$ 0.14 (40% EtOAc in hexane)

H. (S)-4-[[[2-(4-fluorophenyl)-4-(1-methylethyl)-3-quinolinyl]ethynyl]methoxyphosphinyl]-3-hydroxybutanoic acid, methyl ester

A mixture of (S)-3-[[(1,1-dimethylethyl)diphenylsilyl]oxy]-4-[[[2-(4-fluorophenyl)-4-(1- methylethyl)-3-quinolinyl]ethynyl]methoxyphosphinyl]butanoic acid, methyl ester (729 mg, 0.997 mmol), tetra-n-butylammonium fluoride (1.0 M in THF, 3.0 ml, 3.0 mmol), and HOAc (304 mg, 5.06 mmol) in THF (12 ml) was stirred at room temperature for 20 hours. The solution was cooled to 0° C, quenched with 5% $KHSO_4$, and extracted twice with EtOAc. The pooled EtOAc layers were washed twice with 5% $KHSO_4$ and once with brine. The combined aqueous layers were back-extracted 3x with EtOAc and pooled with the initial EtOAc extracts. Drying ($Na_2SO_4$), followed by filtration and removal of the solvent afforded a yellow oil. The oil was dissolved in $Et_2O$ and EtOAc and treated with excess diazomethane for 30 minutes. The excess diazomethane was destroyed by the addition of HOAc and solvent was removed *in vacuo*. The residue was chromatographed (flash, Merck $SiO_2$, 40% acetone in hexane) to afford (S)-4-[[[2-(4-fluorophenyl)-4-(1-methylethyl)-3-quinolinyl]ethynyl]-methoxyphosphinyl]-3-hydroxybutanoic acid, methyl ester (349 mg, 72%) as a white oily foam.

TLC: $R_f$ 0.38 (1:1 acetone:hexane)

I. (S)-4-[[[2-(4-Fluorophenyl)-4-(1-methylethyl)-3-quinolinyl]ethynyl]hydroxyphosphinyl]-3-hydroxybutanoic acid, dilithium salt.

A solution of (S)-4-[[[2-(4-fluorophenyl)-4-(1-methylethyl)-3-quinolinyl]ethynyl]-methoxyphosphinyl]-3-hydroxybutanoic acid, methyl ester (333 mg, 0.689 mmol) in dioxane (5.5 ml) was treated with 1 N LiOH (2.08 ml, 2.08 mmol) at room temperature and the mixture was subsequently heated at 55° C under argon for one hour. The solvent was evaporated and the residue was chromatographed on HP-20 eluting in succession with $H_2O$ (200 ml), 25% MeOH in $H_2O$ (100 ml), and 50% MeOH in $H_2O$ (250 ml). The desired fractions were pooled and evaporated and the residue was taken up in $H_2O$ and lyophilized to give (S)-4-[[-[2-(4-fluorophenyl)-4-(1-methylethyl)-3-quinolinyl]ethynyl]hydroxyphosphinyl]-3-hydroxybutanoic acid, dilithium salt (314 mg, 92%) as a white solid.

TLC: $R_f$ 0.46 (7:2:1, i-PrOH, $NH_{64}OH$, $H_2O$)

## Example 6

(S)-4-[[2-[4-[4-Fluorophenyl)-2-(1-methylethyl)-6-phenyl-3-pyridinyl]ethyl]hydroxyphosphinyl]-3-hydroxybutanoic acid, dilithium salt.

A. [3S,4(Z)]-3-[[(1,1-dimethylethyl)diphenylsilyl]oxy]-4-[[2-[4-(4-fluorophenyl)-2-(1-methylethyl)-6-phenyl)-3-pyridinyl]ethenyl]methoxyphosphinyl]butanoic acid, methyl ester and (S)-3-[[1,1-dimethylethyl)diphenylsilyl]-

oxy]-4-[[2-[4-(4-fluorophenyl)-2-(1-methylethyl)-6-phenyl)-3-pyrindinyl]ethyl]methoxyphosphinyl]butanoic acid, methyl ester

(S)-3-[[(1,1-dimethylethyl)diphenylsilyl]oxy]-4-[[[4-(4-fluorophenyl)-2-(1-methylethyl)-6-phenyl)-3-pyridinyl]ethynyl]methoxyphosphinyl]butanoic acid, methyl ester (1.016 gm, 1.36 mmol) (the preparation of which is described in Example 4) was dissolved in MeOH (20 ml) and the mixture was purged with argon. Platinum on carbon (5% pt/C, 316 mg) was then added and the mixture was hydrogenated in a Parr apparatus at 40 psi for 16 hours. The solution was filtered through Celite and the filtrate was stripped and chromatographed (flash, Merck $SiO_2$, 20% acetone in hexane) to give the [3S,4(Z)]-3-[[(1,1-dimethylethyl)-diphenylsilyl]oxy]-4-[[2-[4-(4-fluorophenyl)-2-(1-methylethyl)-6-phenyl)-3-pyridinyl]ethenyl]-methoxyphosphinyl]butanoic acid, methyl ester (795 mg, 78%) as a colorless foam. In addition, the desired (S)-3-[[1,1-dimethylethyl)diphenylsilyl]oxy]-4-[[2-[4-(4-fluorophenyl)-2-(1-methylethyl)-6-phenyl)-3-pyrindinyl]-ethyl]methoxyphosphinyl]butanoic acid, methyl ester was also obtained (95 mg, 9%) as a colorless oil.

[3S,4(Z)]-3-[[(1,1-dimethylethyl)diphenylsilyl]oxy]-4-[[2-[4-(4-fluorophenyl)-2-(1-methylethyl)-6-phenyl)-3-pyridinyl]ethenyl]methoxyphosphinyl]butanoic acid, methyl ester (781 mg, 1.04 mmol) was dissolved in MeOH, and the solution was treated with 5% Pt/C (386 mg) and hydrogenated at 40 psi in a Parr apparatus for 4 days. Filtration and removal of the solvent afforded a residue which was chromatographed (flash, Merck $SiO_2$, 20% acetone in hexane) to give the desired saturated (S)-3-[[1,1-dimethylethyl)diphenylsilyl]-oxy]-4-[[2-[4-(4-fluorophenyl)-2-(1-methylethyl)-6-phenyl)-3-pyrindinyl]ethyl]methoxyphosphinyl]butanoic acid, methyl ester (144 mg, 18%) along with unreacted [3S,4(Z)]-3-[[(1,1-dimethylethyl)diphenylsilyl]-oxy]-4-[[2-[4-(4-fluorophenyl)-2-(1-methylethyl)-6-phenyl)-3-pyridinyl]ethenyl]methoxyphosphinyl]butanoic acid, methyl ester (546 mg).

[3S,4(Z)]-3-[[(1,1-dimethylethyl)diphenylsilyl]oxy]-4-[[2-[4-(4-fluorophenyl)-2-(1-methylethyl)- 6-phenyl)-3-pyridinyl]ethenyl]methoxyphosphinyl]butanoic acid, methyl ester:
TLC: $R_f$ 0.16 (20% acetone in hexane)
(S)-3-[[1,1-dimethylethyl)diphenylsilyl]oxy]-4-[[2-[4-(4-fluorophenyl)-2-(1-methylethyl)-6-phenyl)-3-pyrindinyl]-ethyl]methoxyphosphinyl]butanoic acid, methyl ester:
TLC: $R_f$ 0.09 (20% acetone in hexane)

B. (S)-4-[[2-[4-(4-fluorophenyl)-2-(1-methylethyl)-6-phenyl-3-pyridinyl]ethyl]methoxyphosphinyl]-3-hydroxybutanoic acid, methyl ester

A mixture of (S)-3-[[1,1-dimethylethyl)diphenylsilyl]oxy]-4-[[2-[4-(4-fluorophenyl)-2-(1-methylethyl)-6-phenyl)-3-pyrindinyl]ethyl]methoxyphosphinyl]butanoic acid, methyl ester (278 mg, 0.37 mmol), tetra-n-butylammonium fluoride (1.0 M in THF, 1.1 ml, 1.1 mmol), and HOAc (111 mg, 1.85 mmol) in THF (4 ml) was stirred at room temperature for 18 hours. The solution was quenched with cold $H_2O$ and extracted twice with EtOAc. The pooled EtOAc layers were washed with saturated $NaHCO_3$ and brine. Drying ($Na_2SO_4$), followed by filtration and removal of the solvent afforded an oil. The oil was chromatographed (flash, Merck $SiO_2$, 50% acetone in hexane followed by 70% acetone in hexane) to afford (S)-4-[[2-[4-(4-fluorophenyl)-2-(1-methylethyl)-6-phenyl-3-pyridinyl]ethyl]methoxyphosphinyl]-3-hydroxybutanoic acid, methyl ester (168 mg, 88%) as a colorless foam.
TLC: $R_f$ 0.18 (1:1 acetone:hexane)

C. (S)-4-[[2-[4-[4-Fluorophenyl)-2-(1-methylethyl)-6-phenyl-3-pyridinyl]ethyl]hydroxyphosphinyl]-3-hydroxybutanoic acid, dilithium salt

A solution of (S)-4-[[2-[4-(4-fluorophenyl)-2-(1-methylethyl)-6-phenyl-3-pyridinyl]-ethyl]-methoxyphosphinyl]-3-hydroxybutanoic acid, methyl ester (177 mg, 0.345 mmol) of dioxane (3.5 ml) was treated with 1 N LiOH (1.05 ml, 1.05 mmol) at room temperature and the mixture was subsequently heated at 57° C under argon for 1.3 hours. The solvent was evaporated and the residue was chromatographed on HP-20 eluting in succession with $H_2O$ (300 ml), 25% MeOH in $H_2O$ (100 ml), and 50% MeOH in $H_2O$ (250 ml). The desired fractions were pooled and evaporated and the residue was taken up in $H_2O$ and lyophilized to give (S)-4-[[2-[4-[4-Fluorophenyl)-2-(1-methylethyl)-6-phenyl-3-pyridinyl]ethyl]hydroxyphosphinyl]-3-hydroxybutanoic acid, dilithium salt (156 mg, 85%) as a white solid.
TLC: $R_f$ 0.40 (7:2:1, i-PrOH, $NH_4OH$, $H_2O$)

Example 7

(S,E)-4-[[2-[4-(4-Fluorophenyl)-2-(1-methylethyl)-6-phenyl-3-pyridinyl]ethenyl]hydroxyphosphinyl]-3-hydroxybutanoic acid, dilithium salt.

A. (E)-4-(4-Fluorophenyl)-3-(2-iodoethenyl)-2-(1-methylethyl)-6-phenylpyridine

A mixture of 3-Ethynyl-4-(4-fluorophenyl)-2-(1-methylethyl)-6-phenylpyridine (1.500 gm, 4.76 mmol) (The preparation of which is described in example 4) and AIBN (14 mg) in tri-n-butylstannyl hydride (1.90 ml) was rapidly heated to 120° C. After 4 minutes of heating, the mixture was treated with additional $Bu_3SnH$ (0.4 ml) and the temperature of the reaction was raised to 130° C. Approximately 14 mg of AIBN was added to the reaction mixture 0.5, 1.5 and 2.5 hours after heating was initiated. After 4 hours, the mixture was cooled to room temperature, diluted with $Et_2O$ (40 ml) and treated with solid $I_2$ (1.92 gm, 7.56 mmol). The dark reaction mixture was stirred for 1.5 hours, then quenched with 10% $Na_2SO_3$ in saturated $NaHCO_3$. The layers were shaken and separated. The ethereal layer was washed with brine, dried ($Na_2SO_4$), filtered and stripped to yield a yellow oil. Flash chromatography (Merck $SiO_2$, 1% EtOAc in hexane followed by 1.5% EtOAc in hexane afforded (E)-4-(4-Fluorophenyl)-3-(2-iodoethenyl)-2-(1-methylethyl)-6-phenylpyridine as a solid. Recrystallization of the solid from hot hexane gave 1.438 gm (E)-4-(4-Fluorophenyl)-3-(2-iodoethenyl)-2-(1-methylethyl)-6-phenylpyridine. An additional 0.19 gm of compound was obtained from the mother liquor to give a total of 1.628 gm (77%) product.
m.p. 111.0-112.3° C
TLC: $R_f$ 0.28 (2% EtOAC in hexane)

B. (S,E)-3-[[(1,1-Dimethylethyl)diphenylsilyl]oxy]-4-[[2-[4-(4-fluorophenyl)-2-(1-methylethyl)-6-phenyl-3-pyridinyl]ethenyl]methoxyphosphinyl]butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (4.501 gm, 7.123 mmol) (2.000 gm, 3.17 mmol), the preparation of which is described at the end of this example 7, was partitioned between EtOAc and 5% $KHSO_4$. The EtOAc layer was washed 3x with 5% $KHSO_4$, then with brine, then dried ($Na_2SO_4$), filtered and stripped to give a colorless oil (phosphinic acid monomethyl ester). The oil was dissolved in dry $CH_2Cl_2$ (7 ml) and treated with N,N-diethyltrimethylsilylamine (1.20 ml, 0.92 gm, 6.33 mmol). After stirring at room temperature for one hour, the solvent was removed *in vacuo* and the residue was azeotroped with dry benzene (10 ml). The residue was dissolved in dry $CH_2Cl_2$ (7 ml), cooled to 10° C and treated with 1 drop DMF and oxalyl chloride (320 ul). After 15 minutes, the solution was warmed to room temperature and stirred for an additional 50 minutes. The solvent was then stripped and the yellow residue (phosphinylchloridate) was azeotroped with benezene (20 ml) and dried in *in vacuo* (oil pump) for 30 minutes.

Meanwhile,. a solution of (E)-4-(4-Fluorophenyl)-3-(2-iodoethenyl)-2-(1-methylethyl)-6-phenylpyridine (827 mg, 1.86 mmol) in THF (2 ml) was added over a 2 minute period to a -78° C solution of t-butyllithium (1.7 M in pentane, 2.20 ml 3.74 mmol) in THF (7 ml). The resulting deep red solution was stirred for 25 minutes then cooled to -100° C. The vinyl anion solution was added over a 30 second period to a -100° C solution of the phosphinylchloridate in THF (9 ml). The resulting orange mixture was stirred at -100° C for 5 minutes and at -78° C for 30 minutes, then quenched with 50% saturated $NH_4Cl$. The solution was warmed to room temperature, diluted with $H_2O$, and poured into saturated $NaHCO_3$. The aqueous phase was extracted 2x with $Et_2O$. The combined $Et_2O$ layers were washed with brine, dried ($Na_2SO_4$), filtered and stripped. The resulting yellow oil was chromatographed (flash, Merck $SiO_2$, 15% acetone in hexane followed by 20% acetone in hexane) to afford (S,E)-3-[[(1,1-Dimethylethyl)diphenylsilyl]oxy]-4-[[2-[4-(4-fluorophenyl)-2-(1-methylethyl)-6-phenyl-3-pyridinyl]ethenyl]methoxyphosphinyl]butanoic acid, methyl ester as an off-white foam (826 mg, 59%).
TLC: $R_f$ 0.28 (20% acetone in hexane)

C. (S,E)-4-[[2-[4-(4-Fluorophenyl)-2-(1-methylethyl)-6-phenyl-3-pyridinyl]ethenyl]methoxyphosphinyl]-3-hydroxybutanoic acid, methyl ester

A solution of (S,E)-3-[[(1,1-Dimethylethyl)diphenylsilyl]oxy]-4-[[2-[4-(4-fluorophenyl)-2-(1-methylethyl)-6-phenyl-3-pyridinyl]ethenyl]methoxyphosphinyl]butanoic acid, methyl ester (826 mg, 1.10 mmol) in THF (10 ml) was treated with HOAc (320 ul, 336 mg, 5.6 mmol) and tetra-n-butylammonium fluoride (1.0 M in THF, 3.3 ml, 3.3 mmol). After stirring at room temperature for 22 hours, the solution was poured into saturated $NaHCO_3$ and extracted with EtOAc. The EtOAc extract was washed with brine, dried ($Na_2SO_4$), filtered, and stripped to give an oil which was subsequently chromatographed (flash, Merck $SiO_2$, 30% acetone in hexane followed by 50% acetone in hexane). (S,E)-4-[[2-[4-(4-Fluorophenyl)-2-(1-methylethyl)-6-phenyl-3-pyridinyl]ethenyl]methoxyphosphinyl]-3-hydroxybutanoic acid, methyl ester (497 mg, 88%) was obtained as a white oily foam.
TLC: $R_f$ 0.40 (1:1 acetone:hexane)

D. (S,E)-4-[[2-[4-(4-Fluorophenyl)-2-(1-methethyl)-6-phenyl-3-pyridinyl]ethenyl]hydroxyphosphinyl]-3-hydroxybutanoic acid, dilithium salt

A solution of (S,E)-4-[[2-[4-(4-Fluorophenyl)-2-(1-methylethyl)-6-phenyl-3-pyridinyl]ethenyl]-methoxyphosphinyl]-3-hydroxybutanoic acid, methyl ester (474 mg, 0.93 mmol) in dioxane (5 ml) was treated with 1 N LiOH (3.0 ml, 3.0 mmol) at room temperature and the mixture was subsequently heated at 55° C under argon for 1.5 hours. The solvent was evaporated and the residue was chromatographed on HP-20 eluting in succession with $H_2O$ (200 ml), 25% MeOH in $H_2O$ (100 ml), and 50% MeOH in $H_2O$ (200 ml). The desired fractions were pooled and evaporated and the residue was taken up in $H_2O$ and lyophilized to give (S,E)-4-[[2-[4-(4-Fluorophenyl)-2-(1-methethyl)-6-phenyl-3-pyridinyl]ethenyl]hydroxyphosphinyl]-3-hydroxybutanoic acid, dilithium salt (431 mg, 89%) as a white solid.
TLC: $R_f$ 0.48 (7:2:1, i-PrOH, $NH_4OH$, $H_2O$)

(S)-4-(Hydroxymethoxyphosphinyl)-3-[[(1, 1-dimethylethyl)diphenylsilyl]oxy]butanoic acid, methyl ester, dicyclohexylamine (1:1) salt

(1) (S)-4-Bromo-3-hydroxybutanoic acid, methyl ester

(1)(a) [R-(R*,R*)]-2,3,4-trihydroxybutanoic acid, calcium salt, hydrate

Ref. Carbohydrate Research 72, pp. 301-304 (1979).

Calcium carbonate (50 g) was added to a solution of D-isoascorbic acid (44.0 g, 250 mmol) in $H_2O$ (625 ml), the suspension cooled to 0° C (ice bath) and treated portionwise with 30% $H_2O_2$ (100 ml). The mixture was stirred at 30°-40° C (oil bath) for 30 minutes. Darco (10 g) was added and the black suspension heated on a steam bath until evolution of $O_2$ ceased. The suspension was filtered through Celite, evaporated *in vacuo* (bath temperature 40° C). The residue was taken up in $H_2O$ (50 ml), warmed on a steam bath and $CH_3OH$ was added until the solution was turbid. The gummy precipitated solid was collected by filtration and air dried to give 30.836 g (75.2%) of desired calcium salt as a powdery white solid.
TLC: (7:2:1) iPrOH-$NH_4OH$-$H_2O$, Rf = 0.19, PMA.

(1)(b) [S-(R*,S*)]-2,4-Dibromo-3-hydroxybutanoic acid, methyl ester

Ref. Bock, K. et al., Acta Scandinavica (B) 37, pp. 341-344 (1983).

Part (1)(a) calcium salt (30 g) was dissolved in 30-32% HBr in acetic acid (210 ml) and stirred at room temperature for 24 hours. Methanol (990 ml) was then added to the brown solution and it was stirred overnight. The mixture was evaporated to an orange oil, taken up in $CH_3OH$ (75 ml), refluxed for 2.0 hours and evaporated. The residue was partitioned between EtOAc (100 ml) and $H_2O$, the organic phase washed with $H_2O$ (2x) and brine then dried over anhydrous $Na_2SO_4$ and evaporated to give 22.83 g (90.5%) of crude dibromide as a light orange oil.
TLC: (1:1) EtOAc-Hex, Rf = 0.69, UV & PMA.

(1)(c) (S)-4-Bromo-3-hydroxybutanoic acid, methyl ester

Ref. the same as for preparation of (1)(b).

An argon purged solution of the dibromide (20.80 g, 75.4 mmol) and anhydrous NaOAc (21.0 g) in EtOAc (370 ml) and glacial HOAc (37 ml) was treated with 5% Pd/C (1.30 g) and the black suspension stirred under of $H_2$ (1 atm) while monitoring $H_2$ uptake. After 2.0 hours $H_2$ uptake was complete, the mixture was filtered through Celite, the filtrate washed with saturated $NaHCO_3$ and brine then dried over anhydrous $MgSO_4$ and evaporated to give crude dibromoester as a brown oil. The crude oil was combined with another batch (starting from 36.77 g of the dibromide) and vacuum distilled to give 25.77 g (61.3%) of desired title bromoester as a clear oil with b.p. = 79°-80° C (1.0 mm Hg).

TLC: (1:1) EtOAc-Hex, Rf = 0.44, PMA.

Anal Calcd for $C_5H_9O_3Br$: C, 30.48; H, 4.60; Br, 40.56

Found: C, 29.76; H, 4.50; Br, 39.86

(2) (S)-4-Bromo-3-[[(1,1-dimethylethyl)diphenylsilyl]oxy]butanoic acid, methyl ester

A solution of part (1)(c) bromohydrin (4.0 g, 20.4 mmol), imidazole (6.94 g, 5.0 eg.), and 4-dimethylamino pyridine (4-DMAP) (12 mg, 0.005 eg.) in dry DMF (40 ml) was treated with t-butyldiphenylsilyl chloride (5.84 ml, 1.1 eg.) and the homogeneous mixture stirred overnight under argon at room temperature. The mixture was partitioned between 5% $KHSO_4$ and EtOAc, the organic phase washed with $H_2O$ and brine, dried over anhydrous $Na_2SO_4$ and evaporated to give 9.32 g (100%) of crude silyl ether as a clear, viscous oil.

TLC: (3:1) Hex-EtOAc, Rf silyl ether = 0.75, U.V. and PMA.

(3) (S)-4-Iodo-3-[[(1,1-dimethylethyl)diphenylsilyl]oxy]butanoic acid, methyl ester

A solution of the crude Part 2 bromide (9.32 g, 201 mmole) in methyl ethyl ketone (60 ml, dried over 4 Å sieves) was treated with sodium iodide (15.06 g, 100.5 mmole, 5.0 eg.) and the yellow suspension refluxed for 5.0 hours under argon. The mixture was cooled, diluted with EtOAc, filtered, the filtrate washed with dilute $NaHSO_3$ (until colorless) and brine then dried over anhydrous $Na_2SO_4$ and evaporated *in vacuo* to give 10.17 g of a yellow oil. The crude oil was purified by flash chromatography on silica gel (600 g) eluting with (3:1) Hexane-$CH_2Cl_2$. Product fractions were combined and evaporated to give 7.691 g (74.2%, overall yield for both steps) of desired title iodide as a clear, colorless, viscous oil.

TLC: (3:1) Hex-EtOAc, product. Rf = 0.75, U.V. and PMA. (Note: product iodide co-spots with starting bromide).

(4) (S)-4-Diisopropyloxyphosphinyl)-3-[[(1,1-dimethylethyl)diphenylsilyl]oxy]-butanoic acid, methyl ester

The Part (3) iodide (45.1 mmol., 21.70 g) was stirred under high vacuum for 30 minutes. Freshly distilled triisopropyl phosphite (0.451 mol., 93.92 g, 113.37 ml.) was added in one portion and the reaction mixture was stirred under argon and heated in a 155° C oil bath for 16.5 hours. The mixture was then cooled to room temperature. Excess triisopropyl phosphite and volatile reaction products were removed by short path distillation (10 mm Hg) followed by Kugelrohr distillation (0.50 mm Hg, 100° C, 8 hours). The product was further purified via flash chromatography (95 mm diam. column, 6"/Merck silica gel, 6/3/1 Hexane/acetone/toulene eluent, 2"/min flow rate, 50 ml fractions) to afford 17.68 g (33.96 mmol, 75% yield) of the title isopropylphosphonate as a clear viscous oil.

TLC: Silica gel Rf = 0.32 (6:3:1 Hexane/acetone toluene)

$^1$HNMR: (270 $MH_z$, $CDCl_3$)

δ 7.70-7.65 (m,4H)

7.45-7.35 (m,6H)

4.57-4.44 (m,3H)

3.59 (s,3H)

2.94 and 2.88 (2xd, 1H J = 3.7 Hz)

2.65 and 2.60 (2xd, 1H J = 7.4 Hz)

2.24-1.87 (Series of m, 2H)

1.19 and 1.12 (2xd, 12H J = 6.3 Hz)
1.01 (s, 9H)

(5) (S)-4-(Hydroxymethoxyphosphinyl)-3-[[(1-dimethylethyl)diphenylsilyl]oxy]butanoic acid, methyl ester, dicyclohexylamine (1:1) salt

The Part (4) isopropyl phosphonate (30.5 mmol, 10.66 g) was stirred under argon, at room temperature, in 80 ml of dry $CH_2Cl_2$. This solution was treated dropwise (5 min) with bistrimethylsilyltrifluoroacetamide (BSTFA) (32.8 mmol, 8.44 g, 8.71 ml), followed by dropwise addition (10 min) of trimethylsilylbromide (TMSBr) (51.3 mmol), 7.84 g, 6.75 ml). After stirring at room temperature for 20 hours, the reaction mixture was quenched with 200 ml of 5% aqueous $KHSO_4$ and stirred vigorously for 15 minutes. The aqueous layer was extracted 3 times with ethylacetate. The organic extracts were combined, washed once with brine, dried over $Na_2SO_4$ and concentrated *in vacuo*. The residue was azeotroped 2 times with 50 ml of toluene. The precipitate which formed was suspended in toluene and filtered. The filtrate was concentrated and the azeotrope/filter process repeated. The resulting filtrate was evaporated *in vacuo* and then pumped under high vacuum for 5 hours. The resulting viscous clear oil was stirred under argon, at room temperature, in 50 ml of dry pyridine. This solution was treated in one portion with dicyclohexylcarbodiimide (DCC) (22.6 mmol, 4.65 g), followed by addition of methanol (41.0 mmol, 1.31 g, 1.67 ml). After stirring at room temperature for 20 hours, the reaction mixture was filtered through a celite pad in a sintered glass funnel. The celite was washed with ethyl acetate and the combined filtrates were evaporated *in vacuo*. The residue was redissolved in ethyl acetate and washed 2 times with 5% aqueous $KHSO_4$ and once with brine. The organic extract was dried over $Na_2SO_4$, filtered, the filtrate concentrated and azeotroped 2 times with toluene, suspended in toluene and filtered. The resulting filtrate was again concentrated, azeotroped, filtered and the filtrate evaporated *in vacuo* and place under high vacuum for 6 hours to afford the phosphonate monoester as a clear viscous oil (10.2 g, >100% yield). TLC: silica gel $R_f$ = 0.50 (7:2:1 nPrOH/$NH_4OH$/$H_2O$). The phosphonate monoester [1.21 g was pumped under high vacuum for 4 hours, affording 1.16 g (2.57 mmol)] was dissolved in 10 ml of dry ethyl ether and treated dropwise with dicyclohexylamine (2.65 mmol, 0.481 g, 0.528 ml). The resulting homogeneous solution sat at room temperature for 7 hours resulting in significant crystal formation. The mixture was stored at -20° C for 16 hours and then warmed to room temperature and filtered. The crystals were washed with cold, dry ethyl ether and then pumped under high vacuum over $P_2O_5$ for 18 hours. The crystals were subsequently pumped under high vacuum at 45° C for 4 hours, affording 1.25g (1.98 mmol, 77% yield) of the title dicyclohexylamine salt as a white powdery solid, m.p. 155-156° C.

TLC: Silica gel $R_f$ = 0.57 (20% MeOH/$CH_2Cl_2$) 'H NMR: (270 $MH_2$, $CDCl_3$)
7.71-7.65 (m, 4H)
7.40-7.32 (m, 6H)
4.02 (m, 1H)
3.52 (s, 3H)
3.28 and 3.22 (m, 1H)
3.11 (d, 3H J = 11 Hz)
2.77-2.64 (m, 2H)
2.62-2.56 (m, 1H)
1.92-1.08 (Series of m, 22H)
1.00 (S, 9H)
Mass Spec: (FAB) 632 $(M\&H)^+$
IR:(KBr) 3466-3457 (broad)
3046, 3016, 2997, 2937, 2858, 2836, 2798, 2721, 2704, 2633, 2533, 2447, 1736, 1449, 1435, 1426, 1379, 1243, 1231, 1191, 1107, 1074, 1061, 1051, 820 CM-1

Anal Calcd for $C_{22}H_{31}O_6PSi \cdot C_{12}H_{23}N$:

C, 64.63; H, 8.61; N, 2.22
Found: C, 64.51; H, 8.49; N, 2.18

Example 8

(S)-4-[[[4-(4-Fluorophenyl)-2-(1-methylethyl)-3-quinolinyl]ethynyl]hydroxyphosphinyl]-3-hydroxybutanoic acid, dilithium salt

A. 2-Aminophenyl 4-fluorophenyl methanone

A dry 500 ml 3-neck flask fitted with a reflux condenser and an addition funnel was charged with Mg turnings (2.80 gm, 0.115 mol) and dry $Et_2O$ (35 ml). A solution of 1-bromo-4-fluorobenzene (18.5 gm, 0.106 mol) in $Et_2O$ (20 ml) was added to the addition funnel and approximately 15% of this solution was added to the Mg/$Et_2O$ mixture. Once the exothermic reaction had begun (ultrasound was required), the aryl bromide solution was added at such a rate as to maintain a gentle reflux. After the addition was complete, the mixture was refluxed for an additional 30 minutes, then cooled to 0° C. Dropwise addition of a solution of 2-aminobenzonitrile (5.00 gm, 0.042 mol) in dry $Et_2O$ (20 ml) over a 10 minute period resulted in the formation of a thick yellow slurry. The resulting mixture was warmed to room temperature and stirred overnight. The solution was then cooled to 0° C and cautiously quenched with 10% HCl (50 ml). After stirring for 5 minutes, the solution was made basic with 10% NaOH (70 ml) and the aqueous layer was extracted three times with $Et_2O$. The combined $Et_2O$ layers were pooled, washed with brine, dried ($Na_2SO_4$), filtered and stripped to yield an orange oil. The oil was chromatographed (Flash, Merck $SiO_2$, 25% EtOAc in hexane) to give impure 2-aminophenyl 4-fluorophenyl methanone (~1 gm) and the corresponding impure imine (~ 5.8 gm, Rf 0.28 in 30% EtOAc in hexane). The imine was stirred with 10% HCl (100 ml) and silica gel (750 mg) for 45 minutes. The mixture was then basicified with NaOH pellets (14 gm) and the aqueous layer was extracted twice with $Et_2O$. The combeined $Et_2O$ layers were washed with brine, dried ($Na_2SO_4$), filtered and stripped to yield a solid. This solid was pooled with impure 2-aminophenyl 4-fluorophenyl methanone from above and recrystallized in hot EtOH/$H_2O$ to give analytically pure 2-aminophenyl 4-fluorophenyl methanone as yellow crystals (3.28 gm, 36%).
m.p. 125.2 - 127.4° C
TLC: $R_f$ 0.41 (30% EtOAc in hexane)

B. 4-(Fluorophenyl)-2-(1-methylethyl)-3-quinolinecarboxylic acid, ethyl ester

A mixture of 2-aminophenyl 4-fluorophenyl methanone (3.051 gm, 14 mmol), ethyl isobutrylacetate (2.24 gm, 14 mmol) and concentrated $H_2SO_4$ (0.14 ml) in glacial HOAc (14 ml) was refluxed for 4 hours. The reaction mixture was cooled to room temperature and poured into an ice cold solution of concentrated $NH_4OH$ (21 ml) in $H_2O$ (60 ml). The resulting mixture was extracted twice with $Et_2O$ and the combined $Et_2O$ extracts were washed with $H_2O$ and brine, then dried ($Na_2SO_4$), filtered and stripped to yield an orange oil. Flash chromatography of the oil (Merck $SiO_2$, 10% EtOAc in hexane) afforded quinoline 4-(Fluorophenyl)-2-(1-methylethyl)-3-quinolinecarboxylic acid, ethyl ester (3.212 gm, 68%) as a light yellow oil which slowly solidified overnight under vacuum.
m.p. 65.4 - 69.0° C
TLC: $R_f$ 0.46 (20% EtOAc in hexane)

C. 4-(4-Fluorophenyl)-2-(1-methylethyl)-3-quinolinemethanol

A solution of 4-(Fluorophenyl)-2-(1-methylethyl)-3-quinolinecarboxylic acid, ethyl ester (3.018 gm, 8.94 mmol) in $Et_2O$ (5 ml) was added to a slurry of $LiAlH_4$ (689 mg, 18.0 mmol) in $Et_2O$ (22 mol) at 0° C. After 2.5 hours, the solution was quenched and diluted with $H_2O$ and the aqueous layer was extracted three times with $Et_2O$. The combined $Et_2O$ extracts were washed with brine, dried ($Na_2SO_4$), filtered and stripped. The resulting oil was triturated with 30% EtOAc in hexane to obtain a white solid. The solid was collected and the filtrate was concentrated and triturated with hexane to obtain additional solid. The pooled solids were recrystallized from hot hexane/EtOAc to give analytically pure 4-(4-fluorophenyl)-2-(1-methylethyl)-3-quinolinemethanol as white crystals (1.516 gm). The mother liquor was stripped and recrystallized again from hot hexane/EtOAc, giving a total of 2.039 gm (77%) 4-(4-fluorophenyl)-2-(1-methylethyl)-3-quinolinemethanol.
m.p. 132.5 - 134° C
TLC: $R_f$ 0.22 (20% EtOAc in hexane)

D. 4-(4-fluorophenyl)-2-(1-methylethyl)-3-quinolinecarboxaldehyde

Dess-Martin periodinane (1.018 gm, 2.40 mmol) was dissolved in dry $CH_2Cl_2$ (12 ml), treated with t-butanol (178 mg, 2.40 mmol) and the resulting mixture was stirred at room temperature for 15 minutes. A solution of 4-(4-fluorophenyl)-2- (1-methylethyl)-3-quinolinemethanol (539 mg, 1.82 mmol) in $CH_2Cl_2$ (8 ml) was then added. After 25 minutes, the solution was then diluted with $Et_2O$ (40 ml) and 1 N NaOH (25 ml), then stirred an additional 5 minutes. The phases were separated and the aqueous layer was extracted with $Et_2O$. The combined organic layers were washed with saturated $NaHCO_3$ and brine, then dried ($Na_2SO_4$), filtered and stripped to give a yellow solid. Flash chromatography (Merck $SiO_2$, 40% EtOAc in hexane) gave 4-(4-fluorophenyl)-2-(1-methylethyl)-3-quinolinecarboxaldehyde (491 mg, 92%) as a white solid. Analytically pure material was obtained by recrystallization from hot hexane.
m.p. 120.4 - 122.2° C
TLC: $R_f$ 0.47 (20% EtOAc in hexane)

E. 3-(2,2-Dibromoethenyl)-4-(4-fluorophenyl)-2-(1-methylethyl)quinoline

A solution of carbon tetrabromide (12.620 gm, 38.0 mmol) in $CH_2Cl_2$ (20 ml) was added over a 10 minute period to a cold (-10° C) solution of 4-(4-Fluorophenyl)-2-(1-methylethyl)-3-quinolinecarboxaldehyde (7.394 gm, 25.2 mmol) and triphenylphosphine (19.86 gm, 75.7 mmol) in $CH_2Cl_2$ (140 ml). After the addition was complete, the mixture was stirred for 20 minutes then quenched with saturated $NaHCO_3$ and extracted 2X with $CH_2Cl_2$. The combined organic layers were washed with brine, dried ($Na_2SO_4$), filtered and concentrated to 50 ml. The residue was triturated with $Et_2O$ and the precipitated triphenylphosphine oxide was removed by filtration. The filtrate was stripped and the residue was chromatographed (flash, Merck $SiO_2$, 10% EtOAc in hexane) to give 3-(2,2-Dibromoethenyl)-4-(4-fluorophenyl)-2-(1-methylethyl)quinoline as a light yellow foamy oil (11.556 gm, 102% theory).
TLC: $R_f$ 0.50 (20% EtOAc in hexane)

F. 3-Ethynyl-4-(4-fluorophenyl)-2-(1-methylethyl)quinoline

A solution of 3-(2,2-Dibromoethenyl)-4-(4-fluorophenyl)-2-(1-methylethyl)quinoline (25.2 mmol) in dry THF (140 ml) was cooled to -78° C and treated with n-BuLi (1.6 M in hexane, 33 ml, 52.8 mmol) over a 1 minute period. After stirring at -78° C for one hour, the dark brown solution was quenched with saturated $NH_4Cl$, warmed to room temperature, diluted with $H_2O$, and extracted with $Et_2O$. The $Et_2O$ extract was washed with brine, dried ($Na_2SO_4$), filtered and stripped to give a yellow oil. The oil was boiled in hot hexane and cooled (-15° C) to afford 3-Ethynyl-4-(4-fluorophenyl)-2-(1-methylethyl)-quinoline (5.198 gm) as yellow crystals. The mother liquor was stripped and chromatographed (flash, Merck $SiO_2$, 4% EtOAc in hexane) to afford additional product which was recrystallized from hot hexane. A total of 6.793 gm (93%) of 3-Ethynyl-4-(4-fluorophenyl)-2-(1-methylethyl)quinoline was obtained.
TLC: $R_f$ 0.49 (10% EtOAc in hexane)

G. (S)-3-[[(1,1-Dimethylethyl)diphenylsilyl]oxy]-4-[[[4-(4-fluorophenyl)-2-(1-methylethyl)-3-quinolinyl]ethynyl]-methoxyphosphinyl]butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (4.501 gm, 7.123 mmol) (4.458 gm, 7.05 mmol) the preparation of which is described at the end of example 7, was partitioned between EtOAc and 5% $KHSO_4$), filtered and stripped to give a colorless oil (phosphinic acid monomethyl ester). The oil was dissolved in dry $CH_2Cl_2$ (15 ml) and treated with N,N-diethyltrimethylsilylamine (2.70 ml, 2.07 gm, 14.25 mmol). After stirring at room temperature for one hour, the solvent was removed *in vacuo* and the residue was azeotroped with dry benzene (15 ml). The residue was dissolved in dry $CH_2Cl_2$ (15 ml), cooled to -10° C and treated with 2 drops DMF and

oxalyl chloride (677 ul). After 15 minutes, the solution was warmed to room temperature and stirred for an additional 50 minutes. The solvent was then stripped and the yellow residue (phosphinylchloridate) was azeotroped with benzene (15 ml) and dried *in vacuo* (oil pump) for 30 minutes.

Meanwhile, a solution of 3-Ethynyl-4-(4-fluorophenyl)-2-(1-methylethyl)quinoline (1.200 gm, 4.15 mmol) in THF (10 ml) at -78° C was treated with n-BuLi (1.6 M in hexane, 2.85 ml, 4.56 mmol) and the resulting clear brown mixture was stirred for 30 minutes. The acetylenic anion solution was added dropwise *via canula* over a one minute period to a -78° C solution of the phosphinylchloridate in THF (12 ml). The resulting yellow-brown mixture was stirred at -78° C for 30 minutes, then quenched with 50% saturated $NH_4Cl$. The solution was warmed to room temperature, diluted with $H_2O$, and poured into saturated $NaHCO_3$. The aqueous phase was extracted 2X with $Et_2O$. The combined $Et_2O$ layers were washed with brine, dried ($Na_2SO_4$), filtered and stripped. The residue was chromatographed (flash, Merck $SiO_2$, 40% EtOAc in hexane followed by 1:1 EtOAc:hexane) to afford (S)-3-[[(1,1-Dimethylethyl)diphenylsilyl]oxy]-4-[[[4-(4-fluorophenyl)-2-(1-methylethyl)-3-quinolinyl]ethynyl]methoxyphosphinyl]-butanoic acid, methyl ester as a colorless foam (1.800 gm, 60%).

TLC: $R_f$ 0.24 (40% EtOAc in hexane)

H. (S)-4-[[[4-(4-Fluorophenyl)-2-(1-methylethyl)-3-quinolinyl]ethynyl]-methoxyphosphinyl]-3-hydroxybutanoic acid, methyl ester

A mixture of (S)-3-[[(1,1-Dimethylethyl)diphenylsilyl]oxy]-4-[[[4-(4-fluorophenyl)-2-(1-methylethyl)-3-quinolinyl]ethynyl]methoxyphosphinyl]butanoic acid, methyl ester (1.041 gm, 1.44 mmol), tetra-n-butylammonium fluoride (1.0 M in THF, 5.7 ml, 5.7 mmol), and HOAc (525 mg, 8.73 mmol) in THF (14 ml) was stirred at room temperature for 18 hours. The solution was partitioned between 5% $KHSO_4$ and EtOAc. The layers were shaken and separated and the EtOAc layer was washed again with 5% $KHSO_4$. The pooled aqueous washings were back-extracted twice with EtOAc. The combined EtOAc layers were washed with brine and dried ($Na_2SO_4$). Filtration and removal the solvent afforded an oil. The oil was dissolved in cold (0° C) 1:1 $Et_2O$:MeOH and treated with excess diazomethane for 30 minutes. The excess diazomethane was destroyed by the addition of HOAc and the solvent was removed *in vacuo*. The residue was chromatographed (flash, Merck $SiO_2$, 40% acetone in hexane followed by 1:1 actone:hexane) to afford (S)-4-[[[4-(4-Fluorophenyl)-2-(1-methylethyl)-3-quinolinyl]ethynyl]-methoxyphosphinyl]-3-hydroxybutanoic acid, methyl ester (612 mg, 91%) as an oil.

TLC: $R_f$ 0.38 (1:1 acetone:hexane)

I. (S)-4-[[[4-(4-Fluorophenyl)-2-(1-methylethyl)-3-quinolinyl]ethynyl]hydroxyphosphinyl]-3-hydroxybutanoic acid, dilithium salt

A solution of (S)-4-[[[4-(4-Fluorophenyl)-2-(1-methylethyl)-3-quinolinyl]ethynyl]-methoxyphosphinyl]-3-hydroxybutanoic acid, methyl ester (285 mg, 0.59 mmol) in dioxane (4.0 ml) was treated with 1 N LiOH (2.05 ml, 2.05 mmol) at room temperature and the mixture was subsequently heated at 55° C under argon for 1.5 hours. The solvent was evaporated and the residue was chromatographed on HP-20 eluting in succession with $H_2O$ (200 ml), 25% MeOH in $H_2O$ (100 ml), and 50% MeOH in $H_2O$ (200 ml). The desired fractions were pooled and evaporated and the residue was taken up in $H_2O$ and lyophilized to give (S)-4-[[[4-(4-Fluorophenyl)-2-(1-methyl-ethyl)-3-quinolinyl]ethynyl]hydroxyphosphinyl]-3-hydroxybutanoic acid, dilithium salt (243 mg, 84%) as a white solid.

TLC: $R_f$ 0.41 (7:2:1, i-PrOH, $NH_4OH$, $H_2O$)

## Example 9

(S)-4-[[[4-(4-Fluorophenyl)-2-(1-methylethyl)-3-quinolinyl]ethynyl]hydroxyphosphinyl]-3-hydroxybutanoic acid, N-oxide, dilithium salt

A. (S)-4-[[[4-(4-Fluorophenyl)-2-(1-methylethyl)-3-quinolinyl]ethynyl]-methoxyphosphinyl]-3-hydroxybutanoic

acid, methyl ester. 1-oxide

A solution of (S)-4-[[[4-(4-Fluorophenyl)-2-(1-methylethyl)-3-quinolinyl]ethynyl]-methoxyphosphinyl]-3-hydroxybutanoic acid, methyl ester (332 mg, 0.67 mmol) (the preparation of which is described in Example 8) and m-CPBA (80-85%, 585 mg) in dry $CH_2Cl_2$ (18 ml) was stirred at room temperature for 16 hours. Additional m-CPBA (277 mg) was added and stirring was continued for 2 hours. The mixture was diluted with $Et_2O$ and washed with 50 ml saturated $NaHCO_3$ containing 2.05 gm $Na_2SO_3$. The organic layer was subsequently washed with brine, dried ($Na_2SO_4$), filtered and stripped to yield an orange-brown residue. The residue was chromatographed (flash, Merck $SiO_2$, 40% hexane in acetone followed by 30% hexane in acetone) giving (S)-4-[[[4-(4-Fluorophenyl)-2-(1-methylethyl)-3-quinolinyl]ethynyl]-methoxyphosphinyl]-3-hydroxybutanoic acid, methyl ester. 1-oxide. (226 mg, 68%) as a viscous yellow oil.
TLC: $R_f$ 0.18 (1:1 acetone:hexane)

B. (S)-4-[[[4-(4-Fluorophenyl)-2-(1-methylethyl)-3-quinolinyl]ethynyl]hydroxyphosphinyl]-3-hydroxybutanoic acid, N-oxide dilithium salt

A solution of (S)-4-[[[4-(4-Fluorophenyl)-2-(1-methylethyl)-3-quinolinyl]ethynyl]-methoxyphosphinyl]-3-hydroxybutanoic acid, methyl ester. 1-oxide.(220 mg, 0.44 mmol) in dioxane (3.0 ml) was treated with 1 N LiOH (1.9 ml, 1.9 mmol) at room temperature and the mixture was subsequently heated at 55°C under argon for 1 hour. The solvent was evaporated and the residue was chromatographed on HP-20 eluting in succession with $H_2O$ (200 ml), 25% MeOH in $H_2O$ (100 ml), and 50% MeOH in $H_2O$ (200 ml). The desired fractions were pooled and evaporated and the residue was taken up in $H_2O$ and lyophilized to give (S)-4-[[[4-(4-Fluorophenyl)-2-(1-methylethyl)-3-quinolinyl]ethynyl]hydroxyphosphinyl]-3-hydroxybutanoic acid, N-oxide, dilithium salt (196 mg, 85%) as a dense yellow solid.
TLC: $R_f$ 0.43 (7:2:1, i-PrOH, $NH_4OH$, $H_2O$)

## Example 10

[3S,4(E)]-4-[[2-[2-(4-Fluorophenyl)-4-(1-methylethyl)-3-quinolinyl]ethenyl]hydroxyphosphinyl]-3-hydroxybutanoic acid, dilithium salt

A. (E)-2-(4-Fluorophenyl)-3-(2-iodoethenyl)-4-(1-methylethyl)quinoline

A mixture of 3-Ethynyl-2-(4-fluorophenyl)-4-(1-methylethyl)-quinoline (1.000 gm, 3.64 mmol) (the preparation of which is described in Example 5) and AIBN (13 mg) in tri-n-butylstannyl hydride (1.40 ml) was rapidly heated to 110°C. After 5 minutes of heating, the mixture was treated with additional $Bu_3SnH$ (0.4 ml) and the temperature of the reaction was raised to 130°C. Approximately 10 mg of AIBN was added to the reaction mixture every 0.5 hours. After 4.5 hours, the mixture was cooled to room temperature, diluted with $Et_2O$ (25 ml) and treated with solid $I_2$ (3.08 gm, 12.2 mmol). The dark reaction mixture was stirred for 1 hour, then quenched with 10% $Na_2SO_3$ in saturated $NaHCO_3$. The layers were snaken and separated. The ethereal layer was washed with brine. dried ($Na_2SO_4$), filtered and stripped to yield a yellow oil which was triturated with cold hexane to give a precipitate. Recrystallization of the solid from hot hexane gave 1.438 gm (E)-2-(4-Fluorophenyl)-3-(2-iodoethenyl)-4-(1-methylethyl)quinoline (917 mg, 64%) as light yellow crystals.
m.p. 145-147°C
TLC: $R_f$ 0.15 (6% EtOAC in hexane)

B. (S,E)-3-[[(1,1-Dimethylethyl)diphenylsilyl]-4-[[2-[2-(4-fluorophenyl)-4-(1-methylethyl)-3-quinolinyl]ethenyl]-methoxyphosphinyl]butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (4.501 gm, 7.123 mmol) (1.548 gm, 2.45 mmol), the preparation of which is

described at the end of example 7, was partitioned between EtOAc and 5% $KHSO_4$. The EtOAc layer was washed 3X with 5% KHSO4, then with brine, then dried ($Na_2SO_4$), filtered and stripped to give a colorless oil (phosphinic acid monomethyl ester). The oil was dissolved in dry $CH_2Cl_2$ (10 ml) and treated with N,N-diethyltrimethylsilylamine (930 ul, 713 gm, 4.91 mmol). After stirring at room temperature for one hour, the solvent was removed *in vacuo* and the residue was azeotroped with dry benzene (10 ml). The residue was dissolved in dry $CH_2Cl_2$ (10 ml), cooled to -10°C and treated with 2 drops DMF and oxalyl chloride (235 ul). After 15 minutes, the solution was warmed to room temperature and stirred for an additional 50 minutes. The solvent was then stripped and the yellow residue (phosphinylchloridate) was azeotroped with benzene (20 ml) and dried *in vacuo* (oil pump) for 30 minutes.

Meanwhile, a solution of (E)-2-(4-Fluorophenyl)-3-(2-iodoethenyl)-4-(1-methylethyl)quinoline (600 mg, 1.44 mmol) in THF (2 ml) was added over a 2 minute period to a -78°C solution of t-butyllithium (1.7 M in pentane, 1.78 ml, 3.03 mmol) in THF (7 ml). The resulting green-brown solution was stirred for 20 minutes then cooled to -100°C. The vinyl anion solution was added over a 30 second period to a -100°C solution of the phosphinylchloridate in THF (10 ml). The resulting orange mixture was stirred at -100°C for 10 minutes and at -78°C for 20 minutes, then quenched with 50% saturated $NH_4Cl$. The solution was warmed to room temperature, diluted with $H_2O$, poured into saturated $NaHCO_3$. The aqueous phase was extracted 2X with $Et_2O$. The combined $Et_2O$ layers were washed with brine, dried ($Na_2SO_4$), filtered and stripped. The resulting yellow oil was chromatographed (flash, Merck $SiO_2$, 20% acetone in hexane followed by 30% acetone in hexane) to afford (S,E)-3-[[(1,1-Dimethylethyl)diphenylsilyl]-4-[[2-[2-(4-fluorophenyl)-4-(1-methylethyl)-3-quinolinyl]ethenyl]methoxyphosphinyl]butanoic acid, methyl esteras a white foam (561 mg, 54%).

TLC: $R_f$ 0.31 (1:2 acetone:hexane)

C. (S,E)-4-[[2-[2-(4-Fluorophenyl)-4-(1-methylethyl)-3-quinolinyl]-ethenyl]methoxyphosphinyl]-3-hydroxybutanoic acid, methyl ester

A solution of (S,E)-3-[[(1,1-Dimethylethyl)diphenylsilyl]-4-[[2-[2-(4-fluorophenyl)-4-(1-methylethyl)-3-quinolinyl]ethenyl]methoxyphosphinyl]butanoic acid, methyl ester(542 mg, 0.749 mmol) in THF (10 ml) was treated with HOAc (215 ul, 226 mg, 3.76 mmol) and tetra-n-butylammonium fluoride (1.0 M in THF, 2.25 ml, 2.25 mmol). After stirring at room temperature for 22 hours, the solution was poured into saturated $NaHCO_3$ and extracted with EtOAc. The EtOAc extract was washed with brine, dried ($Na_2SO_4$), filtered and stripped to give an oil which was subsequently chromatographed (flash, Merck, $SiO_2$, 1:1 acetone: hexane followed by 20% hexane in acetone). (S,E)-4-[[2-[2-(4-Fluorophenyl)-4-(1-methylethyl)-3-quinolinyl]-ethenyl]-methoxyphosphinyl]-3-hydroxybutanoic acid; methyl ester (265 mg, 73%) was obtained as a white foam.

TLC: $R_f$ 0.28 (1:1 acetone:hexane)

D. [3S,4(E)]-4-[[2-[2-(4-Fluorophenyl)-4-(1-methylethyl)-3-quinolinyl]ethenyl]hydroxyphosphinyl]-3-hydroxybutanoic acid, dilithium salt

A solution of (S,E)-4-[[2-[2-(4-Fluorophenyl)-4-(1-methyl-ethyl)-3-quinolinyl]-ethenyl]methoxyphosphinyl]-3-hydroxybutanoic acid, methyl ester (255 mg, 0.525 mmol) in dioxane (3 ml) was treated with 1 N LiOH (1.90 ml, 1.90 mmol) at room temperature and the mixture was subsequently heated at 55°C under argon for 1.25 hours. The solvent was evaporated and the residue was chromatographed on HP-20 eluting in succession with $H_2O$ (200 ml), 25% MeOH in $H_2O$ (100 ml), and 50% MeOH in $H_2O$ (200 ml). The desired fractions were pooled and evaporated and the residue was taken up in $H_2O$ and lyophilized to give [3S,4(E)-]-4-[[2-[2-(4-Fluorophenyl)-4-(1-methylethyl)-3-quinolinyl]ethenyl]hydroxy-phosphinyl]-3-hydroxybutanoic acid, dilithium salt (232 mg, 89%) as a white solid.

TLC: $R_f$ 0.40 (7:2:1, i-PrOH, $NH_4OH$, $H_2O$)

## Example 11

[3S,4(E)]-4-[[2-[4-(4-Fluorophenyl)-2-(1-methylethyl)-3-quinolinyl]ethenyl]hydroxy-phosphinyl]-3-hydroxybutanoic acid, dilithium salt

A. (E)-4-(4-Fluorophenyl)-3-(2-iodoethenyl)-2-(1-methylethyl)quinoline

A mixture of 3-Ethynyl-4-(4-fluorophenyl)-2-(1-methylethyl)quinoline (1.500 gm, 5.18 mmol) (the preparation of which is described in Example 8) and AIBN (15 mg) in tri-n-butylstannyl hydride (2.1 ml) was rapidly heated to 120 °C. After 3 minutes of heating, the mixture was treated with additional $Bu_3SnH$ (0.6 ml) and the temperature of the reaction was raised to 130 °C. Approximately 15 mg of AIBN was added to the reaction mixture of 0.5, 1, and 2 hours after heating was initiated. After 2.5 hours, the mixture was cooled to room temperature, diluted with $Et_2O$ (30 ml) and treated with solid $I_2$ (3.86 gm, 15.2 mmol). The dark reaction mixture was stirred for 1 hour, then quenched with 10% $Na_2SO_3$ in saturated $NaHCO_3$. The layers were shaken and separated. The ethereal layer was washed with brine, dried ($Na_2SO_4$), filtered and stripped to yield a liquid/solid residue. The residue was chromatographed (flash, Merck $SiO_2$, 4% EtOAc in hexane) to give slightly impure (E)-4-(4-Fluorophenyl)-3-(2-iodoethenyl)-2-(1-methylethyl)quinoline as a solid. Recrystallization of the solid from hot hexane gave (E)-4-(4-Fluorophenyl)-3-(2-iodoethenyl)-2-(1-methylethyl)quinoline (1.725 gm, 80%) as light yellow crystals.
m.p. 129.4-131 °C
TLC: $R_f$ 0.22 (4% EtOAc in hexane)

B. (S,E)-3-[[(1,1-Dimethylethyl)diphenylsilyl]-oxy]-4-[[2-[4-(4-fluorophenyl)-2-(1-methylethyl)-3-quinolinyl]-ethenyl]methoxyphosphinyl]butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (4.501 gm, 7.123 mmol) (3.86 gm, 6.11 mmol) the preparation of which is described at the end of example 7, was partitioned between EtOAc and 5% $KHSO_4$. The EtOAc layer was washed 3X with 5% $KHSO_4$, then with brine, then dried ($Na_3SO_4$), filtered and stripped to give a colorless oil (phosphinic acid monomethyl ester). The oil was dissolved in dry $CH_2Cl_2$ (15 ml) and treated with N,N-diethyltrimethylsilylamine (2.32 ml, 1.78 gm, 12.2 mmol). After stirring at room temperature for one hour, the solvent was removed *in vacuo* and the residue was azeotroped with dry benzene (20 ml). The residue was dissolved in dry $CH_2Cl_2$ (15 ml), cooled to -10 °C and treated with 2 drops DMF and oxalyl chloride (590 ul, 858 mg, 6.76 mmol). After 15 minutes, the solution was warmed to room temperature and stirred for an additional 50 minutes. The solvent was then stripped and the yellow residue (phosphinylchloridate) was azeotroped with benzene (20 ml) and dried *in vacuo* (oil pump) for 30 minutes.

Meanwhile, a solution of (E)-4-(4-Fluorophenyl)-3-(2-iodoethenyl)-2-(1-methylethyl)quinoline (1.500 mg, 3.59 mmol) in THF (3 ml) was added over a 3 minute period to a -78 °C solution of t-butyllithium (1.7 M in pentane, 4.40 ml, 7.48 mmol) in THF (10 ml). The resulting deep green solution was stirred for 20 minutes then cooled to -100 °C. The vinyl anion solution was added over a one minute period to a -100 °C solution of the phosphinylchloridate in THF (15 ml). The resulting mixture was stirred at -100 °C for 10 minutes and at -78 °C for 20 minutes, then quenched with 50% saturated $NH_4Cl$. The solution was warmed to room temperature, diluted with $H_2O$, and poured into saturated $NaHCO_3$. The aqueous phase was extracted 2X with $Et_2O$. The combined $Et_2O$ layers were washed with brine, dried ($Na_2SO_4$), filtered and stripped. The resulting yellow oil was chromatographed (flash, Merck $SiO_2$, 40% EtOAc in hexane followed by 1:1 EtOAc:hexane) to afford (S,E)-3-[[(1,1-Dimethylethyl)diphenylsilyl]oxy]-4-[[2-[4-(4-fluorophenyl)-2-(1-methylethyl)-3-quinolinyl]ethenyl]methoxyphosphinyl]butanoic acid, methyl ester as an off-white foam (1.615 gm, 62%).
TLC: $R_f$ 0.28 (1:1 EtoAc:hexane)

C. (S,E)-4-[[2-[4-(4-Fluorophenyl)-2-(1-methylethyl)-3-quinolinyl]-ethenyl]methoxyphosphinyl]-3-hydroxybutanoic acid, methyl ester

A solution of (S,E)-3-[[(1,1-Dimethylethyl)diphenylsilyl]-oxy]-4-[[2-[4-(4-fluorophenyl)-2-(1-methylethyl)-3-quinolinyl]ethenyl]methoxyphosphinyl]butanoic acid, methyl ester (1.136 gm, 1.57 mmol) in THF (14 ml) was treated with HOAc (450 ul, 472 mg, 7.9 mmol) and tetra-n-butylammonium fluoride (1.0 M in THF, 5.40 ml, 5.40 mmol). After stirring at room temperature for 18 hours, the solution was poured into saturated $NaHCO_3$ and extracted with EtOAc. The EtOAc extract was washed with brine, dried ($Na_2SO_4$), filtered, and stripped to give an oil which was subsequently chromatographed (flash, Merck $SiO_2$, 1:1 acetone: hexane). (S,E)-4-[[2-[4-(4-Fluorophenyl)-2-(1-methylethyl)-3-quinolinyl]-ethenyl]methoxyphosphinyl]-3-hydroxybutanoic acid, methyl ester(709 mg, 93%) was obtained as a colorless oil.

TLC: $R_f$ 0.32 (1:1 acetone:hexane)

D. [3S,4(E)]-4-[[2-[4-(4-Fluorophenyl)-2-(1-methylethyl)-3-quinolinyl]ethenyl]hydroxyphosphinyl]-3-hydroxybutanoic acid, dilithium salt

A solution of (S,E)-4-[[2-[4-(4-Fluorophenyl)-2-(1-methylethyl)-3-quinolinyl]-ethenyl]methoxyphosphinyl]-3-hydroxybutanoic acid, methyl ester (240 mg, 0.496 mmol) in dioxane (3 ml) was treated with 1 N LiOH (1.70 ml, 1.70 mmol) at room temperature and the mixture was subsequently heated at 55° C under argon for 1.5 hours. The solvent was evaporated and the residue was chromatographed on HP-20 on HP-20 eluting in succession with $H_2O$ (200 ml), 25% MeOH in $H_2O$ (100 ml), and 50% MeOH in $H_2O$ (200 ml). The desired fractions were pooled and evaporated and the residue was taken up in $H_2O$ and lyophilzed to give [3S,4(E)]-4-[[2-[4-(4-Fluorophenyl)-2-(1-methylethyl)-3-quinolinyl]ethenyl]hydroxyphosphinyl]-3-hydroxybutanoic acid, dilithium salt (210 mg, 86%) as a white solid.
TLC: $R_f$ 0.36 (7:2:1, i-PrOH, $NH_4OH$, $H_2O$)

## Example 12

(S)-4-[[[2-(4-Fluorophenyl)-4-(1-methyl-ethyl)-3-quinolinyl]ethynyl]hydroxyphosphinyl]-3-hydroxybutanoic acid, 1-oxide, dilithium salt.

A. (S)-4-[[[2-(4-Fluorophenyl)-4-(1-methylethyl)-3-quinolinyl]ethynyl]methoxyphosphinyl]-3-hydroxybutanoic acid, methyl ester. 1-oxide

A solution of (S)-4-[[[2-(4-Fluorophenyl)-4-(1-methylethyl)-3-quinolinyl]ethynyl]methoxyphosphinyl]-3-hydroxybutanoic acid, methyl ester (486 mg, 1.00 mmol) (the preparation of which is described in Example 5) and m-CPBA (80-85%, 846 mg) in dry $CH_2Cl_2$ (20 ml) was stirred at room temperature for 15 hours. The mixture was diluted with $Et_2O$ and washed with 70 ml saturated $NaHCO_3$ containing 4.0 gm $Na_2SO_3$. The organic layer was subsequently washed with brine, dried ($Na_2SO_4$), filtered and stripped to yield a yellow oil. The oil was chromatographed (flash, Merck $SiO_2$, 30% hexane in acetone) giving (S)-4-[[[2-(4-Fluorophenyl)-4-(1-methylethyl)-3-quinolinyl]ethynyl]methoxyphosphinyl]-3-hydroxybutanoic acid, methyl ester. 1-oxide (404 mg, 81%) as a light yellow oily foam.
TLC: $R_f$ 0.15 (1:1 acetone:hexane)

B. (S)-4-[[[2-(4-Fluorophenyl)-4-(1-methylethyl)-3-quinolinyl]ethynyl]hydroxyphosphinyl]-3-hydroxybutanoic acid, 1-oxide, dilithium salt

A solution of (S)-4-[[[2-(4-Fluorophenyl)-4-(1-methylethyl)-3-quinolinyl]ethynyl]methoxyphosphinyl]-3-hydroxybutanoic acid, methyl ester. 1-oxide (395 mg, 0.79 mmol) in dioxane (5.0 ml) was treated with 1 N LiOH (3.0 ml, 3.0 mmol) at room temperature and the mixture was subsequently heated at 55° C under argon for 1.25 hours. The solvent was evaporated and the residue was chromatographed on HP-20 eluting in succession with $H_2O$ (200 ml), 25% MeOH in $H_2O$ (100 ml), and 50% MeOH in $H_2O$ (200 ml). The desired fractions were pooled and evaporated and the residue was taken up in $H_2O$ and lyophilized to give (S)-4-[[[2-(4-Fluorophenyl)-4-(1-methyl-ethyl)-3-quinolinyl]ethynyl]hydroxyphosphinyl]-3-hydroxybutanoic acid, 1-oxide, dilithium salt (333 mg, 81%) as a dense yellow solid.
TLC: $R_f$ 0.33 (7:2:1, i-PrOH, $NH_4OH$, $H_2O$)

## Example 13

[3S, 4(E)]-4-[[2-[4-(4-Fluorophenyl)-2-(1-methylethyl)-3-quinolinyl]ethenyl]hydroxyphosphinyl]-3-hydroxybutanoic acid, 1-oxide, dilithium salt

A. (S,E)-4-[[2-[4-(4-Fluorophenyl)-2-(1-methylethyl)-3-quinolinyl]ethenyl]methoxyphosphinyl]-3-hydroxybutanoic acid, methyl ester. 1-oxide

A solution of (S,E)-4-[[2-[4-(4-Fluorophenyl)-2-(1-methylethyl)-3-quinolinyl]ethenyl]methoxyphosphinyl]-3-hydroxybutanoic acid, methyl ester (438 mg, 0.906 mmol) (the preparation of which is described in Example 11) and m-CPBA (80-85%, 755 mg) in dry $CH_2Cl_2$ (16 ml) was stirred at room temperature for 2 hours. Additional m-CPBA (285 mg) was added and stirring was continued for another 2.5 hours. The mixture was diluted with EtOAc and washed with 75 ml saturated $NaHCO_3$ containing 5.0 gm $Na_2SO_3$. The organic layer was subsequently washed with brine, dried ($Na_2SO_4$), filtered and stripped to yield an orange-brown residue. The residue was chromatographed (flash, Merck $SiO_2$, 30% hexane in acetone followed by 100% acetone) giving (S,E)-4-[[2-[4-(4-Fluorophenyl)-2-(1-methylethyl)-3-quinolinyl]ethenyl]-methoxyphosphinyl]-3-hydroxybutanoic acid, methyl ester. 1-oxide (298 mg, 66%) as a yellow gum.
TLC: $R_f$ 0.12 (1:1 acetone:hexane)

B. [3S, 4(E)]-4-[[2-[4-(4-Fluorophenyl)-2-(1-methylethyl)-3-quinolinyl]ethenyl]hydroxyphosphinyl]-3-hydroxybutanoic acid, 1-oxide, dilithium salt

A solution of (S,E)-4-[[2-[4-(4-Fluorophenyl)-2-(1-methylethyl)-3-quinolinyl]ethenyl]methoxyphosphinyl]-3-hydroxybutanoic acid, methyl ester. 1-oxide(282 mg, 0.56 mmol) in dioxane (4.0 ml) was treated with 1 N LiOH (1.8 ml, 1.8 mmol) at room temperature and the mixture was subsequently heated at 55 °C under argon for 1.5 hours. The solvent was evaporated and the residue was chromatographed on HP-20 eluting in succession with $H_2O$ (200 ml), 25% MeOH in $H_2O$ (100 ml), and 50% MeOH in $H_2O$ (200 ml). The desired fractions were pooled and evaporated and the residue was taken up in $H_2O$ and lyophilized to give [3S, 4-(E)]-4-[[2-[4-(4-Fluorophenyl)-2-(1-methylethyl)-3-quinolinyl]ethenyl]hydroxyphosphinyl]-3-hydroxybutanoic acid, 1-oxide, dilithium salt (260 mg, 88%) as a dense pale yellow solid.
TLC: $R_f$ 0.37 (7:2:1, i-PrOH, $NH_4OH$, $H_2O$)

## Example 14

(S)-4-[[[2-(4-Fluorophenyl)-4-(1-methylethyl)-6-phenyl-3-pyridinyl]ethynyl]hydroxyphosphinyl]-3-hydroxybutanoic acid, dilithium salt

A. 3-(2,2-Dibromoethenyl)-2-(4-fluorophenyl)-4-(1-methylethyl)-6-phenylpyridine

A solution of carbon tetrabromide (2.866 gm, 8.64 mmol) in $CH_2Cl_2$ (8 ml) was added over a 10 minute period to a cold (-10 °C) solution of 2-(4-Fluorophenyl)-4-(1-methylethyl)-6-phenyl-3-pyridinecarboxaldehyde (1.840 gm, 5.76 mmol) (the preparation of which is described in Example 3) and triphenylphosphine (4.590 gm, 17.5 mmol) in $CH_2Cl_2$ (30 ml). After the addition was complete, the cooling bath was removed and the mixture was stirred for 20 minutes. The solution was quenched with saturated $NaHCO_3$ and extracted 2X with $CH_2Cl_2$. The combined organic layers were washed with saturated $NaHCO_3$, dried ($Na_2SO_4$), filtered and concentrated. The concentrate was chromatographed (flash, Merck, $SiO_2$, 1:1 $CH_2Cl_2$: hexane) to give 3-(2,2-Dibromoethenyl)-2-(4-fluorophenyl)-4-(1-methylethyl)-6-phenylpyridine as a white solid (2.701 gm, 99%).
m.p. 124-126 °C
TLC: $R_f$ 0.28 (1:1 $CH_2Cl_2$:hexane)

B. 3-Ethynyl-2-(4-fluorophenyl)-4-(1-methylethyl)-6-phenylpyridine

A solution of 3-(2,2-Dibromoethenyl)-2-(4-fluorophenyl)-4-(1-methylethyl)-6-phenylpyridine (2.677 gm,

5.63 mmol) in dry THF (25 ml) was cooled to -78° C and treated with n-BuLi (1.6 M in hexane, 7.75 ml, 12.4 mmol) over a 1 minute period. After stirring at -78° C for one hours, the deep green solution was quenched with saturated $NH_4Cl$, warmed to room temperature, diluted with $H_2O$, and extracted once with $Et_2O$. The $Et_2O$ extract was washed with brine, dried ($Na_2SO_4$), filtered and stripped to give a yellow solid. The solid was recrystallized from a minimum amount of hot hexane to afford analytically pure 3-Ethynyl-2-(4-fluorophenyl)-4-(1-methylethyl)-6-phenylpyridine as yellow crystals (1.572 gm, 89%).
m.p. 121-123.5° C
TLC: $R_f$ 0.33 (5% EtOAc in hexane)

C. (S)-3-[[1,1-Dimethylethyl)diphenylsilyl]oxy]-4-[[[2-(4-fluorophenyl)-4-(1-methylethyl)-6-phenyl-3-pyridinyl]-ethynyl]-methoxyphosphinyl]butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (4.501 gm, 7.123 mmol) (2.018 gm, 3.19 mmol) the preparation of which is described at the end of example 7, was partitioned between EtOAc and 5% $KHSO_4$. The EtOAc layer was washed 3X with $KHSO_4$, then with brine, then dried ($Na_2SO_4$), filtered and stripped to give a colorless oil (phosphinic acid monomethyl ester). The oil was dissolved in dry $CH_2Cl_2$ (10 ml) and treated with N,N-diethyltrimethylsilylamine (1.22 ml, 0.94 gm, 6.44 mmol). After stirring at room temperature for one hour, the solvent was removed *in vacuo* and the residue was azeotroped with dry benzene (10 ml). The residue was redissolved in dry $CH_2Cl_2$ (10 ml), cooled to -12° C and treated with 2 drops DMF and oxalyl chloride (307 ul, 447 mg, 3.52 mmol). After 15 minutes, the solution was warmed to room temperature and stirred for an additional 45 minutes. The solvent was stripped and the yellow residue (phosphinylchloridate) was azeotroped with benzene (20 ml) and dried *in vacuo* (oil pump) for 30 minutes.

Meanwhile, a solution of 3-Ethynyl-2-(4-fluorophenyl)-4-(1-methylethyl)-6-phenylpyridine (599 mg, 1.9 mmol) in THF (8 ml) at -78° C was treated with n-BuLi (1.6 M in hexane, 1.30 ml, 2.08 mmol) and the resulting clear green mix:ture was stirred for 30 minutes. The acetylenic anion solution was added dropwise *via canula* over a 2 minute period to a -78° C solution of the phosphinylchloridate in THF (12 ml). The resulting yellow-brown mixture was stirred at -78° C for 35 minutes, then quenched with 50% saturated $NH_4Cl$. The solution was warmed to room temperature, diluted with $H_2O$, and poured into saturated $NaHCO_3$. The aqueous phase was extracted once with $Et_2O$. The $Et_2O$ extract was washed with brine, dried ($Na_2SO_4$), filtered and stripped. The residual oil was chromatographed (flash, Merck $SiO_2$, 1:1 EtOAc:hexane) to afford (S)-3-[[1,1-Dimethylethyl)diphenylsilyl]oxy]-4-[[[2-(4-fluorophenyl)-4-(1-methylethyl)-6-phenyl-3-pyridinyl]ethynyl]methoxyphosphinyl]butanoic acid, methyl ester as a yellow-white foam (773 mg, 55%).
TLC: $R_f$ 0.18 (40% EtOAc in hexane)

D. (S)-4-[[[2-(4-Fluorophenyl)-4-(1-methylethyl)-6-phenyl-3-pyridinyl]ethynyl]methoxyphosphinyl]-3-hydroxybutanoic acid, methyl ester

A mixture of (S)-3-[[1,1-Dimethylethyl)diphenylsilyl]oxy]-4-[[[2-(4-fluorophenyl)-4-(1-methylethyl)-6-phenyl-3-pyridinyl]ethynyl]methoxyphosphinyl]butanoic acid, methyl ester (750 mg, 1.00 mmol) tetra-n-butylammonium fluoride (1.0 M in THF, 3.50 mmol), and HOAc (330 mg, 5.50 mmol) in THF (12 ml) was stirred at room temperature for 15 hours. The solution was partitioned between 5% $KHSO_4$ and EtOAc. The layers were shaken and separated and the EtOAc layer was washed again with 5% $KHSO_4$. The pooled aqueous washings were back-extracted twice with EtOAc. The combined EtOAc layers were washed with brine and dried ($Na_2SO_4$). Filtration and removal the solvent afforded a yellow oil. The oil was dissolved in $Et_2O$ and treated with excess diazomethane for 20 minutes. The excess diazomethane was destroyed by the addition of HOAc and the solvent was removed *in vacuo*. The residue was chromatographed (flash, Merck $SiO_2$, 40% acetone in hexane followed by 1:1 acetone:hexane) to afford (S)-4-[[[2-(4-Fluorophenyl)-4-(1-methylethyl)-6-phenyl-3-pyridinyl]ethynyl]methoxyphos phinyl]-3-hydroxybutanoic acid, methyl ester (446 mg, 88%) as a colorless oil.
TLC: $R_f$ 0.34 (1:1 acetone:hexane)

E. (S)-4-[[[2-(4-Fluorophenyl)-4-(1-methylethyl)-6-phenyl-3-pyridinyl]ethynyl]hydroxyphosphinyl]-3-hydroxybutanoic acid, dilithium salt

A solution of (S)-4-[[[2-(4-Fluorophenyl)-4-(1-methylethyl)-6-phenyl-3-pyridinyl]ethynyl]-methoxyphosphinyl]-3-hydroxybutanoic acid, methyl ester (436 mg, 0.86 mmol) in dioxane (5 ml) was treated with 1 N LiOH (2.6 ml, 2.6 mmol) at room temperature and the mixture was subsequently heated at 55° C under argon for 1.5 hours. The solvent was evaporated and the residue was chromatographed on HP-20 eluting in succession with $H_2O$ (200 ml), 25% MeOH in $H_2O$ (100 ml), 50% MeOH in $H_2O$ (250 ml). The desired fractions were pooled and evaporated and the residue was taken up in $H_2O$ and lyophilized to give (S)-4-[[[2-(4-Fluorophenyl)-4-(1-methylethyl)-6-phenyl-3-pyridinyl]ethynyl]hydroxyphosphinyl]-3-hydroxybutanoic acid, dilithium salt (369 mg, 84%) as a white solid.
TLC: $R_f$ 0.57 (7:2:1, i-PrOH, $NH_4OH$, $H_2O$)

## Example 15

### [3S,4(E)]-4-[[2-[2-(4-Fluorophenyl)-4-(1-methylethyl)-6-phenyl-3-pyridinyl]ethenyl]hydroxyphosphinyl]-3-hydroxybutanoic acid, dilithium salt.

#### A. (E)-2-(4-Fluorophenyl)-3-(2-iodoethenyl)-4-(1-methylethyl)-6-phenyl-pyridine

A mixture of 3-Ethynyl-2-(4-fluorophenyl)-4-(1-methylethyl)-6-phenylpyridine (700 mg, 2.22 mmol) (the preparation of which is described in Example 14)and AIBN (11 mg) in tri-n-butylstannyl hydride (1.40 ml) was rapidly heated to 120° C. After 5 minutes of heating, the temperature of the reaction was raised to 140° C and approximately 12 mg of AIBN was added to the reaction mixture every 0.5 hour thereafter. After 2.5 hours, the mixture was cooled to room temperature, diluted with $Et_2O$ (20 ml) and treated with solid $I_2$ - (2.00 gm, 7.9 mmol). The dark reaction mixture was stirred for one hour, then quenched with 60 ml saturated $NaHCO_3$ containing 4 gm $Na_2SO_3$. The layers were shaken and separated. The ethereal layer was washed with brine, dried ($Na_2SO_4$), filtered and stripped to yield a yellow oil. The oil was chromatographed twice (Merck $SiO_2$, 5% EtOAc in hexane (1st) then 3% EtOAc in hexane (2nd)) to (E)-2-(4-Fluorophenyl)-3-(2-iodoethenyl)-4-(1-methylethyl)-6-phenyl-pyridine as a foam which solidified (671 mg). Recrystallization of the solid from hot hexane gave 482 mg of (E)-2-(4-Fluorophenyl)-3-(2-iodoethenyl)-4-(1-methylethyl)-6-phenyl-pyridine. An additional 100 mg of 2 was obtained from the mother liquor to give a total of 582 mg (59%) product.
m.p. 110-111° C
TLC: $R_f$ 0.39 (10% EtOAC in hexane)

#### B. (S,E)-3-[[1,1-Dimethylethyl)diphenylsilyl]oxy]-4-[[2-[2-(4-fluorophenyl)-4-(1-methylethyl)-6-phenyl-3-pyridinyl]ethenyl]methoxyphosphinyl]butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (4.501 gm, 7.123 mmol) (1.211 gm, 1.92 mmol) the preparation of which is described at the end of example 7, was partitioned between EtOAc and 5% $KHSO_4$. The EtOAc layer was washed 3X with 5% $KHSO_4$, then with brine, then dried ($Na_2SO_4$), filtered and stripped to give a colorless oil (phosphinic acid monomethyl ester). The oil was dissovled in dry $CH_2Cl_2$ (10 ml) and treated with N,N-diethyltrimethylsilylamine (727 ul, 558 mg, 3.84 mmol). After stirring at room temperature for one hour, the solvent was removed *in vacuo* and the residue wa azeotroped with dry benzene (10 ml). The residue was dissolved in dry $CH_2Cl_2$ (10 ml) cooled to -10° C and treated with 2 drops DMF and oxalyl chloride (184 ul, 268 mg, 2.11 mmol). After 15 minutes, the solution was warmed to room temperature and stirred for an additional 50 minutes. The solvent was then stripped and the yellow residue (phosphinylchloridate) was azeotroped with benzene (10 ml) and dried *in vacuo* (oil pump) for 30 minutes.

Meanwhile, a solution of (E)-2-(4-Fluorophenyl)-3-(2-iodoethenyl)-4-(1-methylethyl)-6-phenyl-pyridine (502 mg, 1.13 mmol) in THF (2 ml) was added over a 2 minute period to a -78° C solution of t-butyllithium (1.7 M in pentane, 1.46 ml, 2.48 mmol) in THF (6 ml). The mixture was stirred for 20 minutes then cooled to -100° C. The vinyl anion solution was added over a one minute period to a -100° C solution of the phosphinylchloridate in THF (10 ml). The resulting mixture was stirred at -100° C for 30 minutes and at -78° C for 30 minutes, then quenched with 50% saturated $NH_4Cl$. The solution was warmed to room

temperature, diluted with $H_2O$, and poured into saturated $NaHCO_3$. The aqueous phase was extracted 2X with $Et_2O$. The combined $Et_2O$ layers were washed with brine, dried ($Na_2SO_4$), filtered and stripped. The resulting yellow oil was chromatographed (flash, Merck $SiO_2$, 15% acetone in hexane followed by 20% acetone in hexane) to afford (S,E)-3-[[1,1-Dimethylethyl)diphenylsilyl]oxy]-4-[[2-[2-(4-fluorophenyl)-4-(1-methylethyl)-6-phenyl-3-pyridinyl]ethenyl]methoxyphosphinyl]butanoic acid, methyl esteras an off-white foam (256 mg, 30%).
TLC: $R_f$ 0.29 (1:2 acetone:hexane)

C. (S,E)-4-[[2-[2-(4-Fluorophenyl)-4-(1-methylethyl)-6-phenyl-3-pyridinyl]ethenyl]methoxyphosphinyl]butanoic acid, methyl ester

A solution of (S,E)-3-[[1,1-Dimethylethyl)diphenylsilyl]oxy]-4-[[2-[2-(4-fluorophenyl)-4-(1-methylethyl)-6-phenyl-3-pyridinyl]ethenyl]methoxyphosphinyl]butanoic acid, methyl ester (241 mg, 0.321 mmol) in THF (7 ml) was treated with HOAc (92 ul, 96 mg, 1.61 mmol) and tetra-n-butylammonium fluoride (1.0 M in THF, 963 ul, 0.963 mmol). After stirring at room temperature for 18 hours, the solution was poured into saturated $NaHCO_3$ and extracted with EtOAc. The EtOAc extract was washed with brine, dried, ($Na_2SO_4$), filtered, and stripped to give an oil which was subsequently chromatographed (flash, Merck $SiO_2$, 1:1 acetone:hexane followed 100% acetone). (S,E)-4-[[2-[2-(4-Fluorophenyl)-4-(1-methylethyl)-6-phenyl-3-pyridinyl]ethenyl]-methoxyphosphinyl]butanoic acid, methyl ester (124 mg, 76%) was obtained as a colorless oil.
TLC: $R_f$ 0.29 (1:1 acetone:hexane)

D. [3S,4(E)]-4-[[2-[2-(4-Fluorophenyl)-4-(1-methylethyl)-6-phenyl-3-pyridinyl]ethenyl]-hydroxyphosphinyl]-3-hydroxybutanoic acid, dilithium salt

A solution of (S,E)-4-[[2-[2-(4-Fluorophenyl)-4-(1-methylethyl)-6-phenyl-3-pyridinyl]ethenyl]-methoxyphosphinyl]butanoic acid, methyl ester(120 mg, 0.234 mmol) in dioxane (2 ml) was treated with 1 N LiOH (0.9 ml, 0.9 mmol) at room temperature and the mixture was subseqently heated at 55 °C under argon for 1.5 hours. The solvent was evaporated and the residue was chromatographed on HP-20 eluting in succession in $H_2O$ (150 ml), 25% MeOH in $H_2O$ (75 ml) and 50% MeOH in $H_2O$ (200 ml). The desired fractions were pooled evaporated and the residue was taken up in $H_2O$ and lyophilized to give [3S,4(E)]-4-[-[2-[2-(4-Fluorophenyl)-4-(1-methylethyl)-6-phenyl-3-pyridinyl]ethenyl]hydroxyphosphinyl]-3-hydroxybutanoic acid, dilithium salt (108 mg, 86%) as a white solid.
TLC: $R_f$ 0.52 (7:2:1, i-PrOH, $NH_4OH$, $H_2O$)

## Examples 16 to 51

Following the procedures as outlined heretofore and as described in the previous working examples, the following additional compounds may be prepared.

Examples of Structure 1 where Am = $A_1$, X = C≡C

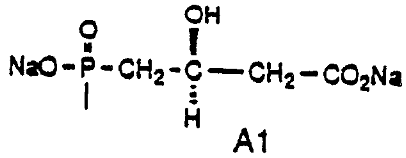

A1

| Ex. # | Structure 1 | | Structure 1 |
|---|---|---|---|
| 16 | 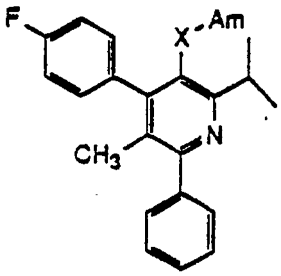 | 19 | 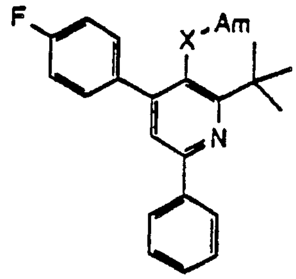 |
| 17 | 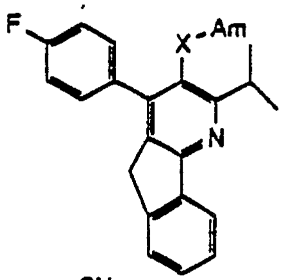 | 20 | 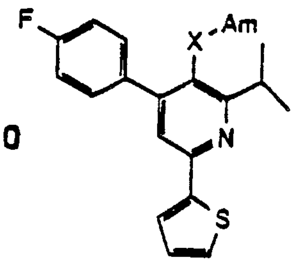 |
| 18 | 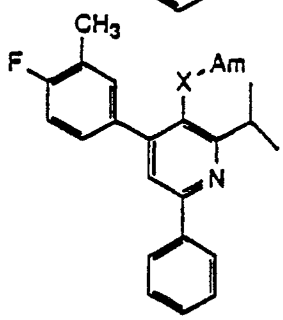 | 21 | 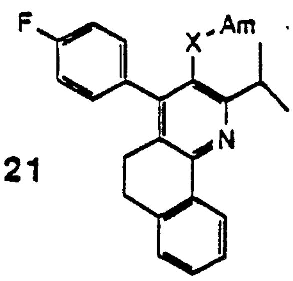 |

Examples of Structure 1 where Am = $A_1$, X = C≡C

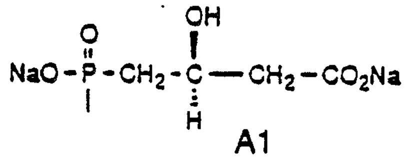

A1

Ex. # Structure 1 Structure 1

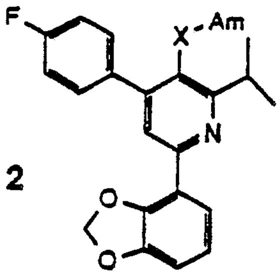

22

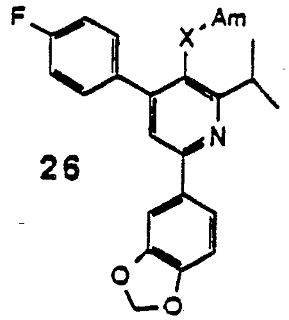

26

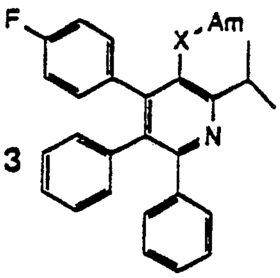

23

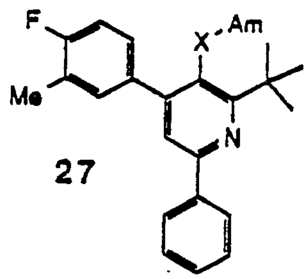

27

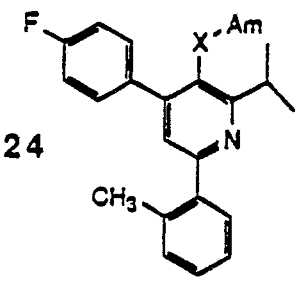

24

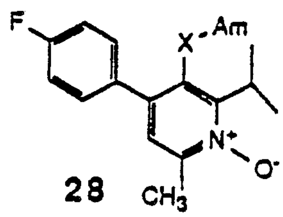

28

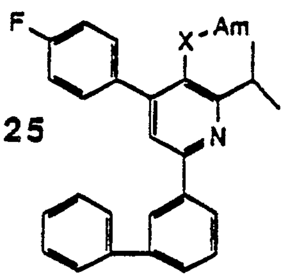

25

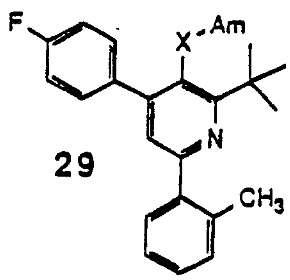

29

Examples of Structure 1 where Am = $A_1$, X = C≡C

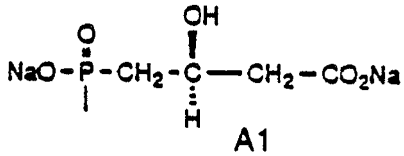

A1

Ex. # Structure 1 Structure 1

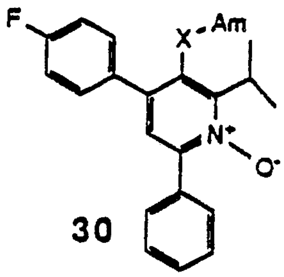

30

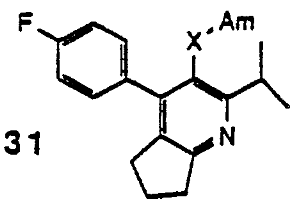

31

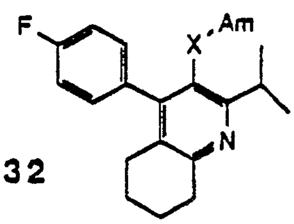

32

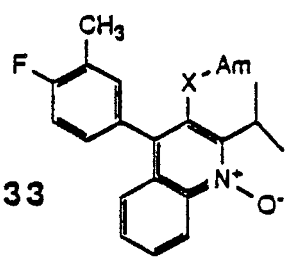

33

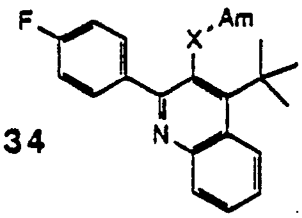

34

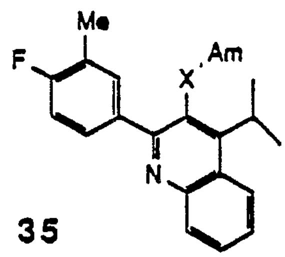

35

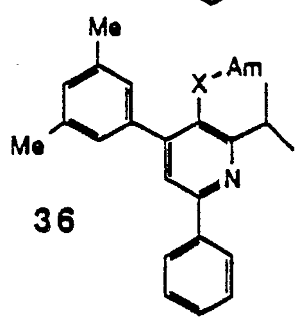

36

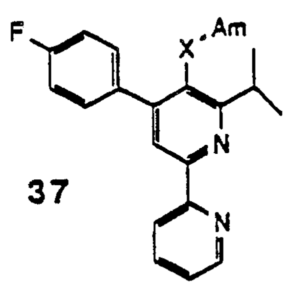

37

Examples of Structure 1 where Am = $A_1$, X = C≡C

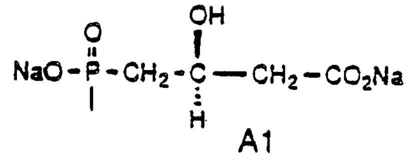

A1

| Ex. # | Structure 1 | | Structure 1 |
|---|---|---|---|
| 38 | 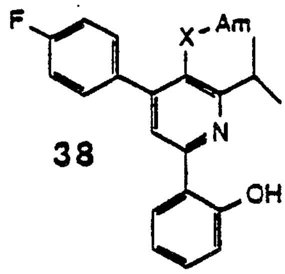 | 39 | 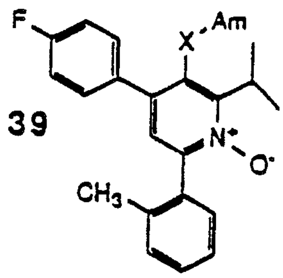 |

Examples of Structure 1 where Am = $A_2$, X = CH=CH(E)

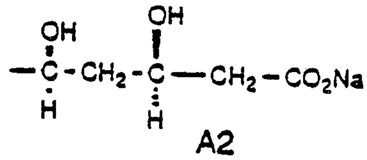

A2

| Ex. # | Structure 1 | | Structure 1 |
|---|---|---|---|
| 40 | 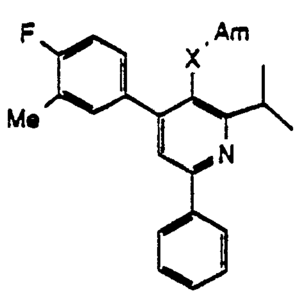 | 44 | 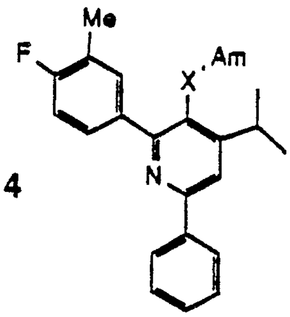 |
| 41 | 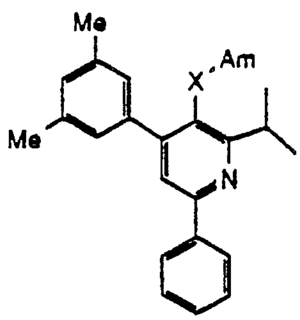 | 45 | 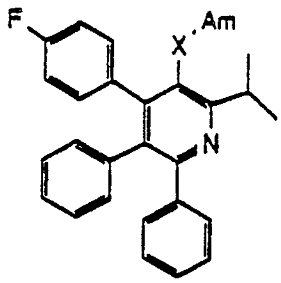 |
| 42 | 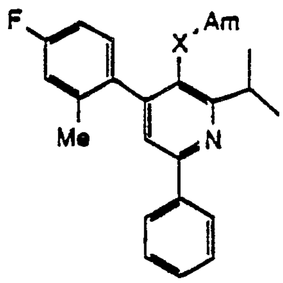 | 46 | 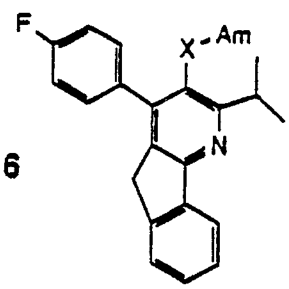 |
| 43 | 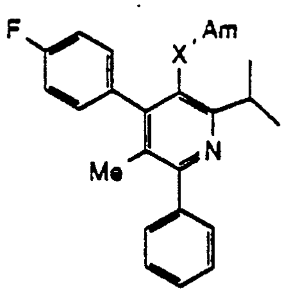 | 47 | 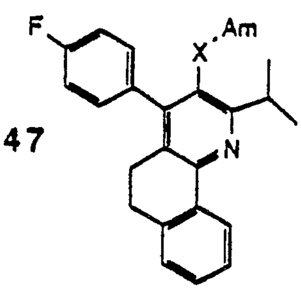 |

Examples of Structure 1 where Am = $A_2$, X = CH=CH(E)

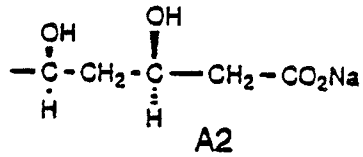

A2

| Ex. # | Structure 1 | Structure 1 |
|---|---|---|
| | 48 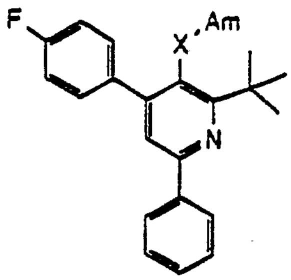 | 50 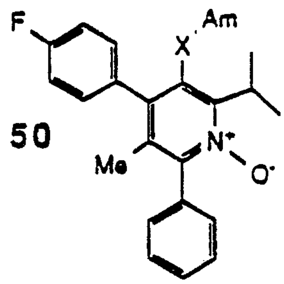 |
| | 49 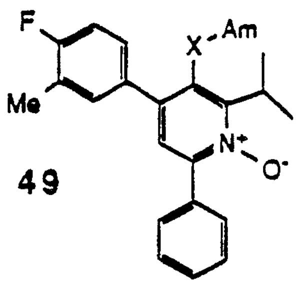 | 51 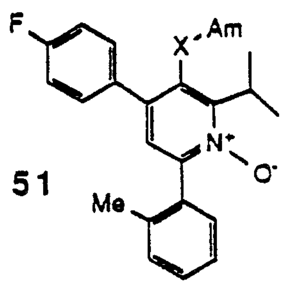 |

The following are the IUPAC names for the compounds described above:

16. (S)-4-[[[4-(4-Fluorophenyl)-5-methyl-2-(1-methylethyl)-6-phenyl-3-pyridinyl]ethynyl]-hydroxyphosphinyl]-3-hydroxybutanoic acid, disodium salt;

17. (S)-4-[[[4-Fluorophenyl)-2-(1-methylethyl)-5H-indeno[1,2-b]pyridin-3-yl]ethynyl]-hydroxyphospphinyl]-3-hydroxybutanoic acid, disodium salt;

18. (S)-4-[[[4-(4-Fluoro-3-methylphenyl)-2-(1-methylethyl)-6-phenyl-3-pyridinyl]ethynyl]-hydroxyphosphinyl-3-hydroxybutanoic acid, disodium salt;

19. (S)-4-[[[2-(1,1-Dimethylethyl)-4-(4-fluorophenyl)-6-phenyl-3-pyridinyl]ethynyl]hydroxyphosphinyl]-3-hydroxybutanoic acid, disodium salt;

20. (S)-4-[[[4-(4-Fluorophenyl)-2-(1-methylethyl)-6-(2-thienyl)-3-pyridinyl]ethynyl]hydroxyphosphinyl]-3-hydroxybutanoic acid, disodium salt;

21. (S)-4-[[[4-(4-Fluorophenyl)-5,6-dihydro-2-(1-methylethyl)benzo[h]quinolin-3-yl]ethynyl]-hydroxyphosphinyl]-3-hydroxybutanoic acid, disodium salt;

22. (S)-4-[[[6-(1,3-Benzodioxol-4-yl)-4-(4-fluorophenyl)-2-(1-methylethyl)-3-pyridinyl]ethynyl]-hydroxyphosphinyl]-3-hydroxybutanoic acid, disodium salt;

23. (S)-4-[[[4-(4-Fluorophenyl)-2-(1-methylethyl)-5,6-diphenyl-3-pyridinyl]ethynyl]hydroxyphosphinyl]-3-hydroxybutanoic acid, disodium salt;

24. (S)-4-[[[4-(4-Fluorophenyl)-2-(1-methylethyl)-6-(2-methylphenyl)-3-pyridinyl]ethynyl]-hydroxyphosphinyl]-3-hydroxybutanoic acid, disodium salt;

25. (S)-4-[[[6-[[1,1′-Biphenyl]-3-yl]-4-(4-fluorophenyl)-2-(1-methylethyl)-3-pyridinyl]ethynyl]-hydroxyphosphinyl]-3-hydroxybutanoic acid, disodium salt;

26. (S)-4-[[[6-(1,3-Benzodioxol-5-yl)-4-(4-fluorophenyl)-2-(1-methylethyl)-3-pyridinyl]ethynyl]-hydroxyphosphinyl]-3-hydroxybutanoic acid, disodium salt;

27. (S)-4-[[[2-(1,1-Dimethylethyl)-1-(4-fluoro-3-methylphenyl)-6-phenyl-3-pyridinyl]ethynyl]-hydroxyphosphinyl]-3-hydroxybutanoic acid, disodium salt;

28. (S)-4-[[[4-(4-Fluorophenyl)-6-methyl-2-(1-methylethyl)-3-pyridinyl]ethynyl]hydroxyphosphinyl]-3-hydroxybutanoic acid, 1-oxide, disodium salt;

29. (S)-4-[[[2-(1,1-Dimethylethyl)-4-(4-fluorophenyl)-6-(2-methylphenyl)-3-pyridinyl]ethynyl]-hydroxyphosphinyl]-3-hydroxybutanoic acid, disodium salt;

30. (S)-4[[[4-(4-Fluorophenyl)-2-(1-methylethyl)-6-phenyl-3-pyridinyl]ethynyl]hydroxyphosphinyl]-3-hydroxybutanoic acid, 1-oxide, disodium salt;

31. (S)-4-[[[4-(4-Fluorophenyl)-6,7-dihydro-2-(1-methylethyl)-5H-1-pyridin-3-yl]ethynyl]-hydroxyphosphinyl]-3-hydroxybutanoic acid, disodium salt;

32. (S)-4-[[[4-(4-Fluorophenyl)-5,6,7,8-tetrahydro-2-(1-methylethyl)-3-quinolinyl]-ethynyl]-hydroxyphosphinyl]-3-hydroxybutanoic acid, disodium salt;

33. (S)-4-[[[4-(4-Fluoro-3-methylphenyl)-2-(1-methylethyl)-3-quinolinyl]ethynyl]hydroxyphosphinyl]-3-hydroxybutanoic acid, 1-oxide, disodium salt;

34. (S)-4-[[[4-(1,1-Dimethylethyl)-2-(4-fluorophenyl)-3-quinolinyl]ethynyl]hydroxyphosphinyl]-3-hydroxybutanoic acid, disodium salt;

35. (S)-4-[[[2-(4-Fluoro-3-methylphenyl)-4-(1-methylethyl)-3-quinolinyl]-ethynyl]hydroxyphosphinyl]-3-hyroxybutanoic acid, sodium salt;

36. (S)-4-[[[4-(3,5-Dimethylphenyl)-2-(1-methylethyl)-6-phenyl-3-pyridinyl]ethynyl]hydroxyphosphinyl]-3-hydroxybutanoic acid, disodium salt;

37. (S)-4-[[[4-Fluorophenyl)-2-(1-methylethyl)-6-(2-pyridinyl)-3-pyridinyl]ethnyl]hydroxyphosphinyl]-3-hydroxybutanoic acid, disodium salt;

38. (S)-4-[[[4-Fluorophenyl)-6-(2-hydroxyphenyl)-2-(1-methylethyl)-3-pyridinyl]ethynyl]-hydroxyphosphinyl]-3-hydroxybutanoic acid, disodium salt;

39. (S)-4-[[[4-(4-Fluorophenyl)-2-(1-methylethyl)-6-(2-methylphenyl)-3-pyridinyl]ethynyl]-hydroxyphosphinyl]-3-hydroxybutanoic acid, 1-oxide, disodium salt;

40. (3R,5S,6E)-7-[4-(4-Fluoro-3-methylphenyl)-2-(1-methylethyl)-6-phenyl-3-pyridinyl]-3,5-dihydroxy-6-heptenoic acid, monosodium salt;

41. (3R,5S,6E)-7-[4-(3,5-Dimethylphenyl)-2-(1-methylethyl)-6-phenyl-3-pyridinyl]-3,5-dihydroxy-6-heptenoic acid, monosodium salt;

42. (3R,5S,6E)-7-[4-(4-Fluoro-2-methylphenyl)-2-(1-methylethyl)-6-phenyl-3-pyridinyl]-3,5-dihydroxy-6-heptanoic acid, monosodium salt;

43. (3R,5S,6E)-7-[4-(4-Fluorophenyl)-5-methyl-2-(1-methylethyl)-6-phenyl-3-pyridinyl]-3,5-dihydroxy-6-heptenoic acid, monosodium salt;

44. (3R,5S,6E)-7-[2-(4-Fluoro-methylphenyl)-4-(1-methylethyl)-6-phenyl-3-pyridinyl]-3,5-dihydroxy-6-heptenoic acid, monosodium salt;

45. (3R,5S,6E)-7-[4-(4-Fluorophenyl)-2-(1-methylethyl)-5,6-diphenyl-3-pyridinyl]-3,5-dihydroxy-6-heptenoic acid, monosodium salt;

46. (3R,5S,6E)-7-[4-(4-Fluorophenyl)-2-(1-methylethyl)-5H-indeno[1,2-b]-pyridin-3-yl]-3,5-dihydroxy-6-heptenoic acid, monosodium salt;

47. (3R,5S,6E)-7-[4-(4-Fluorophenyl)-5,6-dihydro-2-(1-methylethyl)benzo-[h]quinolin-3-yl]-3,5-dihydroxy-6-heptenoic acid, monosodium salt;

48. (3R,5S,6E)-7-[2-(1,1-Dimethylethyl)-4-(4-fluorophenyl)-6-phenyl-3-pyridinyl-3,5-dihydroxy-6-heptenoic acid, monosodium salt;

49. (3R,5S,6E)-7-[4-Fluoro-3-methylphenyl)-2-(1-methylethyl)-6-phenyl-3-pyridinyl]-3,5-dihydroxy-6-heptenoic acid, 1-oxide, monosodium salt;

50. (3R,5S,6E)-7-[4-(4-Fluorophenyl)-5-methyl-2-(1-methylehtyl)-6-phenyl-3-pyridinyl]-3,5-dihydroxy-6-heptenoic acid, 1-oxide, monosodium salt;

51. (3R,5S,6E)-7-[4-(4-Fluorophenyl)-2-(1-methylethyl)-6-(2-methylphenyl)-3-pyridinyl-3,5-dihydroxy-6-heptenoic acid, 1-oxide, monosodium salt.

The abbreviations used herein have the following meaning:

| | |
|---|---|
| $H_2SO_4$ | Sulfuric Acid |
| HOAc | Acetic Acid |
| $Et_2O$ | Diethyl Ether |
| THF | Tetrahydrofuran |
| EtOH | Ethanol |
| $LiAlH_4$ | Lithium Aluminum Hydride |
| DlBAL-H | Diisobutyl Aluminum Hydride |
| DMSO | Dimethyl Sulfoxide |
| EtONa | Sodium Ethoxide |
| $KOBu^t$ | Potassium t-butoxide |
| NaH | Sodium Hydride |
| LDA | Lithium Diisopropylamide |
| LiTMP | Lithium Tetramethylpiperdide |
| $LiN(TMS)_2$ | Lithium Bistrimethylsilylamide |
| $NH_2OH.HCl$ | Hydroxylamine Hydrochloride |
| $NH_4OAc$ | Ammonium Acetate |
| $Cu(OAc)_2$ | Copper (II) Acetate |
| $O_2$ | Oxygen |
| MeOH | Methanol |
| Pd | Palladium |
| Pt | Platinum |
| Ru | Ruthenium |
| $NaBH_4$ | Sodium Borohydride |
| EtOAc | Ethyl Acetate |
| $CH_2Cl_2$ | Dichloromethane |
| $CH_3CN$ | Acetonitrile |
| n-BuLi | n-Butyl Lithium |
| $H_2$ | Hydrogen |

| | |
|---|---|
| $CBr_4$ | Carbon Tetrabromide |
| $PPh_3$ | Triphenylphosphine |
| TBAF | Tetrabutyl Ammonium Fluoride |
| $(MeO)_2POCHN_2$ | Dimethyl Diazomethylphosphonate |
| $CHCl_3$ | Chloroform |
| $Bu_3SnH$ | Tri-n-butyltin Hydride |
| AIBN | α,α'-Azaisobutyrylnitrile |
| TEA | Triethylamine |
| $ClSi(T\text{-butyl})Ph_2$ | t-Butyldiphenylsilyl Chloride |
| $ClSi(t\text{-butyl})Me_2$ | t-Butyldimethylsilyl Chloride |
| ClSi $Ph_3$ | Triphenylsilyl Chloride |
| HF | Hydrofluoric Acid |
| m-CPBA | meta-Chloroperoxybenzoic Acid |
| $CF_3CO_3H$ | Peroxytrifluoroacetic Acid |
| t-butylLi | t-Butyl Lithium |

$A_2$, is

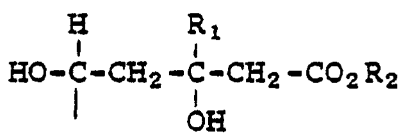

wherein $R_1$ is H or lower alkyl; and $R_2$ is H or lower alkyl in free acid form or in the form of a physiologically acceptable and hydrolyzable ester or δ lactone thereof, or an alkali metal salt ion or alkaline earth metal salt ion.

## Claims

1. A compound comprising a quinoline and pyridine structure (I)

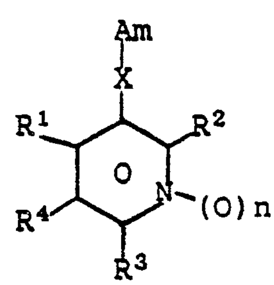

I

wherein $R^1$, $R^2$, $R^3$, and $R^4$ are the same or different and are each independently selected from H or a lower alkyl, aryl, substituted aryl or aralkyl, or aralkoxy and n is 0 or 1, linked by an appropriate linker (X) to a binding domain sidechain (Am); $R^3$ & $R^4$ taken together can be $-(CH_2)p-$,

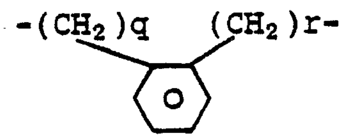

or $(CH=CH)_2$ wherein p = 3-5; q=0-3 and r=0-3; except when Am = $A_2$ as described in Claim 3, $R^3$, $R^4$ cannot be $(CH=CH)_2$.

2. The compound defined in Claim 1 wherein the binding domain sidechain (Am) is a dihydroxy acid and salt thereof;

3. The compound as defined in Claim 1 wherein the dihydroxy acid hereinafter $A_2$, is

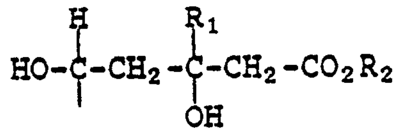

wherein $R_1$ is H or lower alkyl; and $R_2$ is H or lower alkyl in free acid form or in the form of a physiologically acceptable and hydrolyzable ester or $\delta$ lactone thereof, or an alkali metal salt ion or alkaline earth metal salt ion.

4. The compound as described in Claim 1 wherein the binding domain sidechain (Am) is a phosphinic acid and salt thereof.

5. The compound as defined in Claim 4 wherein the phosphinic acid hereinafter $A_1$, is

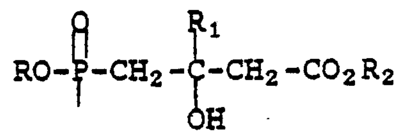

wherein R and $R_2$ are independently selected from hydrogen, lower alkyl, alkali metal salt ion and alkaline earth metal salt ion; and $R_1$ is H or lower alkyl.

6. The compound defined in Claim 3 wherein the linker (X) is $-(CH_2)_a$, -CH=CH-, -C≡C-, $-CH_2O$ where O is linked to the aromatic anchor) and "a" is 1 to 3 carbon atoms.

7. The compound defined in Claim 4 wherein the linker (X) is $-(CH_2)_a$, -CH=CH-, -C=C-, or $-CH_2O$ where O is linked to the phosphorus atom or to the aromatic anchor) and "a" is 1 to 3 atoms.

8. The compound as defined in Claim 6 wherein the linker is trans-CH=CH-.

9. The compound as defined in Claim 6 wherein the linker is cis-CH=CH-.

10. The compound as defined in Claim 7 wherein the linker is trans-CH=CH-.

11. The compound as defined in Claim 7 wherein the linker is cis-CH=CH-.

12. The compound as defined in Claim 1 having the name

(3R*, 5S*, 6E)-7-[4-(4-Fluorophenyl)-2-(1-methyl-ethyl)-6-phenyl-3-pyridinyl]-3,5-dihydroxy-6-heptenoic acid, monolithium salt;

(3R*, 5S*, 6E)-7-[4-(4-Fluorophenyl)-2-methyl-6-phenyl-3-pyridinyl]-3,5-dihydroxy-6-heptenoic acid, monolithium salt;

(3R*, 5S*, 6E)-7-[2-(4-Fluorophenyl)-4-(1-methyl-ethyl)-6-phenyl-3-pyridinyl]-3,5-dihydroxy-6-heptenoic acid, monolithium salt;

(S)-4-[[[4-(4-Fluorophenyl)-2-(1-methylethyl)-6-phenyl-3-pyridinyl]ethynyl]hydroxyphosphinyl]-3-hydroxybutanoic acid, dilithium salt;

(S)-4-[[[2-(4-Fluorophenyl)-4-(1-methylethyl)-3-quinolinyl]ethynyl]hydroxyphosphinyl]-3-hydroxy-butanoic acid, dilithium salt;

(S)-4-[[2-[4-(4-Fluorophenyl)-2-(1-methylethyl)-6-phenyl-3-pyridinyl]ethyl]hydroxyphosphinyl]-3-hydroxybutanoic acid, dilithium salt;

(S,E)-4-[[2-[4-(4-Fluorophenyl)-2-(1-methethyl)-6-phenyl-3-pyridinyl]ethenyl]hydroxyphosphinyl]-3-hydroxybutanoic acid, dilithium salt;

(S)-4-[[[4-(4-Fluorophenyl)-2-(1-methylethyl)-3-quinolinyl]ethynyl|hydroxyphosphinyl]-3-hydroxybutanoic acid, dilithium salt;

(S)-4-[[[4-(4-Fluorophenyl)-2-(1-methylethyl)-3-quinolinyl]ethynyl]hydroxyphosphinyl]-3-hydroxybutanoic

acid, N-oxide, dilithium salt
[3S,4(E)]-4-[[2-[2-(4-Fluorophenyl)-4-(1-methylethyl)-3-quinolinyl]ethenyl]hydroxyphosphinyl]-3-hydroxybutanoic acid, dilithium salt;
[3S,4(E)]-4-[[2-[4-(4-Fluorophenyl)-2-(1-methyl-ethyl)-3-quinolinyl]ethenyl]hydroxyphosphinyl]-3-hydroxybutanoic acid, dilithium salt;
(S)-4-[[[2-(4-Fluorophenyl)-4-(1-methylethyl)-3-quinolinyl]ethynyl]hydroxyphosphinyl]-3-hydroxybutanoic acid, 1-oxide, dilithium salt;
[3S, 4(E)]-4-[[2-[4-(4-Fluorophenyl)-2-(1-methyl-ethyl)-3-quinolinyl]ethenyl]hydroxyphosphinyl]-3-hydroxybutanoic acid, 1-oxide, dilithium salt;
(S)-4-[[[2-(4-Fluorophenyl)-4-(1-methylethyl)-6-phenyl-3-pyridinyl]ethynyl]hydroxyphosphinyl]-3-hydroxybutanoic acid, dilithium salt;
[3S,4(E)]-4-[[2-[2-(4-Fluorophenyl)-4-(1-methylethyl)-6-phenyl-3-pyridinyl]ethenyl]hydroxyphosphinyl]-3-hydroxybutanoic acid, dilithium salt;
(S)-4-[[[4-(4-Fluorophenyl)-5-methyl-2-(1-methylethyl)-6-phenyl-3-pyridinyl]ethynyl]hydroxyphosphinyl]-3-hydroxybutanoic acid, disodium salt;
(S)-4-[[[4-Fluorophenyl)-2-(1-methylethyl)-5H-indeno[1,2-b]pyridin-3-yl]ethynyl]hydroxyphospphinyl]-3-hydroxybutanoic acid, disodium salt;
(S)-4-[[[4-(4-Fluoro-3-methylphenyl)-2-(1-methylethyl)-6-phenyl-3-pyridinyl]ethynyl]hydroxyphosphinyl-3-hydroxybutanoic acid, disodium salt;
(S)-4-[[[2-(1,1-Dimethylethyl)-4-(4-fluorophenyl)-6-phenyl-3-pyridinyl]ethynyl]hydroxyphosphinyl]-3-hydroxybutanoic acid, disodium salt;
(S)-4-[[[4-(4-Fluorophenyl)-2-(1-methylethyl)-6-(2-thienyl)-3-pyridinyl]ethynyl]hydroxyphosphinyl]-3-hydroxybutanoic acid, disodium salt;
(S)-4-[[[4-(4-Fluorophenyl)-5,6-dihydro-2-(1-methylethyl)benzo[h]quinolin-3-yl]ethynyl]hydroxyphosphinyl]-3-hydroxybutanoic acid, disodium salt;
(S)-4-[[[6-(1,3-Benzodioxol-4-yl)-4-(4-fluorophenyl)-2-(1-methylethyl)-3-pyridinyl]ethynyl]hydroxyphosphinyl]-3-hydroxybutanoic acid, disodium salt;
(S)-4-[[[4-(4-Fluorophenyl)-2-(1-methylethyl)-5,6-diphenyl-3-pyridinyl]ethynyl]hydroxyphosphinyl]-3-hydroxybutanoic acid, disodium salt;
(S)-4-[[[4-(4-Fluorophenyl)-2-(1-methylethyl)-6-(2-methylphenyl)-3-pyridinyl]ethynyl]hydroxyphosphinyl]-3-hydroxybutanoic acid, disodium salt;
(S)-4-[[[6-[[1,1′-Biphenyl]-3-yl]-4-(4-fluorophenyl)-2-(1-methylethyl)-3-pyridinyl]ethynyl]hydroxyphosphinyl]-3-hydroxybutanoic acid, disodium salt;
(S)-4-[[[6-(1,3-Benzodioxol-5-yl)-4-(4-fluorophenyl)-2-(1-methylethyl)-3-pyrindinyl]ethynyl]-hydroxyphosphinyl]-3-hydroxybutanoic acid, disodium salt;
(S)-4-[[[2-(1,1-Dimethylethyl)-1-(4-fluoro-3-methylphenyl)-6-phenyl-3-pyridinyl]ethynyl]hydroxyphosphinyl]-3-hydroxybutanoic acid, disodium salt;
(S)-4-[[[4-(4-Fluorophenyl)-6-methyl-2-(1-methylethyl)-3-pyridinyl]ethynyl]hydroxyphosphinyl]-3-hydroxybutanoic acid, 1-oxide, disodium salt;
(S)-4-[[[2-(1,1-Dimethylethyl)-4-(4-fluorophenyl)-6-(2-methylphenyl)-3-pyridinyl]ethynyl]hydroxyphosphinyl]-3-hydroxybutanoic acid, disodium salt;
(S)-4-[[[4-(4-Fluorophenyl)-2-(1-methylethyl)-6-phenyl-3-pyridinyl]ethynyl]hydroxyphosphinyl]-3-hydroxybutanoic acid, 1-oxide, disodium salt;
(S)-4-[[[4-(4-Fluorophenyl)-6,7-dihydro-2-(1-methylethyl)-5H-1-pyridin-3-yl]ethynyl]hydroxyphosphinyl]-3-hydroxybutanoic acid, disodium salt;
(S)-4-[[[4-(4-Fluorophenyl)-5,6,7,8-tetrahydro-2-(1-methylethyl)-3-quinolinyl]-ethynyl]hydroxyphosphinyl]-3-hydroxybutanoic acid, disodium salt;
(S)-4-[[[4-(4-Fluoro-3-methylphenyl)-2-(1-methylethyl)-3-quinolinyl]ethynyl]hydroxyphosphinyl]-3-hydroxybutanoic acid, 1-oxide, disodium salt;
(S)-4-[[[4-(1,1-Dimethylethyl)-2-(4-fluorophenyl)-3-quinolinyl]ethynyl]hydroxyphosphinyl]-3-hydroxybutanoic acid, disodium salt;
(S)-4-[[[2-(4-Fluoro-3-methylphenyl)-4-(1-methylethyl)-3-quinolinyl]-ethynyl]hydroxyphosphinyl]-3-hyroxybutanoic acid, sodium salt;
(S)-4-[[[4-(3,5-Dimethylphenyl)-2-(1-methylethyl)-6-phenyl-3-pyridinyl]ethynyl]hydroxyphosphinyl]-3-hydroxybutanoic acid, disodium salt;
(S)-4-[[[4-Fluorophenyl)-2-(1-methylethyl)-6-(2-pyridinyl)-3-pyridinyl]ethnyl]hydroxyphosphinyl]-3-hydroxybutanoic acid, disodium salt;
(S)-4-[[[4-Fluorophenyl)-6-(2-hydroxyphenyl)-2-(1-methylethyl)-3-pyridinyl]ethynyl]hydroxyphosphinyl]-3-hydroxybutanoic acid, disodium salt;
(S)-4-[[[4-(4-Fluorophenyl)-2-(1-methylethyl)-6-(2-methylphenyl)-3-pyridinyl]ethynyl]hydroxyphosphinyl]-3-hydroxybutanoic acid, 1-oxide, disodium salt;
(3R,5S,6E)-7-[4-(4-Fluoro-3-methylphenyl)-2-(1-methylethyl)-6-phenyl-3-pyridinyl]-3,5-dihydroxy-6-heptenoic acid, monosodium salt;
(3R,5S,6E)-7-[4-(3,5-Dimethylphenyl)-2-(1-methylethyl)-6-phenyl-3-pyridinyl]-3,5-dihydroxy-6-heptenoic acid, monosodium salt;
(3R,5S,6E)-7-[4-(4-Fluoro-2-methylphenyl)-2-(1-methylethyl)-6-phenyl-3-pyridinyl]-3,5-dihydroxy-6-heptanoic acid, monosodium salt;
(3R,5S,6E)-7-[4-(4-Fluorophenyl)-5-methyl-2-(1-methylethyl)-6-phenyl-3-pyridinyl]-3,5-dihydroxy-6-heptenoic acid, monosodium salt;
(3R,5S,6E)-7-[2-(4-Fluoro-methylphenyl)-4-(1-methylethyl)-6-phenyl-3-pyridinyl]-3,5-dihydroxy-6-heptenoic acid, monosodium salt;
(3R,5S,6E)-7-[4-(4-Fluorophenyl)-2-(1-methylethyl)-5,6-diphenyl-3-pyridinyl]-3,5-dihydroxy-6-heptenoic acid, monosodium salt;
(3R,5S,6E)-7-[4-(4-Fluorophenyl)-2-(1-methylethyl)-5H-indeno[1,2-b]-pyridin-3-yl]-3,5-dihydroxy-6-heptenoic acid, monosodium salt;
(3R,5S,6E)-7-[4-(4-Fluorophenyl)-5,6-dihydro-2-(1-methylethyl)benzo-[h]quinolin-3-yl]-3,5-dihydroxy-6-heptenoic acid, monosodium salt;
(3R,5S,6E)-7-[2-(1,1-Dimethylethyl)-4-(4-fluorophenyl)-6-phenyl-3-pyridinyl-3,5-dihydroxy-6-heptenoic acid, monosodium salt;
(3R,5S,6E)-7-[4-Fluoro-3-methylphenyl)-2-(1-methylethyl)-6-phenyl-3-pyridinyl]-3,5-dihydroxy-6-heptenoic acid, 1-oxide, monosodium salt;
(3R,5S,6E)-7-[4-(4-Fluorophenyl)-5-methyl-2-(1-methylehtyl)-6-phenyl-3-pyridinyl]-3,5-dihydroxy-6-heptenoic acid, 1-oxide, monosodium salt;
(3R,5S,6E)-7-[4-(4-Fluorophenyl)-2-(1-methylethyl)-6-(2-methylphenyl)-3-pyridinyl-3,5-dihydroxy-6-heptenoic acid, 1-oxide, monosodium salt.

13. A hypocholesterolemic or hypolipidemic composition comprising a compound as defined in Claim 1 and a pharmaceutically acceptable carrier therefore.

14. Use of a compound as defined in Claim 1 for preparing a medicament for inhibiting cholesterol biosynthesis.